(19) Europäisches Patentamt
European Patent Office
Office européen des brevets

(11) **EP 4 356 901 A1**

(12) **EUROPEAN PATENT APPLICATION**
published in accordance with Art. 153(4) EPC


(43) Date of publication:
**24.04.2024 Bulletin 2024/17**

(21) Application number: **22825404.1**

(22) Date of filing: **07.04.2022**

(51) International Patent Classification (IPC):
***A61K 9/16*** *(2006.01)* ***A61K 38/13*** *(2006.01)*
***A61K 9/00*** *(2006.01)* ***A61P 17/14*** *(2006.01)*
***A61K 47/34*** *(2017.01)* ***A61K 47/32*** *(2006.01)*
***A61K 38/12*** *(2006.01)*

(52) Cooperative Patent Classification (CPC):
**A61K 9/00; A61K 9/16; A61K 38/12; A61K 38/13; A61K 47/32; A61K 47/34; A61P 17/14**

(86) International application number:
**PCT/KR2022/095079**

(87) International publication number:
**WO 2022/265481 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.06.2021 KR 20210076648**
**20.01.2022 KR 20220008307**



(71) Applicant: **Orientbio Inc.**
**Seongnam-si, Gyeonggi-do 13201 (KR)**

(72) Inventors:
- **KIM, Sang Nyun**
**Seoul 04712 (KR)**
- **CHANG, Jae Jin**
**Seoul 02823 (KR)**

(74) Representative: **AWA Denmark A/S**
**Strandgade 56**
**1401 Copenhagen K (DK)**


# (54) RELEASE-CONTROLLED TOPICAL INJECTION COMPOSITION CONTAINING CYCLIC PEPTIDE AND PREPARATION METHOD THEREFOR


(57) Disclosed herein are particles including a position 3-substituted cyclosporine derivative and a biodegradable polymer, a controlled-release composition for topical injection including the same, and use of the same for prevention of hair loss and promotion of hair growth.


【FIG. 42】

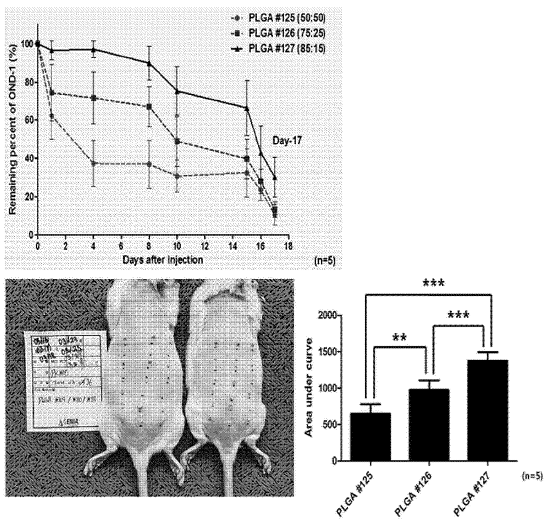


EP 4 356 901 A1

## Description

[Technical Field]

The present invention relates to a cyclic peptide-containing controlled-release composition for topical injection and a method of preparing the same.

[Background Art]

There are three phases of human hair growth: anagen, catagen, and telogen. Alopecia is an abnormal increase in the number of hairs falling out due to decrease in hairs in the anagen phase and increase in hairs in the catagen or telogen phases. Known causes of hair loss include excessive male hormones, poor blood circulation, excessive sebum production, bacterial infections, genetic factors, aging, and stress. In particular, androgenetic alopecia (AGA) is a type of hair loss caused by genetic factors and androgens.

Cyclosporine A (CsA) as an immunosuppressant has been shown to be more effective than minoxidil and finasteride in inducing the anagen phase and inhibiting the catagen phase. However, cyclosporine A has side effects such as hypertension, headache, and decline in immunity when applied systemically.

In order to overcome the disadvantages of cyclosporine A, a method was studied to topically administrate cyclosporine A using a polymer-based controlled release drug delivery system. The polymer-based controlled release drug delivery systems can control the rate of drug release and reduce toxicity of drugs due to systemic action, thereby greatly improving therapeutic efficiency. However, when cyclosporine A is loaded into a conventional polymer-based drug delivery system, an initial burst occurs, making it difficult to achieve sustained and stable release. The initial burst phenomenon means that a significant amount of a drug is released within a very short period of time, for example, in a few hours or 1 to 2 days.

Therefore, there is a need for a formulation that can provide prolonged and stable release of topically administered cyclosporine A drugs, thereby reducing systemic side effects and the number of doses, and can adjust the rate of drug release depending on the degree and type of hair loss.

[Disclosure]

[Technical Problem]

The present invention provides a controlled-release composition for topical administration that comprises a position 3-substituted cyclosporine derivative and a biodegradable polymer, and a method for preventing hair loss and promoting hair growth using the same.

[Technical Solution]

One aspect of the present invention provides controlled-release particles including a position 3-substituted cyclosporine derivative and a biodegradable polymer.

Cyclosporine A ($C_{62}H_{111}N_{11}O_{12}$) has a cyclic structure consisting of 11 amino acids. Seven thereof are N-methylated, and the remaining four are not N-methylated. Cyclosporine A is a poorly water-soluble drug since hydrogen bonded to a non-N-methylated nitrogen atom forms a hydrogen bond with a carbonyl group in the molecule, making it difficult to interact with water molecules. Due to very large molecular weight (1,202,63 Da), strong hydrophobicity, and low water solubility (7.3 μg/mL at 37°C) thereof, cyclosporine A exhibits a bioavailability of less than 30% and thus a very low absorption rate when administered orally.

Cyclosporine A may be represented by Structural Formula 1.

[Structural Formula 1]

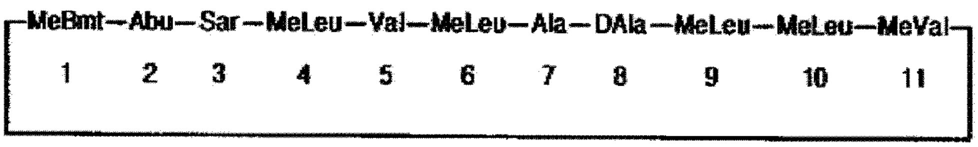

where MeBmt is N-methyl-(4R)-4-[(E)-2-butenyl]-4-methyl-L-threonine, Abu is L-aminobutyric acid, Sar is sarcosine,

MeLeu is N-methyl-L-leucine, Val is L-valine, Ala is L-alanine, DAla is D-alanine, and MeVal is N-methyl-L-Valine.

The amino acids of cyclosporine A have L-configuration unless otherwise noted, and amino acid residues are numbered such that MeBmt is designated as number 1 and MeVal (N-methyl-L-Valine) as the last residue in clockwise order is designated as number 11, as shown in Structural Formula 1. For details of the nomenclature of cyclosporine A and derivatives thereof, refer to Helv. Chim. Acta, 1987;70;13-36.

Here, the position 3-substituded cyclosporine derivative refers to a derivative in which sarcosine as the residue at position 3 is substituted with a different residue. For example, when sarcosine as the position 3 residue of cyclosporine A is substituted with [2-methylthio-sarcosine$^3$], a corresponding derivative may be named [2-methylthio-Sar$^3$]cyclosporine A. Herein, [D-2-methylthio-Sar$^3$]cyclosporine A may be referred to as OND-1, and has a chemical formula of $C_{63}H_{113}N_{11}O_{12}S$ and a MW of 1248.70.

The position 3-substituted cyclosporine derivative may be represented by Formula 1.

[Formula 1]

MeBmt-Abu-**C**-MeLeu-Val-MeLeu-Ala-DAla-MeLeu-MeLeu-MeVal

In Formula 1, C may be an amino acid represented by $-N(R_1)-CH(R_2)-C(=O)-$,

wherein $R_1$ may be hydrogen or a methyl group, and

$R_2$ may be hydrogen; a linear or branched $C_1$-$C_6$ alkyl group, alkenyl group, or alkynyl group; or an aryl group. Each carbon in the alkyl group, the alkenyl group, the alkynyl group, and the aryl group may be substituted or unsubstituted with 1 to 3 substituents selected from the group consisting of an amino group, a hydroxyl group, a halogen group, a haloalkyl group, an ester group, an alkoxy group, a cyano group, a nitro group, an alkylamino group, and a dialkylamino group. The aryl group may be a phenyl group.

$R_2$ may be X-R', wherein X may be oxygen (O) or sulfur (S) and R' may be: hydrogen; a linear or branched $C_1$-$C_6$ alkyl group, alkenyl group, or alkynyl group; or an aryl group. Each carbon in the alkyl group, the alkenyl group, the alkynyl group, and the aryl group may be substituted or unsubstituted with 1 to 3 substituents selected from the group consisting of an amino group, a hydroxyl group, a halogen group, a haloalkyl group, an ester group, an alkoxy group, a cyano group, a nitro group, an alkylamino group, and a dialkylamino group.

X may be sulfur (S), and R' may be a methyl group, or may be a methyl group substituted with 1 to 3 substituents selected from the group consisting of an amino group, a hydroxyl group, a halogen group, a haloalkyl group, an ester group, an alkoxy group, a cyano group, a nitro group, an alkylamino group, and a dialkylamino group.

In Formula 1, C may be a D-amino acid or an L-amino acid.

The position 3-substituted cyclosporine derivative may be [2-methylthio-sarcosine$^3$]cyclosporine A; [2-(4-methylbenzentio)sarcosine$^3$]cyclosporine A; [2-dimethylamino-ethylthio-sarcosine$^3$]cyclosporine A; [N-methyl-aminobutyric acid$^3$]cyclosporine A; [N-methyl-norvaline$^3$]cyclosporine A; [2-(methylamino)-hexa-4-ynoyl$^3$]cyclosporine A; [2-(methylamino)-pent-4-ynoyl$^3$]cyclosporine A; [N-methyl-O-propenyl-serine$^3$]cyclosporine A; [N-methyl-serine$^3$]cyclosporine A; [methyl-alanine$^3$]cyclosporine A; [N-methyl-D-norvaline$^3$]cyclosporine A; or a combination thereof.

The position 3-substituted cyclosporine derivative may be amorphous. The physical state of a drug is crystalline, amorphous, or a combination thereof, and is one of the important properties that affects the rate of drug release from a formulation. According to experimental examples described below, the position 3-substituted cyclosporine derivative was found to be amorphous and have good interaction with polymers.

As used herein, the term "controlled-release particles" refers to particles that allow a drug to be released into the body in specified amounts over a specified period of time through adjustment of the ratio of the position 3-substituted cyclosporine derivative to the biodegradable polymer, preparation conditions, properties of the biodegradable polymer, and the like. The controlled-release particles according to the present invention can provide sustained drug release without an initial burst through interaction between the position 3-substituted cyclosporine derivative and the biodegradable polymer. Here, the interaction may be an intermolecular force such as van der Waals forces or hydrogen bonding, which can be confirmed by Fourier transform infrared spectroscopy (FT-IR) or differential scanning calorimetry (DSC). In the experimental examples described below, due to binding between the position 3-substituted cyclosporine derivative and PLGA, the particles showed shifts of a peak at 1,622 $cm^{-1}$ of the amide I band (amide C=O) and a peak at 1,746 $cm^{-1}$ of an ester bond (ester C=O) of PLGA and changes in intrinsic melting point or glass transition temperature of the position 3-substituted cyclosporine derivative. Conversely, simple physical mixing of the position 3-substituted cyclosporine derivative with the polymer did not result in these changes. This indicates that the position 3-substituded

cyclosporine derivative drug and the polymer in the controlled-release particles were strongly bound to each other by an intermolecular force such as van der Waals forces or hydrogen bonding.

The biodegradable polymer may be a synthetic polymer or a natural polymer.

The synthetic polymer may include at least one selected from the group consisting of polyethylene (PEA), polyethylene glycol (PEG), polycaprolactone (PCL), polyalkylcarbonate, polyamino acid, polyhydroxybutyric acid, polyorthoester, polyanhydride, Pluronic (poly(ethylene oxide)poly(propylene oxide)poly(ethylene oxide)), polylactide (PLA), polyglycolide (PGA), polybutylene succinate (PBS), poly(3-hydroxybutyrate-co-3-hydroxyvalerate) (PHBV), polybutylene terephthalate (PBT), polytrimethylene terephthalate (PTT), polyethylene naphthalate (PEN), polylactide-co-glycolide (poly(lactic-co-glycolic acid), PLGA), polylactide-co-glycolide-glucose (PLGA-glucose), and methoxy polyethylene glycol-(polycaprolactone-co-polylactide) (MPEG-(PCL-co-PLLA)).

The synthetic polymer may be: polylactide-co-glycolide (poly(lactic-co-glycolic acid), PLGA); or a di- or tri-block copolymer having polylactide-co-glycolide as a hydrophobic block. Polylactide-co-glycolide (PLGA) is degraded in the body into water and carbon dioxide, which are harmless to humans, by the citric acid cycle, which is a normal metabolic process, and thus has good biocompatibility.

The biodegradable polymer may form spherical particles loaded with a therapeutic agent for hair loss diseases.

By changing the type and composition of the biodegradable polymer, the rate of drug release can be controlled through changes in rate of biodegradation of the biodegradable polymer. For example, since the rate of biodegradation of polylactide-co-glycolide (PLGA) varies depending on the ratio of polylactide (PLA) to polyglycolide (PGA), the rate of drug release can be controlled through adjustment of the ratio of PLA to PGA. Since glycolide is less hydrophobic than lactide, increase in proportion of glycolide in a PLGA copolymer as the biodegradable polymer can increase the rate of hydrolysis of the polymer, thereby increasing the rate of drug release.

The polylactide-co-glycolide (PLGA) may have a molar ratio of lactide monomer (LA) to glycolide monomer (GA) of 90:10 to 40:60 or 85:15 to 50:50, for example, 50:50, 65:35, 75:25, or 85:15, without being limited thereto.

The biodegradable polymer may be a single polylactide-co-glycolide (PLGA) polymer having a specific LA:GA molar ratio, or may be a mixture of two or more polylactide-co-glycolide (PLGA) polymers each having a different LA:GA molar ratio.

The biodegradable polymer may be a blend of PLGA having a molar ratio of LA:GA of 50:50 and PLGA having a molar ratio of LA:GA of 75:25; a blend of PLGA having a molar ratio of LA:GA of 50:50 and PLGA having a molar ratio of LA:GA of 85:15; a blend of PLGA having a molar ratio of LA: GA of 75:25 and PLGA having a molar ratio of LA:GA of 85:15; or a blend of PLGA having a molar ratio of LA: GA of 50:50, PLGA having a molar ratio of LA: GA of 75:25, and PLGA having a molar ratio of LA: GA of 85:15.

The polylactide-co-glycolide (PLGA) may have an ester or carboxylic acid as an end group.

The polylactide-co-glycolide (PLGA) may have a molecular weight of 4,000 Da to 240,000 Da, 4,000 Da to 75,000 Da, 4,000 Da to 15,000 Da, 7,000 Da to 17,000 Da, or 50,000 Da to 75,000 Da.

The rate of drug release from the particles may depend on the molecular weight of the biodegradable polymer. Low molecular weight PLGA can form porous particles, thereby increasing the rate of polymer degradation and the rate of drug release, whereas high molecular weight PLGA can form dense particles, thereby providing an S-shaped slow release profile. Increasing the molecular weight and concentration of PLGA used in drug preparation can achieve high drug encapsulation efficiency, large particle size, and slow drug release. In addition to the type of end group and molecular weight of the biodegradable polymer, other physical properties, such as crystallinity and glass transition temperature (Tg), can indirectly affect the rate of polymer degradation.

The PLGA polymer may be an enantiomer (for example, poly(D-lactic-co-glycolic acid or poly(L-lactic-co-glycolic acid)) or a racemate (for example, poly(D,L-lactic-co-glycolic acid)).

Various PLGA polymers may be used depending on the molar ratio of lactide to glycolide, the end group, and the molecular weight thereof. The PLGA polymer may include, for example: Expansorb® series available from PCAS, such as 10P001 (50:50), 10P002 (75:25), 10P003 (75:25), 10P007 (90:10), 10P008 (85:15), 10P009 (85:15E), 10P010 (65:35E), 10P016 (50:50E), 10P017 (50:50), 10P019 (50:50E), 10P020 (85:15), 10P022 (50:50), 10P023 (90:10), 10P024 (50:50), and 10P025 (90:10); Resomer® series available from Aldrich® Evonik Rohm Pharma GmbH, such as RG 502 (50: 50), RG 502H (50:50), RG 503 (50:50), RG 503H (50:50), RG 504 (50:50), RG 504H (50:50), RG 505 (50:50), RG 653H (65: 35), RG 752H (75:25), and RG 756S (75:25); Purasorb® series available from Purac Biomaterials, such as PDLG 7502 (75:25), PDLG 7502A (75:25), PDLG 7507 (75:25), PDLG 5002 (50:50), PDLG 5002A (50:50), PDLG 5004 (50:50), PDLG 5004A (50:50), PDLG 5010 (50: 50); andPLGA-7505 (75:25), PLGA-7510 (75:25), PLGA-7515 (75:25), PLGA-7520 (75:25), PLGA-5005 (50:50), PLGA-5010 (50:50), and PLGA-5015 (50:50) available from Wako Pure Chemical Industries, without being limited thereto.

The polylactide-co-glycolide (PLGA) polymer may be a cationic graft copolymer with poly-L-Lysine (PLL) introduced thereinto. Introduction or grafting of PLL into PLGA can increase cationicity and thus adhesion to cell membranes, which is well known to those skilled in the art to which the present invention pertains.

The natural polymer may include at least one selected from the group consisting of carboxymethylcellulose

(CMC), algin, alginic acid, alginate, hyaluronic acid, polypeptides, proteins, gelatin, and collagen, albumin, dextran, starch, casein, chitin derivatives, chitosan, and small intestinal submucosa (SIS).

A weight ratio of the position 3-substituted cyclosporine derivative to the biodegradable polymer may range from 5:95 to 90:10, 10:90 to 90:10, 20:80 to 80:20, or 20:80 to 70:30, for example, 10:90 to 60:40 or 20:80 to 60:40, without being limited thereto. Within this range, an initial burst of drug release can be suppressed, thereby allowing stable drug release over a period of one week to four weeks or more while reducing side effects.

As used herein, "particles" may refer to nanoscopic or microscopic particles. The particles may be used interchangeably herein with "microspheres", "nanospheres", "nanoparticles", and "microparticles". The particles may have an average diameter of 0.01 $\mu$m to 300 $\mu$m, 0.1 $\mu$m to 200 $\mu$m, 0.1 $\mu$m to 100 $\mu$m, or 0.1 $\mu$m to 60 $\mu$m.

An average zeta potential of the particles may have a positive value, or may have a negative value. In some embodiments, the particles may have an average zeta potential of $\pm$2 mV or greater, $\pm$10 mV or greater, or $\pm$30 mV or greater. The particles may have an average zeta potential of +2 mV to -2 mV, +10 mV to -10 mV, or +30 mV to -30 mV. Herein, "zeta-potential" is indirectly calculated from the thickness of an electrical double layer around the particles, and refers to an electrodynamic potential difference derived from the positive charge density difference based on the force of repulsion or attraction between the particles. The thicker the electrical double layer, the more electrostatic repulsion between the particles and the less the particles will aggregate together. When the average zeta potential of the particles falls within the above range, aggregation between the particles can be inhibited, thereby ensuring stability of a dispersion.

The particles may have a spherical shape with a smooth surface. The smooth surface of the particles means that there are no or very few pores on the surface. As the surface of the particles is smooth, drug diffusion and initial burst due to the presence of pores can be inhibited, and drug release can only occur by degradation of the biodegradable polymer. Accordingly, the smooth surface of the particles indicates that the particles have suitable morphological characteristics for use as a controlled-release formulation. The experimental examples described below showed that the particles included in the controlled-release PLGA composition according to the present invention have a spherical shape with a smooth surface, thereby providing good controlled drug release while suppressing initial burst.

The particles may include 5% to 60% (w/w) or 15% to 40% (w/w) of the position 3-substituted cyclosporine derivative. Within this range of content of the position 3-substituted cyclosporine derivative, initial burst of drug release can be suppressed and stable drug release can be achieved over a planned period of time.

Another aspect of the present invention provides a composition for prevention of hair loss or promotion of hair growth, including the controlled-release particles.

The composition may be an injectable formulation for topical administration.

The composition may be used for treatment or prevention of hair loss.

The composition enables sustained release of the position 3-substituted cyclosporine derivative over a period of at least one week, for example, one week to four weeks, two weeks to four weeks, or three weeks to four weeks, when administered topically.

In order to ensure that a drug provides sufficient medicinal effects when administered topically to the site of hair loss, it is important that the drug does not diffuse systemically. Experimental examples described below show that the composition according to the present invention ensures high drug retention in the skin when administered topically and thus can be highly effective in preventing hair loss or promoting hair growth.

Hair loss may be one selected from the group consisting of androgenetic alopecia (AGA), female-pattern hair loss, and alopecia areata, telogen effluvium, and cicatricial alopecia.

As used herein, the term "prevention" refers to any act that inhibits or delays development of a disease or abnormality by administration of the composition according to the invention.

The composition may be administered by intralesional injection. Specifically, the composition may be injected intradermally or by mesotherapy into a targeted site of the scalp, or may be injected into the subcutaneous layer near hair follicles. The composition may be administered intradermally or subcutaneously into a site near hair follicles.

The composition may be intended to be administered by mesotherapy. "Mesotherapy" refers to an injection therapy in which a drug is injected directly into a targeted site, wherein a small amount of the drug is injected into a middle layer of the skin (the dermis layer) with a fine syringe to enhance therapeutic action of the drug, ensure long-lasting drug levels at the targeted site, and minimize side effects.

The composition may further include at least one selected from the group consisting of an aqueous vehicle for injection, an isotonic agent, and a suspending agent. In one embodiment, a controlled-release composition for topical injection according to the present invention may be a homogeneous suspension of position 3-substituted cyclosporine derivative-containing PLGA particles. The suspension may be prepared by mixing the aforementioned composition with at least one selected from the group consisting of an aqueous vehicle for injection, an isotonic agent, and a suspending agent.

The aqueous vehicle for injection may include, for example, at least one selected from the group consisting of saline, sterile water, a Ringer's solution, buffered saline, an albumin injectable solution, a dextrose solution, a maltodextrin

solution, glycerol, and ethanol, without being limited thereto.

The isotonic agent may include, for example, at least one selected from the group consisting of D-mannitol, maltitol, sorbitol, lactitol, xylitol, and sodium chloride, without being limited thereto.

The suspending agent may include at least one selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, and xanthan gum, without being limited thereto.

The composition may be formulated as a topical injection, a depot injection, or a dermal implant.

A further aspect of the present invention provides a method of preparing position 3-substituted cyclosporine derivative-loaded controlled-release particles, comprising:

a) preparing a drug-polymer dispersed phase by dissolving a position 3-substituted cyclosporine derivative and a biodegradable polymer in a solvent;
b) preparing an emulsion by mixing the dispersed phase with a continuous phase including a surfactant (emulsifying agent); and
c) producing position 3-substituted cyclosporine derivative-loaded particles by mixing the prepared emulsion with a quenching medium, followed by stirring to remove the solvent from the dispersed phase.

Here, the position 3-substituted cyclosporine derivative and the biodegradable polymer are the same as described above.

The solvent in step a) may be a dispersion medium. The dispersion medium may include any dispersion medium that can dissolve the biodegradable polymer. In some embodiments, the solvent in the dispersed phase may be an organic solvent having a low boiling point, wherein the organic solvent may have a boiling point of 25°C to 85°C, 25°C to 70°C, 25°C to 60°C, or 25 to 50°C. Within this range of boiling point of the organic solvent, it is possible to facilitate evaporation of the solvent in the dispersed phase and drying of the particles after production of the particles.

The dispersion medium may include, for example, at least one selected from the group consisting of dichloromethane, chloroform, acetonitrile, dimethyl sulfoxide (DMSO), dimethyl formaldehyde, ethyl acetate (EA), butyl acetate, ethyl formate, isopropyl acetate, isopropyl formate, diethyl ether, and glycofurol, without being limited thereto.

In step a), the solvent may further include a co-solvent. The co-solvent serves to improve drug release parameters and encapsulation efficiency. When ethanol is used as the co-solvent, the concentration of ethanol may affect a release profile of a hydrophobic drug. For example, as the concentration of ethanol increases, interfacial tension (IFT) decreases, causing the drug to leach away from the center of PLGA particles and crystallize on the surface of the particles. The co-solvent may be selected from among solvents that can dissolve the biodegradable polymer, are miscible with the dispersion medium, can ensure low solubility of the drug therein, and have a lower boiling point than the dispersion medium. For example, the co-solvent may include at least one selected from the group consisting of methanol, ethanol, acetone, isopropanol, diethyl ether, chloroform, ethyl acetate, DMSO, and acetonitrile, without being limited thereto.

In the preparation method, the dispersed phase in step a) may further include a release modifier. The release modifier serves to prevent microspheres from swelling upon contact with an aqueous solution or to delay release of the drug even when the microspheres swell and collapse. The release modifier may be a hydrophilic release modifier and may include, for example, at least one selected from the group consisting of retinoic acid, polyoxyethylene sorbitan fatty acid esters, glyceryl monooleate, sorbitan fatty acid esters, polyvinyl alcohol, poloxamers, polyethylene glycol, glyceryl palmitostearate, benzyl benzoate, ethyl oleate, α-cyclodextrin, β-cyclodextrin, γ-cyclodextrin, hydroxypropyl β-cyclodextrin, tween 80, span 20, PEG 400, Mrij 52, Brij 58, poloxamer P124, and SLS.

The release modifier may be a vegetable oil and may include, for example, lecithin, castor oil, medium chin triglyceride oil (MCT oil), corn oil, cotton seed oil, sesame oil, soybean oil, cinnamon oil, almond oil, arachis oil, linseed oil, olive oil, caraway oil, rosemary oil, peppermint oil, sunflower oil, eucalyptus oil, lavender oil, coconut oil, orange oil, anise oil, clove oil, concentrated borage oil, SEFOL 860, Miglyol, ATMOS 300, GMOrphic 80, DL-a-tocopheryl acetate, Labrafil, Imwitor 742, Myvacet, Myvaplex, Myverol, Generol, Myritol, Captex, Capmul, Neobee M5, Mazol 1400, Maisine 35-1, Crossential GLO E50 (concentrated borage oil ethyl ester), Nikkol EOO (ethyl olive oleate), ethyl oleate, oleic or linoleic acid, ethyl linoleate, isopropyl palmitate, isopropyl myristate, polyglyceryl oleate, polyglyceryl palmitostearate, diglyceryl monooleate, tetraglyceryl monooleate, hexaglyn, and decaglyn, without being limited thereto.

The release modifier may include an animal oil or a derivative thereof, and may include, for example, squalene, hydrogenated squalene, omega-3 essential fatty acids (EFAs), eicosapentaenoic acid, docosahexaenoic acid, and ethyl-esterified forms thereof, such as Incromega available from Croda Co. Ltd., without being limited thereto.

The surfactant (or emulsifying agent) in the continuous phase may include at least one selected from the group consisting of nonionic surfactants, anionic surfactants, and cationic surfactants.

The surfactant may include, for example, at least one selected from the group consisting of: high molecular weight surfactants such as polyethylene glycol (PEG), hydroxypropyl methylcellulose (HPMC), methyl cellulose, polyvinylpyrrolidone (PVP), gelatin, chitosan, Eudragit, and polyvinyl alcohol (PVA); hydrophilic low molecular weight surfactants such as lecithin, Tween (polyoxyethylene sorbitan fatty acid ester), poloxamers, polyoxyethylene castor oil derivatives, Cremophor, cyclodextrins, sodium lauryl sulfate (SLS), sodium stearate, esteramines, ethylene diamine, Myrj52, Brij 58, and fatty amines; and lipophilic low molecular weight surfactants such as span, labrasol, rheodol MO-60, glycerol monooleate, and triacetin. In one embodiment, the surfactant may be polyvinyl alcohol, without being limited thereto.

The surfactant may be present in an amount of 0.1% to 20% (w/v), 0.1% to 10% (w/v), or 1% to 3% (w/v), in the continuous phase. The content of the surfactant may be adjusted as appropriate to aid in formation of a stable emulsion from a solution of the biodegradable polymer.

The dispersed phase and the continuous phase in step b) may be mixed in a volume ratio of 1:5 to 1:50 or 1:10 to 1:25. Within this range of volume ratio of the dispersed phase to the continuous phase, proper spacing between microdroplets in an O/W emulsion after homogenization can be achieved, thereby facilitating formation of microspheres of uniform size.

In step c), as the emulsion is mixed and stirred with a large amount of the quenching medium, the dispersion medium and the co-solvent are evaporated and diffused into the continuous phase, thereby inducing primary curing of the polymer and causing drug particles to be entrapped into the particles. That is, as the dispersed phase solvent (the dispersion medium (organic solvent) or the co-solvent) in the microdroplets dispersed in the O/W emulsion is rapidly extracted and diffused into the continuous phase, the biodegradable polymer is rapidly solidified to form drug-loaded particles.

The quenching medium may be water, methanol, ethanol, propanol, ethyl acetate, polyvinyl alcohol (PVA), or a combination thereof. The quenching medium may be a solution of the same components as the continuous phase.

The amount of the quenching medium mixed with the emulsion may be, for example, 1 to 100 times, 2 to 100 times, 3 to 100 times, 4 to 100 times, 5 to 100 times, 6 to 100 times, 7 to 100 times, 8 to 100 times, 9 to 100 times, 10 to 100 times, 20 to 100 times, 30 to 100 times, 1 to 50 times, 2 to 50 times, 3 to 50 times, 4 to 50 times, 5 to 50 times, 6 to 50 times, 7 to 50 times, 8 to 50 times, 9 to 50 times, 10 to 50 times, 20 to 50 times, 30 to 50 times, 1 to 20 times, 2 to 20 times, 3 to 20 times, 4 to 20 times, 5 to 20 times, 6 to 20 times, 7 to 20 times, 8 to 20 times, 9 to 20 times, or 10 to 20 times, that of the emulsion or continuous phase, without being limited thereto.

Step c) may be performed by solvent extraction, solvent evaporation, spray drying, or the like. Since properties of the composition for drug delivery, such as particle size, drug release properties, and drug encapsulation efficiency, may vary depending on which preparation method is used, an appropriate preparation method may be selected as needed. For example, step c) may be performed by solvent evaporation, phase separation, self-healing encapsulation, and a mechanical process, such as membrane emulsification, spray drying, a supercritical fluid method, and a microfluidic method. Step c) may be performed by an emulsification method such as mechanical stirring, high pressure emulsification, fluid bed emulsification, static motor emulsification, ultrasonic emulsification, membrane emulsification, nozzle spraying, or dripping. Step c) may be performed by vigorous stirring with a magnetic stirrer to prevent aggregation of the microdroplets.

Step c) may be performed for 4 to 12 hours. A period of time for which Step c) is performed may be adjusted as appropriate to inhibit particle aggregation and ensure adequate drug encapsulation.

In one embodiment, the method of preparing position 3-substituted cyclosporine derivative-loaded controlled-release particles may further include: d) separating the produced position 3-substituted cyclosporine derivative-loaded particles after step c). In one embodiment, step d) may include subjecting the drug-loaded particles produced in step c) to centrifugation.

The method may further include: subjecting the separated particles to vacuum drying or freeze-drying after step d).

Step e) may include subjecting the separated particles to vacuum drying at room temperature or freeze-drying at -40°C to remove the dispersion medium (organic solvent) or the co-solvent remaining in the particles to induce secondary curing of the polymer. Through the drying process, the drug-loaded particles can have a solid structure and the drug can be completely entrapped in the particles.

Vacuum drying may be performed at a temperature of, for example, 30°C to 40°C, and freeze-drying may be performed at a temperature of, for example, -30°C to -40°C.

Step e) may be performed, for example, for 24 to 48 hours.

The method according to the present invention may further include: f) further subjecting the dried particles to vacuum drying or freeze-drying after step e).

Through step f), the solvent remaining in the particles can be completely removed, whereby the structure of the particles can be further solidified and complete drug encapsulation can be achieved.

Vacuum drying or freeze-drying in step f) may be performed under the same conditions as in step e).

If vacuum drying is performed at a temperature of less than 30°C, the solvent remaining in the particles can fail to be completely removed, whereas, if vacuum drying is performed at a temperature exceeding 40°C, the particles

can undergo structural deformation during the drying process due to heating to a temperature exceeding the glass transition temperature (Tg) of the biodegradable polymer, causing deterioration in controlled drug release properties.

Freeze-drying may be performed under a vacuum condition.

Step f) may be performed for 12 to 24 hours, without being limited thereto.

If the drying process in step f) is performed for less than 12 hours, the solvent remaining in the particles can fail to be completely removed, whereas, if the drying process in step f) is performed for more than 24 hours, this can cause reduction in productivity.

[Advantageous Effects]

The composition for prevention of hair loss or promotion of hair growth including controlled-release particles according to the present invention can be administered topically, such as by mesotherapy, to the site of hair loss, thereby ensuring sufficient supply of a position 3-substituted cyclosporine derivative, which is difficult to penetrate percutaneously, and can minimize systemic side effects since there is no rapid increase in drug level in the systemic circulation upon topical administration.

The composition for prevention of hair loss or promotion of hair growth according to the present invention can maintain a constant drug concentration at the site of hair loss, to which the composition is topically administered, for a long period of time, thereby improving patient compliance and convenience.

The controlled-release particles according to the present invention can control the rate of drug release by varying the degree of biodegradation of PLGA (such as the molecular weight thereof), the types of release modifier, solvent, polymer, and surfactant, the content of drug, the stirring speed in the emulsification process, the high-speed emulsification pressure, the duration of ultrasonic irradiation, the ratio of continuous phase to dispersed phase, and the drying method, and can be prepared to have an appropriate drug release rate depending on the type and degree of hair loss disease.

[Description of Drawings]

FIG. 1 is an SEM image of Example 1-A (#125).
FIG. 2 is an SEM image of Example 1-B (#126).
FIG. 3 is an SEM image of Example 1-C (#127).
FIG. 4 is an SEM image of Example 2-A (#109).
FIG. 5 is an SEM image of Example 2-B (#110).
FIG. 6 is an SEM image of Example 2-C (#111).
FIG. 7 is an SEM image of Example 4-1-A (#122).
FIG. 8 is an SEM image of Example 4-1-B (#108).
FIG. 9 is an SEM image of Example 4-1-C (#73).
FIG. 10 is an SEM image of Example 4-1-D (#73-1).
FIG. 11 is SEM images of Examples 31-B to 31-D.
FIG. 12 and FIG. 13 are SEM images of Examples 39-B, 40-B, 41-B, and 42-B.
FIG. 14 to FIG. 16 show results of particle size analysis of Examples 1-A to 1-C.
FIG. 17 to FIG. 19 show results of particle size analysis of Examples 31-B to 31-D.
FIG. 20 to FIG. 26 show results of drug release profile analysis.
FIG. 28 to FIG. 31 show results of XRD and DSC measurement.
FIGS. 32 to FIG. 35 show results of FT-IR measurement.
FIG. 36 to FIG. 38 show results of DSC measurement.
FIG. 39 shows results of determination as to whether a composition according to the present invention has a hair growth effect when administered by injection.
FIG. 40 shows results of determining the extent of drug loss into the systemic circulation upon topical administration of the composition according to the present invention by intradermal (intralesional) injection.
FIG. 41 shows results of determining the quantity of drug remaining in the skin upon topical administration of the composition of the present invention by intradermal (intralesional) injection.
FIG. 42 shows comparison of drug retention rates (%) over time after dermal administration of three drug formulations prepared using PLGA polymers with different LA to GA ratios.

[Mode for Invention]

Hereinafter, the present invention will be described in more detail with reference to some examples. However, it should be noted that these examples are provided for illustration only and are not to be construed in any way as limiting the present invention. In addition, it should be understood that terms not specifically defined herein have a meaning generally understood by those skilled in the art to which the present invention pertains.

Preparative Example: Preparation of position 3-substituted cyclosporine derivative

A general method for alkylation of cyclosporine A is as follows. First, diisopropylamine ((i-Pr)2NH) is added to tetrahydrofuran (THF) under nitrogen, followed by addition of a solution of butyllithium (BuLi) in hexane at 78°C and stirring for 30 minutes. Thereafter, cyclosporine A dissolved in tetrahydrofuran (THF) is added to the obtained LDA solution, followed by stirring for 1 hour, and then an electrophile is added to the resulting solution.

Preparative Example 1. Synthesis of [N-methyl-D-Abu$^3$]cyclosporine A

Using the general method, cyclosporine A (1.0 g) dissolved in tetrahydrofuran (THF) (50 mL) was added to 10 equivalents of an LDA solution at 78°C. Thereafter, the reaction mixture was stirred at 78°C for 2 hours, followed by addition of ethyl iodide (0.4 ml). The mixture was brought to room temperature and further stirred for 24 hours, followed by addition of 20 mL of water and concentration of the mixture. Thereafter, ether ($Et_2O$) was added to the resulting residue, which, in turn, was washed sequentially with water and a saturated aqueous sodium of chloride solution, dried over anhydrous sodium sulfate ($MgSO_4$), and concentrated. Thereafter, the residue was purified by column chromatography on silica gel (100 g, dichloromethane:methyl alcohol=96:4), followed by purification by HPLC to obtain a title compound (0.1 g).

Preparative Example 1. Synthesis of [N-methyl-D-norval$^3$]cyclosporine A

Using the general method, cyclosporine A (1.0 g) dissolved in tetrahydrofuran (THF) (50 mL) was added to 10 equivalents of an LDA solution at 78°C. Thereafter, the reaction mixture was stirred at 78°C for 2 hours, followed by addition of propyl iodide (0.41 ml). The mixture was brought to room temperature and further stirred for 24 hours, followed by addition of 20 mL of water and concentration of the mixture. Thereafter, ether ($Et_2O$) was added to the resulting residue, which, in turn, was washed sequentially with water and a saturated aqueous solution of sodium chloride, dried over anhydrous sodium sulfate ($MgSO_4$), and concentrated. Thereafter, the residue was purified by column chromatography on silica gel (100 g, dichloromethane:methyl alcohol=96:4), followed by purification by HPLC to obtain a title compound (0.12 g).

Preparative Example 3. Synthesis of [D-2-(methylamino)-hexa-4-ynoyl$^3$]cyclosporine A

Using the general method, cyclosporine A (1.0 g) dissolved in tetrahydrofuran (THF) (50 mL) was added to 10 equivalents of an LDA solution at 78°C. Thereafter, the reaction mixture was stirred at 78°C for 2 hours, followed by addition of 1-bromo-2-butyne (0.73 ml). The mixture was brought to room temperature and further stirred for 24 hours, followed by addition of 20 mL of water and concentration of the mixture. Thereafter, ether ($Et_2O$) was added to the resulting residue, which, in turn, was washed sequentially with water and a saturated aqueous solution of chloride solution, dried over anhydrous sodium sulfate ($MgSO_4$), and concentrated. Thereafter, the residue was purified by column chromatography on silica gel (100 g, dichloromethane:methyl alcohol=96:4), followed by purification by HPLC to obtain a title compound (0.13 g).

Preparative Example 4. Synthesis of [D-2-(methylamino)-pent-4-ynoyl$^3$]cyclosporine A

Using the general method, a reaction mixture was obtained using tetrahydrofuran (THF) (200 mL), diisopropylamine ((i-Pr)2NH) (3.2 mL), butyllithium (BuLi) (8 mL), cyclosporine A dissolved in tetrahydrofuran (THF) (50 mL), and propargyl bromide (3.57 g). The mixture was brought to room temperature and further stirred for 2 hours, followed by addition of 20 mL of water and concentration of the mixture. Thereafter, ether ($Et_2O$) was added to the resulting residue, which, in turn, was washed sequentially with water and a saturated aqueous solution of sodium chloride, dried over anhydrous sodium sulfate ($MgSO_4$), and concentrated. Thereafter, the residue was purified by column chromatography on silica gel (100 g, dichloromethane:methyl alcohol=96:4), followed by purification by HPLC to obtain a title compound.

Preparative Example 5. Synthesis of [D-2-methylthio-Sar$^3$]cyclosporine A.

Using the general method, a reaction mixture was obtained using tetrahydrofuran (THF) (100 mL), diisopropylamine ((i-Pr)2NH) (1.6 mL), butyllithium (BuLi) (4.0 mL), cyclosporine A (1.0 g) dissolved in tetrahydrofuran (THF) (30 mL), and methyl disulfide ($Me_2S_2$) (1.5 mL). The mixture was stirred at 0°C for 14 hours, followed by addition of 20 mL of water and concentration of the mixture. Thereafter, ether ($Et_2O$) was added to the resulting residue, which, in turn, was washed sequentially with water and a saturated aqueous solution of sodium chloride, dried over anhydrous sodium sulfate ($MgSO_4$), and concentrated. Thereafter, the residue was purified by column chromatography on silica gel (100 g, dichloromethane:methyl alcohol=50:1 to 96:4), followed by purification by HPLC to obtain a title compound (OND-1).

Preparative Example 6. Synthesis of [D-2-(4-methylbenzenethio)-Sar$^3$]cyclosporine A.

A title compound was obtained in the same manner as in Preparative Example 5 except that di(tol-4-yl) disulfide was used as an electrophile.

Preparative Example 7. Synthesis of [D-2-dimethylamino-ethylthio-Sar$^3$]cyclosporine A

A title compound was obtained in the same manner as in Preparative Example 5 except that bis(2-dimethylaminoethyl) disulfide was used as an electrophile.

Preparative Example 8. Synthesis of [N-methyl-D-Ser$^3$]cyclosporine A

Using the general method, cyclosporine A (1.0 g) dissolved in tetrahydrofuran (THF) (50 mL) was added to 10 equivalents of an LDA solution at 78°C. Thereafter, the reaction mixture was stirred at 78°C for 2 hours, followed by addition of paraformaldehyde (2.0 g). The mixture was brought to room temperature and further stirred for 24 hours, followed by addition of 20 mL of water and concentration of the mixture. Thereafter, ether ($Et_2O$) was added to the resulting residue, which, in turn, was washed sequentially with water and a saturated aqueous solution of sodium chloride, dried over anhydrous sodium sulfate ($MgSO_4$), and concentrated. Thereafter, the residue was purified by column chromatography on silica gel (100 g, dichloromethane:methyl alcohol=96:4), followed by purification by HPLC to obtain a title compound (0.3 g).

Preparative Example 9. Synthesis of [N-methyl-O-propenyl-D-Ser$^3$]cyclosporine A

Using the general method, [D-methylserine$^3$]cyclosporine A (0.62 g, 0.5 mmol), tetrabutylammonium chloride (0.11 g, 0.5 mmol), and aryl bromide (0.24 g, 2.0 mmol) were dissolved in dichloromethane (50 mL), followed by stirring for 2 hours together with 30% sodium hydroxide (NaOH) (1.5 mL), and then 50 mL of dichloromethane was added to the resulting mixture, which, in turn, was washed sequentially with water and a saturated sodium chloride solution, dried over anhydrous sodium sulfate ($MgSO_4$), and concentrated. Thereafter, the resulting residue was purified by column chromatography on silica gel (100 g, dichloromethane:methyl alcohol=97:3), followed by purification by HPLC to obtain a title compound (0.4 g).

Preparative Example 10. Synthesis of [N-methyl-D-Ala$^3$]cyclosporine A

Using the general method, a reaction mixture was obtained using tetrahydrofuran (THF) (120 mL), diisopropylamine ((i-Pr)2NH) (1.74 mL), butyllithium (BuLi) (4.6 mL), cyclosporine A (2.0 g) dissolved in tetrahydrofuran (THF) (30 mL), and methyl iodide (MeI) (0.51 mL). Thereafter, the mixture was brought to room temperature while stirring for 1 hour, followed by addition of 10 mL of water and concentration of the mixture. Thereafter, ether ($Et_2O$) was added to the resulting residue, which, in turn, was washed sequentially with water and a saturated aqueous solution of sodium chloride, dried over anhydrous sodium sulfate ($MgSO_4$), and concentrated. Thereafter, the residue was purified by column chromatography on silica gel (100 g, dichloromethane:methyl alcohol=50:1 to 96:4), followed by purification by HPLC to obtain a title compound (0.26 g).

Example 1. Preparation of sustained-release particles based on PLGA having various LA:GA molar ratios (vacuum drying)

Controlled-release PLGA particles of Examples 1-A to 1-C were prepared by the following method. Details of each component used in preparation of the PLGA particles and preparation conditions are shown in Table 1.

Step 1: OND-1 (40 mg) and PLGA (160 mg) were dissolved in dichloromethane (DCM) (2 ml) to prepare a dispersed phase. (See Preparative Example 5 for OND-1)

Step 2: The prepared dispersed phase was added to an aqueous solution of 1% PVA (500 mg PVA, 50 ml D.W.) as a continuous phase and emulsified using a homogenizer to prepare an emulsion.

Step 3: The prepared emulsion was stabilized in an aqueous solution of 1% PVA (2,500 mg PVA, 250 ml D.W.) as a quenching medium, followed by evaporation of DCM while stirring with a magnetic stirrer for at least 4 to 24 hours, thereby forming PLGA particles.

Step 4: The solution containing the PLGA particles was subjected to centrifugation (under conditions of 13,000 rpm, 10 min, and 4°C) to precipitate the PLGA particles, followed by removal of PVA from a supernatant.

Step 5: The precipitate of the PLGA particles was resuspended in 10 mL of distilled water (D.W.), followed by centrifugation (under conditions of 13,000 rpm, 10 min, and 4°C). This procedure was repeated four times.

Step 6: After completion of washing, the precipitate was vacuum-dried to obtain final PLGA particles, which, in turn, were stored.

[Table 1]

| Examples 1-A to 1-C | | A (#125) | B(#126) | C(#127) |
|---|---|---|---|---|
| Drug | OND-1 1) | 40 mg | | |
| Dispersed phase Solvent | DCM | 2 ml | | |
| Dispersed phase Polymer | Resomer RG502H (PLGA 50:50, acid, 7,000-17,000) 2) | 160 mg | - | - |
| | Resomer RG752H (PLGA 75:25, acid, 4,000-15,000) | - | 160 mg | - |
| | Lactel B6006-1 (PLGA 85:15, ester, 50,000-75,000) | - | - | 160 mg |
| Dispersed phase Additive | - | - | | |
| Continuous phase Purified water | D.W. | 50 ml | | |
| Continuous phase High molecular weight surfactant | PVA | 500 mg | | |
| Continuous phase Additive | - | - | | |
| Quenching medium | Aqueous solution of 1% | 250 ml | | |
| | PVA | | | |
| Emulsification | Homogenizer | 5000 rpm, 8 min | | |
| | Sonicator | - | | |
| Drying | Vacuum drying | 25°C, 48 hr | | |
| | Freeze-drying | - | | |

1) [D-2-methylthio-Sar$^3$]cyclosporine A

2) The numbers in parentheses indicate LA:GA molar ratio, end group, and molecular weight, in that order. For example, "(PLGA 50:50, acid, 7,000-17,000)" represents PLGA having an LA:GA molar ratio of 50:50, a carboxylic acid as an end group, and a molecular weight of 7,000 to 17,000 (the same applies hereinafter).

Example 2. Preparation of sustained-release particles based on PLGA having various LA:GA molar ratios (freeze-drying)

Controlled-release PLGA particles of Examples 2-A to 2-C were prepared in the same manner as in Example 1 except that, in step 6, a freeze-drying process (freeze-drying after dispersion in an aqueous solution of 1% mannitol) was used instead of the vacuum drying process. Details of each component used in preparation of the PLGA particles

and preparation conditions are shown in Table 2.

[Table 2]

| Examples 2-A to 2-C | | A (#109) | B(#110) | C(#111) |
|---|---|---|---|---|
| Drug | OND-1 | 40 mg | | |
| Dispersed phase Solvent | DCM | 2 ml | | |
| Dispersed phase Polymer | Resomer RG502H (PLGA 50:50, acid, 7,000-17,000) | 160 mg | - | - |
| | Resomer RG752H (PLGA 75:25, acid, 4,000-15,000) | - | 160 mg | - |
| | Lactel B6006-1(PLGA 85:15, ester, 50000-75000) | - | - | 160 mg |
| Dispersed phase Additive | - | - | | |
| Continuous phase Purified water | D.W. | 50 ml | | |
| Continuous phase high molecular weight surfactant | PVA | 500 mg | | |
| Continuous phase Additive | - | - | | |
| Quenching medium | Aqueous solution of 1% | 250 ml | | |
| | PVA | | | |
| Emulsification | Homogenizer | 5000 rpm, 8 min | | |
| | Sonicator | - | | |
| Drying | Vacuum drying | - | | |
| | Freeze-drying (with 10 mL of aqueous solution of 1% mannitol) | -40°C, 48 hr | | |

Example 3. Preparation of sustained-release particles based on PLGA having an LA:GA molar ratio of 50:50 and a different end group or molecular weight

Controlled-release PLGA particles of Examples 3-A to 3-C were prepared in the same manner as in Example 1 except that PLGA polymers having an LA:GA molar ratio of 50:50 and a different end group or molecular weight than the PLGA polymers of Example 1 were used in the dispersed phase. Details of each component used in preparation of the PLGA particles and preparation conditions are shown in Table 3.

[Table 3]

| Examples 3-A to 3-C | | | A (#128) | B (#129) | C (#130) |
|---|---|---|---|---|---|
| Dispersed phase | Polymer | Resomer RG502 (PLGA 50:50, ester, 7,000-17,000) | 160 mg | - | - |
| | | Resomer RG504H (PLGA 50:50, acid, 3,8000-54,000) | - | 160 mg | - |
| | | Resomer RG504 (PLGA 50:50, ester, 38,000-54,000) | - | - | 160 mg |

Example 4. Preparation of sustained-release particles based on PLGA polymers having an LA:GA molar ratio of 75:25 and a different end group or molecular weight

Controlled-release PLGA particles of Examples 4-A to 4-C were prepared in the same manner as in Example 1 except that PLGA polymers having an LA:GA molar ratio of 75:25 and a different end group or molecular weight than the PLGA polymers of Example 1 were used in the dispersed phase. Details of each component used in preparation of the PLGA particles and preparation conditions are shown in Table 4.

[Table 4]

| Examples 4-A to 4-C | | | A (#132) | B(#133) | C(#134) |
|---|---|---|---|---|---|
| Dispersed phase | Polymer | Resomer RG752S (PLGA 75:25, ester, 4,000-15,000) | 160 mg | - | - |
| | | Resomer RG753H (PLGA 75:25, acid, 38,000-54000) | - | 160 mg | - |
| | | Resomer RG753S (PLGA 75:25, ester, 38000-54000) | - | - | 160 mg |

Example 4-1. Preparation of PLGA-based sustained-release particles under changed emulsification conditions

Controlled-release particles of Examples 4-1-A to 4-1-D were prepared in the same manner as in Example 1-B or Example 2-B except that emulsification conditions were changed. Details of each component used in preparation of the particles and preparation conditions are shown in Table 5.

[Table 5]

| Examples 4-1-A to 4-1-D | | | A (#122) | B (#108) | C (#73) | D (#73-1) |
|---|---|---|---|---|---|---|
| Dispersed phase | Solvent | DCM | 2 ml | 2 ml | 2 ml | 2 ml |
| | Polymer | Resomer RG752H (PLGA 75:25, acid, 4,000-15,000) | 160 mg | 160 mg | 160 mg | 100 mg |
| Emulsification | | Homogenizer | 15,000 rpm, 5 min | 15,000 rpm, 5 min | 5,000 rpm, 8 min | 5,000 rpm, 5 min |
| | | Sonicator | - | - | - | - |
| Drying | | Vacuum drying | - | 25°C 48 hr | - | 25°C 48 hr |
| | | Freeze-drying | -40°C 48 hr | - | -40°C 48 hr | - |

Example 5. Preparation of sustained-release particles based on PLGA having an LA:GA molar ratio of 85:15 and a different molecular weight

Controlled-release particles of Example 5-A were prepared in the same manner as in Example 1 except that PLGA in the dispersed phase was changed. Details of each component used in preparation of the particles and preparation conditions are shown in Table 6.

[Table 6]

| Example 5 | | | A (#131) |
|---|---|---|---|
| Dispersed phase | Polymer | Resomer LG858S (PLGA 85:15, ester, 190,000-240,000) | 160 mg |

Example 6: Preparation of PLGA-based sustained-release particles using a solvent having a different interfacial tension (IFT) to water as a solvent in a dispersed phase

Controlled-release PLGA particles of Example 6-A to 6-J were prepared in the same manner as in Example 1-B except that the solvent in the dispersed phase was changed to a solvent having a different interfacial tension (IFT) to water. Details of each component used in preparation of the particles and preparation conditions are shown in Tables 7 and 8. The terms used in Tables 7 and 8 are as follows: DCM=dichloromethane; EA=ethyl acetate; DMSO=dimethyl sulfoxide; and ACN=acetonitrile.

[Table 7]

| Examples 6-A to 6-E | | | A | B | C | D | E |
|---|---|---|---|---|---|---|---|
| Dispersed phase | Solvent (IFT value to aqueous phase) | DCM (20.4) | 2 ml | - | - | - | - |
| | | Chloroform (31.4) | - | 2 ml | - | - | - |
| | | EA (6.78) | - | - | 2 ml | - | - |
| | | Ethyl formate | - | - | - | 2 ml | - |
| | | DMSO | | | | | 2 ml |
| | Polymer | Resomer RG752H (PLGA 75:25, acid, 4,000-15,000) | 160 mg | | | | |

[Table 8]

| Examples 6-F to 6-J | | | F | G | H | I | J |
|---|---|---|---|---|---|---|---|
| Dispersed phase | Solvent (IFT value to aqueous phase) | ACN | 2 ml | - | - | - | - |
| | | Butyl acetate | - | 2 ml | - | - | - |
| | | Isopropyl formate | - | - | 2 ml | - | - |
| | | Glycofurol | - | - | - | 2 ml | - |
| | | DCM (20.4) + Chloroform (31.4) | | | | | 0.4 ml + 1.6 ml |
| | Polymer | Resomer RG752H (PLGA 75:25, acid, 4,000-15,000) | 160 mg | | | | |

Example 7. Preparation of PLGA-based sustained-release particles using a solvent having a different interfacial tension (IFT) to water

Controlled-release PLGA particles of Example 7-A to 7-J were prepared in the same manner as in Example 1-A except that the solvent in the dispersed phase was changed to a solvent having a different interfacial tension (IFT) to water. Details of each component used in preparation of the particles and preparation conditions are shown in Tables 9 and 10.

[Table 9]

| Examples 7-A to 7-E | | | A | B | C | D | E |
|---|---|---|---|---|---|---|---|
| Dispersed phase | Solvent (IFT value to aqueous phase) | DCM (20.4) | 2 ml | - | - | - | - |
| | | Chloroform (31.4) | - | 2 ml | - | - | - |
| | | EA (6.78) | - | - | 2 ml | - | - |
| | | Ethyl formate | - | - | - | 2 ml | - |
| | | DMSO | - | - | - | - | 2 ml |
| | Polymer | Resomer RG502H (PLGA 50:50, acid, 7,000-17,000) | 160 mg | | | | |

[Table 10]

| Examples 7-F to 7-J | | | F | G | H | I | J |
|---|---|---|---|---|---|---|---|
| Dispersed phase | Solvent (IFT value to aqueous phase) | ACN | 2 ml | - | - | - | - |
| | | Butyl acetate | - | 2 ml | - | - | - |
| | | Isopropyl formate | - | - | 2 ml | - | - |
| | | Glycofurol | - | - | - | 2 ml | - |
| | | DCM (20.4) + Chloroform(31.4) | | | | | 0.4 ml + 1.6 ml |
| | Polymer | Resomer RG502H (PLGA 50:50, acid, 7,000-17,000) | 160 mg | | | | |

Example 8. Preparation of PLGA-based sustained-release particles using a solvent having a different interfacial tension (IFT) to water

Controlled-release PLGA particles of Example 8-A to 8-J were prepared in the same manner as in Example 1-C except that the solvent in the dispersed phase was changed to a solvent having a different interfacial tension (IFT) to water. Details of each component used in preparation of the particles and preparation conditions are shown in Tables 11 and 12.

[Table 11]

| Examples 8-A to 8-E | | | A | B | C | D | E |
|---|---|---|---|---|---|---|---|
| Dispersed phase | Solvent (IFT value to aqueous phase) | DCM (20.4) | 2 ml | - | - | - | - |
| | | Chloroform (31.4) | - | 2 ml | - | - | - |
| | | EA (6.78) | - | - | 2 ml | - | - |
| | | Ethyl formate | - | - | - | 2 ml | - |
| | | DMSO | - | - | - | - | 2 ml |
| | Polymer | Lactel B6006-1 (PLGA 85.15, ester, 50,000-750,000) | 160 mg | | | | |

[Table 12]

| Examples 8-F to 8-J | | | F | G | H | I | J |
|---|---|---|---|---|---|---|---|
| Dispersed phase | Solvent (IFT value to aqueous phase) | ACN | 2 ml | - | - | - | - |
| | | Butyl acetate | - | 2 ml | - | - | - |
| | | Isopropyl formate | - | - | 2 ml | - | - |
| | | DCM (20.4) + Chloroform(31.4) | | | | | 0.4 ml + 1.6 ml |
| | Polymer | Lactel B6006-1 (PLGA 85.15, ester, 50,000-750,000) | 160 mg | | | | |

Example 9. Preparation of PLGA-based sustained release particles by mixing a co-solvent with a dispersed phase solvent

Controlled release PLGA particles of Example 9-A to 9-I were prepared in the same manner as in Example 1-B except that methanol, ethanol, acetone, isopropanol, diethyl ether, chloroform, ethyl acetate (EA), DMSO, or CAN as a co-solvent was mixed with DCM, chloroform, ethyl acetate (EA), ethyl formate, DMSO, ACN, butyl acetate, isopropyl formate, or glycofurol as a dispersed phase solvent. Details of each component used in preparation of the particles and preparation conditions are shown in Tables 13 and 14.

[Table 13]

| Examples 9-A to 9-E | | | A | B | C | D | E |
|---|---|---|---|---|---|---|---|
| Dispersed phase | Solvent (IFT value to aqueous phase) | DCM (20.4) | 1.2 ml | - | - | - | - |
| | | Chloroform (31.4) | - | 1.2 ml | - | - | - |
| | | EA (6.78) | - | - | 1.2 ml | - | - |
| | | Ethyl formate | - | - | - | 1.2 ml | - |
| | | DMSO | - | - | - | - | 1.2 ml |
| | Co-solvent (IFT value to aqueous phase) | Methanol | 0.8 ml | - | - | - | - |
| | | Ethanol | - | 0.8 ml | - | - | - |
| | | Acetone | - | - | 0.8 ml | - | - |
| | | Isopropanol | - | - | - | 0.8 ml | - |
| | | Diethyl ether | - | - | - | - | 0.8 ml |
| | Polymer | Resomer RG752H (PLGA 75:25, acid, 4000-15000) | 160 mg | | | | |

[Table 14]

| Examples 9-F to 9-I | | | F | G | H | I |
|---|---|---|---|---|---|---|
| Dispersed phase | Solvent (IFT value to aqueous phase) | ACN | 1.2 ml | - | - | - |
| | | Butyl acetate | - | 1.2 ml | | - |
| | | Isopropyl formate | - | - | 1.2 ml | - |
| | | Glycofurol | - | - | - | 1.2 ml |
| | Co-solvent (IFT value to aqueous phase) | Chloroform | 0.8 ml | - | - | - |
| | | Ethyl acetate | - | 0.8 ml | - | - |
| | | DMSO | - | - | 0.8 ml | - |
| | | ACN | - | - | - | 0.8 ml |
| | Polymer | Resomer RG752H (PLGA 75:25, acid, 4000-15000) | 160 mg | | | |

Example 10. Preparation of natural polymer-based sustained-release particles

Controlled-release PLGA particles of Examples 10-A to 10-I were prepared in the same manner as in Example 1 except that natural polymers were used instead of the PLGA polymers of Example 1. Details of each component used in preparation of the particles and preparation conditions are shown in Table 15. In Table 15, CMC is carboxymethylcellulose.

[Table 15]

| Examples 10-A to 10-I | | | A | B | C | D | E | F | G | H | I |
|---|---|---|---|---|---|---|---|---|---|---|---|
| Dispersed phase | Polymer | Starch | 160 mg | - | - | - | - | - | - | - | - |
| | | Chitosan | - | 160 mg | - | - | - | - | - | - | - |
| | | Dextran | - | - | 160 mg | - | - | - | - | - | - |
| | | Hyaluronic acid | - | - | - | 160 mg | - | - | - | - | - |
| | | Albumin | - | - | - | - | 160 mg | - | - | - | - |
| | | Alginate | - | - | - | - | - | 160 mg | - | - | - |
| | | Collagen | - | - | - | - | - | - | 160 mg | - | - |
| | | Gelatin | - | - | - | - | - | - | - | 160 mg | - |
| | | CMC | - | - | - | - | - | - | - | - | 160 mg |

Example 11. Preparation of synthetic biodegradable polymer (aliphatic homopolymer)-based sustained-release particles

Controlled-release PLGA particles of Examples 11-A to 11-1 were prepared in the same manner as in Example 1 except that synthetic biodegradable polymers (aliphatic homopolymers) were used instead of the PLGA polymers of Example 1. Details of each component used in preparation of the particles and preparation conditions are shown in Table 16. In Table 16, PCL is poly-ε-caprolactone; PLA is poly(lactic acid); PGA is polyglycolic acid; PHA is polyhydroxyalkanoate; PHB is polyhydroxybutyrate; and PEG is polyethyleneglycol.

[Table 16]

| Examples 11-A to 11-I | | | A | B | C | D | E | F | G | H | I |
|---|---|---|---|---|---|---|---|---|---|---|---|
| Dispersed phase | Polymer (aliphatic homopolymer) | PCL | 160 mg | - | - | - | - | - | - | - | - |
| | | PLA | - | 160 mg | - | - | - | - | - | - | - |
| | | PGA | - | - | 160 mg | - | - | - | - | - | - |
| | | PHA | - | - | - | 160 mg | - | - | - | - | - |
| | | PHB | - | - | - | - | 160 mg | - | - | - | - |
| | | PEG | - | - | - | - | - | 160 mg | - | - | - |
| | | Polyalkylcarbonate | - | - | - | - | - | - | 160 mg | - | - |
| | | Polyamino acid | - | - | - | - | - | - | - | 160 mg | - |
| | | Polyorthoester | - | - | - | - | - | - | - | - | 160 mg |

Example 12. Preparation of synthetic biodegradable polymer (aliphatic copolymer)-based sustained-release particles

Controlled-release PLGA particles of Examples 12-A to 12-I were prepared in the same manner as in Example 1 except that synthetic biodegradable polymers (aliphatic copolymers) were used instead of the PLGA polymers of Example 1. Details of each component used in preparation of the particles and preparation conditions are shown in Table 17. The terms In Table 17 are as follows: PEA=polyethylene adipate; PBS=polybutylene succinate; PHBV=poly(3-hydroxybutyrate-co-3-hydroxyvalerate); PET=polyethylene terephthalate; PLGA=polylactide-co-glycolide; PLGA-glucose=polylactide-co-glycolide-glucose; MPEG-(PCL-co-PLLA)=methoxy polyethylene glycol-(polycaprolactone-co-polylactide); mPEG-b-PLGA=poly(ethylene glycol) methyl ether-block-poly(lactide-co-glycolide); and PLGA-b-PEG-b-PLGA=poly(lactide-co-glycolide)-block-poly(ethylene glycol)-block-poly(lactide-co-glycolide).

[Table 17]

| Examples 12-A to 12-I | | | A | B | C | D | E | F | G | H | I |
|---|---|---|---|---|---|---|---|---|---|---|---|
| Dispersed phase | Polymer (aliphatic copolymer) | PEA | 160 mg | - | - | - | - | - | - | - | - |
| | | PBS | - | 160 mg | - | - | - | - | - | - | - |
| | | PHBV | - | - | 160 mg | - | - | - | - | - | - |
| | | PET | - | - | - | 160 mg | - | - | - | - | - |
| | | PLGA (same as Example 1-B) | - | - | - | - | 160 mg | - | - | - | - |
| | | PLGA-glucose | - | - | - | - | - | 160 mg | - | - | - |
| | | MPEG-(PCL-co-PLLA) | - | - | - | - | - | - | 160 mg | - | - |
| | | mPEG-b-PLGA | - | - | - | - | - | - | - | 160 mg | - |
| | | PLGA-b-PEG-b-PLGA | - | - | - | - | - | - | - | - | 160 mg |

Example 13. Preparation of synthetic biodegradable polymer (semi-aromatic or aromatic copolymer)-based sustained-release particles

Controlled-release PLGA particles of Examples 13-A to 13-D were prepared in the same manner as in Example 1 except that synthetic biodegradable polymers (semi-aromatic or aromatic copolymers) were used instead of the PLGA polymers of Example 1. Details of each component used in preparation of the particles and preparation conditions are shown in Table 18. The terms in Table 18 are as follows: PBT=polybutylene terephthalate; PTT=polytrimethylene terephthalate; and PEN=polyethylene naphthalate.

[Table 181]

| Examples 13-A to 13-D | | | A | B | C | D |
|---|---|---|---|---|---|---|
| Dispersed phase | Polymer (Semi-aromatic Copolymer) | PBT | 160 mg | - | - | - |
| | | PTT | - | 160 mg | - | - |
| | | PEN | - | - | 160 mg | - |
| | Polymer (Aromatic Copolymer) | Vectran | - | - | - | 160 mg |

Example 14. Preparation of PLGA-based sustained-release particles by changing the type of surfactant in the continuous phase

Controlled-release PLGA particles of Example 14-A to 14-H were prepared in the same manner as in Example 1-B except that the continuous phase of Example 1-B was changed to an aqueous solution of 1% (w/v) PVA, PVP, HPMC, PEG, Eudragit, chitosan, methyl cellulose, or gelatin (500 mg of each surfactant and 50 ml of D.W.). Details of each component used in preparation of the particles and preparation conditions are shown in Tables 19 and 20.

[Table 191

| Examples 14-A to 14-D | | A | B | C | D |
|---|---|---|---|---|---|
| Dispersed phase Polymer | Resomer RG752H (PLGA 75:25, acid, 4,000-15,000) | 160 mg | | | |
| Continuous Phase Purified water | D.W. | 50 ml | | | |
| Continuous phase High molecular weight surfactant | PVA | 500 mg | - | - | - |
| | PVP | - | 500 mg | - | - |
| | HPMC | - | - | 500 mg | - |
| | PEG | - | - | - | 500 mg |
| Continuous phase Additive | - | - | - | - | - |

[Table 20]

| Examples 14-E to 14-H | | E | F | G | H |
|---|---|---|---|---|---|
| Dispersed phase Polymer | Resomer RG752H (PLGA 75:25, acid, 4,000-15,000) | 160 mg | | | |
| Continuous phase Purified water | D.W. | 50 ml | | | |
| Continuous Phase High molecular weight surfactant | Eudragit | 500 mg | - | - | - |
| | Chitosan | - | 500 mg | - | - |
| | Methyl Cellulose | - | - | 500 mg | - |
| | Gelatin | - | - | - | 500 mg |
| Continuous phase Additive | - | - | - | - | - |

Example 15. Preparation of PLGA-based sustained-release particles by changing the type of surfactant in the continuous phase

Controlled-release PLGA particles of Example 15-A to 15-H were prepared in the same manner as in Example 1-A except that the continuous phase of Example 1-A was changed to an aqueous solution of 1% (w/v) PVA, PVP, HPMC, PEG, Eudragit, chitosan, methyl cellulose, or gelatin (500 mg of each surfactant and 50 ml of D.W.). Details of each component used in preparation of the particles and preparation conditions are shown in Table 21.

[Table 21]

| Examples 15-A to 15-H | | A | B | C | D | E | F | G | H |
|---|---|---|---|---|---|---|---|---|---|
| Dispersed phase Solvent | DCM | 2 ml | | | | | | | |
| Dispersed phase Polymer | Resomer RG502H (PLGA 50:50, acid, 7,000-17,000) | 160 mg | | | | | | | |
| Continuous-phase Purified water | D.W. | 50 ml | | | | | | | |
| Continuous-phase High molecular weight surfactant | PVA | 500 mg | - | - | - | - | - | - | - |
| | PVP | - | 500 mg | - | - | - | - | - | - |
| | HPMC | - | - | 500 mg | - | - | - | - | - |
| | PEG | - | - | - | 500 mg | - | - | - | - |
| | Eudragit | - | - | - | - | 500 mg | - | - | - |
| | Chitosan | - | - | - | - | - | 500 mg | - | - |
| | Methyl Cellulose | - | - | - | - | - | - | 500 mg | - |
| | Gelatin | - | - | - | - | - | - | - | 500 mg |
| Continuous phase Additive | - | - | - | - | - | - | - | - | - |

Example 16. Preparation of PLGA-based sustained-release particles by changing the type of surfactant in the continuous phase

Controlled-release PLGA particles of Example 16-A to 16-H were prepared in the same manner as in Example 1-C except that the continuous phase of Example 1-C was changed to an aqueous solution of 1% (w/v) PVA, PVP, HPMC, PEG, Eudragit, chitosan, methyl cellulose, or gelatin (500 mg of each surfactant and 50 ml of D.W.). Details of each component used in preparation of the particles and preparation conditions are shown in Table 22.

[Table 22]

| Examples 16-A to 6-H | | A | B | C | D | E | F | G | H |
|---|---|---|---|---|---|---|---|---|---|
| Dispersed phase Solvent | DCM | 2 ml | | | | | | | |
| Dispersed phase Polymer | Lactel B6006-1 (PLGA 85: 15, ester, 50,000-75,000) | 160 mg | | | | | | | |
| Continuous phase Purified water | D.W. | 50 ml | | | | | | | |

(continued)

| Examples 16-A to 6-H | | A | B | C | D | E | F | G | H |
|---|---|---|---|---|---|---|---|---|---|
| Continuous phase High molecular weight surfactant | PVA | 500 | - | - | - | - | - | - | - |
| | PVP | - | 500 mg | - | - | - | - | - | - |
| | HPMC | - | - | 500 mg | - | - | - | - | - |
| | PEG | - | - | - | 500 mg | - | - | - | - |
| | Eudragit | - | - | - | - | 500 mg | - | - | - |
| | Chitosan | - | - | - | - | - | 500 mg | - | - |
| | Methyl Cellulose | - | - | - | - | - | - | 500 mg | - |
| | Gelatin | - | - | - | - | - | - | - | 500 mg |

Example 17. Preparation of PLGA-based sustained-release particles using a hydrophilic low-molecular weight surfactant (emulsifier or emulsification stabilizer) in the continuous phase

Controlled-release PLGA particles of Examples 17-A to 17-J were prepared in the same manner as in Example 1-B except that, in step 2, the surfactant in the continuous phase was changed to an aqueous solution of 1% (w/v) lecithin, poloxamer, tween, Cremophor, SLS, sodium stearate, ethylene diamine, Mrij 52, Brij 58, or cyclodextrin (500 mg of each hydrophilic low-molecular weight surfactant and 50 ml of D.W.). Details of each component used in preparation of the particles and preparation conditions are shown in Tables 23 and 24. In Table 23, SLS is sodium lauryl sulfate.

[Table 23]

| Examples 17-A to 17-E | | A | B | C | D | E |
|---|---|---|---|---|---|---|
| Dispersed phase Solvent | DCM | 2 ml | | | | |
| Dispersed phase Polymer | Resomer RG752H (PLGA 75:25, acid, 4,000-15,000) | 160 mg | | | | |
| Continuous-phase Purified water | D.W. | 50 ml | | | | |
| Continuous-phase Low-molecular weight surfactant | Lecithin | 500 mg | - | - | - | - |
| | Poloxamer | - | 500 mg | - | - | - |
| | Tween | - | - | 500 mg | - | - |
| | Cremophor | - | - | - | 500 mg | - |
| | SLS | - | - | - | - | 500 mg |

[Table 24]

| Examples 17-F to 17-J | | F | G | H | I | J |
|---|---|---|---|---|---|---|
| Dispersed phase Solvent | DCM | 2 ml | | | | |
| Dispersed phase Polymer | Resomer RG752H (PLGA 75:25, acid, 4,000-15,000) | 160 mg | | | | |
| Continuous phase Purified water | D.W. | 50 ml | | | | |
| Continuous-phase | Sodium Stearate | 500 mg | - | - | - | - |
| | Ethylene diamine | - | 500 mg | - | - | - |
| Hydrophilic low-molecular weight surfactant | Mrij 52 | - | - | 500 mg | - | - |
| | Brij 58 | - | - | - | 500 mg | - |
| | Cyclodextrins | - | - | - | - | 500 mg |

Example 18. Preparation of PLGA-based sustained-release particles using a hydrophilic low-molecular weight surfactant (emulsifier or emulsification stabilizer) in the continuous phase

Controlled-release PLGA particles of Examples 18-A to 18-J were prepared in the same manner as in Example 1-A except that, in step 2, the surfactant in the continuous phase was changed to an aqueous solution of 1% (w/v) lecithin, poloxamer, tween, Cremophor, SLS, sodium stearate, ethylene diamine, Mrij 52, Brij 58, or cyclodextrin (500 mg of each hydrophilic low-molecular weight surfactant and 50 ml of D.W.). Details of each component used in preparation of the particles and preparation conditions are shown in Table 25.

[Table 25]

| Examples 16-A to 16-J | | A | B | C | D | E | F | G | H | I | J |
|---|---|---|---|---|---|---|---|---|---|---|---|
| Dispersed phase Solvent | DCM | 2 ml | | | | | | | | | |
| Dispersed phase Polymer | Resomer RG502H (PLGA 50:50, acid, 7,000-17,000) | 160 mg | | | | | | | | | |
| Continuous phase Purified water | D.W. | 50 ml | | | | | | | | | |

(continued)

| Examples 16-A to 16-J | | A | B | C | D | E | F | G | H | I | J |
|---|---|---|---|---|---|---|---|---|---|---|---|
| Continuous phase Hydrophilic low-molecular weight surfactant | Lecithin | 500 mg | - | - | - | - | - | - | - | - | - |
| | Poloxamer | - | 500 mg | - | - | - | - | - | - | - | - |
| | Tween | - | - | 500 mg | - | - | - | - | - | - | - |
| | Cremophor | - | - | - | 500 mg | - | - | - | - | - | - |
| | SLS | - | - | - | - | 500 mg | - | - | - | - | - |
| | Sodium Stearate | - | - | - | - | - | 500 mg | - | - | - | - |
| | Ethylene diamine | - | - | - | - | - | - | 500 mg | - | - | - |
| | Mrij 52 | - | - | - | - | - | - | - | 500 mg | - | - |
| | Brij 58 | - | - | - | - | - | - | - | - | 500 mg | - |
| | Cyclodextrins | - | - | - | - | - | - | - | - | - | 500 mg |

Example 19. Preparation of PLGA-based sustained-release particles using a hydrophilic low-molecular weight surfactant (emulsifier or emulsification stabilizer) in the continuous phase

Controlled-release PLGA particles of Examples 19-A to 19-J were prepared in the same manner as in Example 1-C except that, in step 2, the surfactant in the continuous phase was changed to an aqueous solution of 1% (w/v) lecithin, poloxamer, tween, cremophor, SLS, sodium stearate, ethylene diamine, Mrij 52, Brij 58, or cyclodextrin (500 mg of each hydrophilic low-molecular weight surfactant and 50 ml of D.W.). Details of each component used in preparation of the particles and preparation conditions are shown in Table 26.

[Table 26]

| Examples 19-A to 19-J | | A | B | C | D | E | F | G | H | I | J |
|---|---|---|---|---|---|---|---|---|---|---|---|
| Dispersed phase Solvent | DCM | 2 ml | | | | | | | | | |
| Dispersed phase Polymer | Lactel B6006-1 (PLGA 85:15, ester, 50,000-75,000) | 160 mg | | | | | | | | | |
| Continuous phase Purified water | D.W. | 50 ml | | | | | | | | | |

(continued)

| Examples 19-A to 19-J | | A | B | C | D | E | F | G | H | I | J |
|---|---|---|---|---|---|---|---|---|---|---|---|
| Continuous phase Hydrophilic low-molecular weight surfactant | Lecithin | 500 mg | - | - | - | - | - | - | - | - | - |
| | Poloxamer | - | 500 mg | - | - | - | - | - | - | - | - |
| | Tween | - | - | 500 mg | - | - | - | - | - | - | - |
| | Cremophor | - | - | - | 500 mg | - | - | - | - | - | - |
| | SLS | - | - | - | - | 500 mg | - | - | - | - | - |
| | Sodium stearate | - | - | - | - | - | 500 mg | - | - | - | - |
| | Ethylene diamine | - | - | - | - | - | - | 500 mg | - | - | - |
| | Mrij 52 | - | - | - | - | - | - | - | 500 mg | - | - |
| | Brij 58 | - | - | - | - | - | - | - | - | 500 mg | - |
| | Cyclodextrins | - | - | - | - | - | - | - | - | - | 500 mg |

Example 20. Preparation of PLGA-based sustained-release particles using a lipophilic low-molecular weight surfactant (emulsifier or emulsification stabilizer) in the continuous phase

Controlled-release PLGA particles of Examples 20-A to 20-E were prepared in the same manner as in Example 1-B except that, in step 2, the surfactant in the continuous phase was changed to an aqueous solution of 1% span, Labrasol, Rheodol MO-60, glycerol monooleate, or triacetin (500 mg of each lipophilic low-molecular weight surfactant and 50 ml of D.W.). Details of each component used in preparation of the particles and preparation conditions are shown in Table 27.

[Table 27]

| Examples 20-A to 20-E | | A | B | C | D | E |
|---|---|---|---|---|---|---|
| Dispersed phase Solvent | DCM | 2 ml | | | | |
| Dispersed phase Polymer | Resomer RG752H (PLGA 75:25, acid, 4,000-15,000) | 160 mg | | | | |
| Continuous phase Purified water | D.W. | 50 ml | | | | |
| Continuous phase Lipophilic low-molecular weight surfactant | Span | 500 mg | - | - | - | - |
| | Labrasol | - | 500 mg | - | - | - |
| | Rheodol MO-60 | - | - | 500 mg | - | - |
| | Glycerol monooleate | - | - | - | 500 mg | - |
| | Triacetin | - | - | - | - | 500 mg |

Example 21. Preparation of PLGA-based sustained-release particles using a lipophilic low-molecular weight surfactant (emulsifier or emulsification stabilizer) in the continuous phase

Controlled-release PLGA particles of Examples 21-A to 21-E were prepared in the same manner as in Example 1-A except that, in step 2, the surfactant in the continuous phase was changed to an aqueous solution of 1% span, Labrasol, Rheodol MO-60, glycerol monooleate, or triacetin (500 mg of each lipophilic low-molecular weight surfactant and 50 ml of D.W.). Details of each component used in preparation of the particles and preparation conditions are shown in Table 28.

[Table 28]

| Examples 21-A to 21-E | | A | B | C | D | E |
|---|---|---|---|---|---|---|
| Drug | OND-1 | 40 mg | | | | |
| Dispersed phase Solvent | DCM | 2 ml | | | | |
| Dispersed phase Polymer | Resomer RG502H (PLGA 50:50, acid, 7,000-17,000) | 160 mg | | | | |
| Continuous phase Purified water | D.W. | 50 ml | | | | |
| Continuous phase Lipophilic low-molecular weight surfactant | Span | 500 mg | - | - | - | - |
| | Labrasol | - | 500 mg | - | - | - |
| | Rheodol MO-60 | - | - | 500 mg | - | - |
| | Glycerol monooleate | - | - | - | 500 mg | - |
| | triacetin | - | - | - | - | 500 mg |
| Continuous phase Additive | - | - | - | - | - | - |

Example 22. Preparation of PLGA-based sustained-release particles using a lipophilic low-molecular weight surfactant (emulsifier or emulsification stabilizer) in the continuous phase

Controlled-release PLGA particles of Examples 22-A to 22-E were prepared in the same manner as in Example 1-C except that, in step 2, the surfactant in the continuous phase was changed to an aqueous solution of 1% span, Labrasol, Rheodol MO-60, glycerol monooleate, or triacetin (500 mg of each lipophilic low-molecular weight surfactant and 50 ml of D.W.). Details of each component used in preparation of the particles and preparation conditions are shown in Table 29.

[Table 29]

| Examples 22-A to 22-E | | A | B | C | D | E |
|---|---|---|---|---|---|---|
| Drug | OND-1 | 40 mg | | | | |
| Dispersed phase Solvent | DCM | 2 ml | | | | |
| Dispersed phase Polymer | Lactel B6006-1 (PLGA 85:15, ester, 50,000-75,000) | 160 mg | | | | |
| Continuous Phase Purified water | D.W. | 50 ml | | | | |

(continued)

| Examples 22-A to 22-E | | A | B | C | D | E |
|---|---|---|---|---|---|---|
| Continuous phase Lipophilic low-molecular weight surfactant | Span | 500 mg | - | - | - | - |
| | Labrasol | - | 500 mg | - | - | - |
| | Rheodol MO-60 | - | - | 500 mg | - | - |
| | Glycerol monooleate | - | - | - | 500 mg | - |
| | Triacetin | - | - | - | - | 500 mg |

Example 23. Preparation of PLGA-based sustained-release particles by adding a modifier (additive, low-molecular weight surfactant) to the dispersed phase

Controlled-release PLGA particles of Examples 23-A to 23-G were prepared in the same manner as in Example 1-B except that, in step 2, 100 mg of tween 80, span 20, PEG 400, Mrij 52, Brij 58, poloxamer P124 or SLS was added as a modifier (additive) to the dispersed phase. Details of each component used in preparation of the particles and preparation conditions are shown in Tables 30 and 31.

[Table 30]

| Examples 23-A to 23-D | | A | B | C | D |
|---|---|---|---|---|---|
| Dispersed phase Solvent | DCM | 2 ml | | | |
| Dispersed phase Polymer | Resomer RG752H (PLGA 75:25, acid, 4,000-15,000) | 160 mg | | | |
| Dispersed phase Additive | Tween 80 | 100 mg | - | - | - |
| | Span 20 | - | 100 mg | - | - |
| | PEG 400 | - | - | 100 mg | - |
| | Mrij 52 | - | - | - | 100 mg |

[Table 31]

| Examples 23-E to 23-G | | E | F | G |
|---|---|---|---|---|
| Dispersed phase Solvent | DCM | 2 ml | | |
| Dispersed phase Polymer | Resomer RG752H (PLGA 75:25, acid, 4,000-15,000) | 160 mg | | |
| Dispersed phase Additive | Brij 58 | 100 mg | - | - |
| | Poloxamer P124 | - | 100 mg | - |
| | SLS | - | - | 100 mg |

Example 24. Preparation of PLGA-based sustained-release particles by adding a modifier (additive, low-molecular weight surfactant) to the dispersed phase

Controlled-release PLGA particles of Examples 24-A to 24-G were prepared in the same manner as in Example 1-A except that, in step 2, 100 mg of tween 80, span 20, PEG 400, Mrij 52, Brij 58, poloxamer P124 or SLS was added as a modifier (additive) to the dispersed phase. Details of each component used in preparation of the particles and

preparation conditions are shown in Tables 32 and 33.

[Table 32]

| Examples 24-A to 24-D | | A | B | C | D |
|---|---|---|---|---|---|
| Dispersed phase Solvent | DCM | 2 ml | | | |
| Dispersed phase Polymer | Resomer RG502H (PLGA 50:50, acid, 7,000-17,000) | 160 mg | | | |
| Dispersed phase Additive | Tween 80 | 100 mg | - | - | - |
| | Span 20 | - | 100 mg | - | - |
| | PEG 400 | - | - | 100 mg | - |
| | Mrij 52 | - | - | - | 100 mg |

[Table 33]

| Examples 24-E to 24-G | | E | F | G |
|---|---|---|---|---|
| Dispersed phase Solvent | DCM | 2 ml | | |
| Dispersed phase Polymer | Resomer RG502H (PLGA 50:50, acid, 7,000-17,000) | 160 mg | | |
| Dispersed phase Additive | Brij 58 | 100 mg | - | - |
| | Poloxamer P124 | - | 100 mg | - |
| | SLS | - | - | 100 mg |

Example 25. Preparation of PLGA-based sustained-release particles by adding a modifier (additive, low-molecular weight surfactant) to the dispersed phase

Controlled-release PLGA particles of Examples 25-A to 25-G were prepared in the same manner as in Example 1-C except that, in step 2, 100 mg of tween 80, span 20, PEG 400, Mrij 52, Brij 58, poloxamer P124 or SLS was added as a modifier to the dispersed phase. Details of each component used in preparation of the particles and preparation conditions are shown in Tables 34 and 35.

[Table 34]

| Examples 25-A to 25-D | | A | B | C | D |
|---|---|---|---|---|---|
| Dispersed phase Solvent | DCM | 2 ml | | | |
| Dispersed phase Polymer | Lactel B6006-1 (PLGA 85:15, ester, 50,000-75,000) | 160 mg | | | |
| Dispersed phase Additive | Tween 80 | 100 mg | - | - | - |
| | Span 20 | - | 100 mg | - | - |
| | PEG 400 | - | - | 100 mg | - |
| | Mrij 52 | - | - | - | 100 mg |

[Table 35]

| Examples 25-E to 25-G | | E | F | G |
|---|---|---|---|---|
| Dispersed phase Solvent | DCM | 2 ml | | |

(continued)

| Examples 25-E to 25-G | | E | F | G |
|---|---|---|---|---|
| Dispersed phase Polymer | Lactel B6006-1 (PLGA 85:15, ester, 50,000-75,000) | 160 mg | | |
| Dispersed phase Additive | Brij 58 | 100 mg | - | - |
| | Poloxamer P124 | - | 100 mg | - |
| | SLS | - | - | 100 mg |

Example 26. Preparation of PLGA-based sustained-release particles by changing the content ratio of drug/polymer

Controlled-release PLGA particles of Examples 26-A to 26-F were prepared in the same manner as in Example 1-B except that the contents of the drug and the polymer were changed. Details of each component used in preparation of the particles and preparation conditions are shown in Table 36.

[Table 36]

| Examples 26-A to 26-F | | | A | B | C | D | E | F |
|---|---|---|---|---|---|---|---|---|
| Drug | | OND-1 | 40 mg | 60 mg | 80 mg | 100 mg | 120 mg | 160 mg |
| Dispersed phase | Solvent | DCM | 2 ml | 2 ml | 2 ml | 2 ml | 2 ml | 2 ml |
| | Polymer | Resomer RG752H (PLGA 75: 25, acid, 4,000-15,000) | 160 mg | 140 mg | 120 mg | 100 mg | 80 mg | 40 mg |
| Continuous phase | Purified water | D.W. | 50 ml | | | | | |
| | High molecular weight surfactant | PVA | 500 mg | | | | | |

Example 27. Preparation of PLGA-based sustained-release particles by changing the content ratio of drug/polymer

Controlled-release PLGA particles of Examples 27-A to 27-F were prepared in the same manner as in Example 1-A except that the contents of the drug and the polymer were changed. Details of each component used in preparation of the particles and preparation conditions are shown in Table 37.

[Table 37]

| Example 27-A to 27-F | | | A | B | C | D | E | F |
|---|---|---|---|---|---|---|---|---|
| Drug | | OND-1 | 40 mg | 60 mg | 80 mg | 100 mg | 120 mg | 160 mg |
| Dispersed phase | Solvent | DCM | 2 ml | 2 ml | 2 ml | 2 ml | 2 ml | 2 ml |
| | Polymer | Resomer RG502H (PLGA 50: 50, acid, 7,000-17,000) | 160 mg | 140 mg | 120 mg | 100 mg | 80 mg | 40 mg |
| Continuous phase | Purified water | D.W. | 50 ml | | | | | |
| | High molecular weight surfactant | PVA | 500 mg | | | | | |

Example 28. Preparation of PLGA-based sustained-release particles by changing the content ratio of drug/polymer

Controlled-release PLGA particles of Examples 28-A to 28-F were prepared in the same manner as in Example 1-C except that the contents of the drug and the polymer were changed. Details of each component used in preparation of the particles and preparation conditions are shown in Table 38.

[Table 38]

| Examples 28-A to 28-F | | | A | B | C | D | E | F |
|---|---|---|---|---|---|---|---|---|
| Drug | | OND-1 | 40 mg | 60 mg | 80 mg | 100 mg | 120 mg | 160 mg |
| Dispersed phase | Solvent | DCM | 2 ml | 2 ml | 2 ml | 2 ml | 2 ml | 2 ml |
| | Polymer | Lactel B6006-1 (PLGA 85:15, ester, 50,000-75,000) | 160 mg | 140 mg | 120 mg | 100 mg | 80 mg | 40 mg |
| Continuous phase | Purified water | D.W. | 50 ml | | | | | |
| | High molecular weight surfactant | PVA | 500 mg | | | | | |

Example 29. Preparation of PLGA-based sustained-release particles by changing the ratio of dispersed phase/continuous phase

Controlled-release PLGA particles of Examples 29-A to 29-F were prepared in the same manner as in Example 1-B except that the content of the continuous phase was changed. Details of each component used in preparation of the particles and preparation conditions are shown in Table 39.

[Table 39]

| Examples 29-A to 29-F | | | A | B | C | D | E | F |
|---|---|---|---|---|---|---|---|---|
| Dispersed phase | Solvent | DCM | 2 ml | 2 ml | 2 ml | 2 ml | 2 ml | 2 ml |
| | Polymer | Resomer RG752H (PLGA 75:25, acid, 4,000-15,000) | 160 mg | 160 mg | 160 mg | 160 mg | 160 mg | 160 mg |
| | Additive | - | - | - | - | - | - | - |
| Continuous phase | Purified water | D.W. | 10 ml | 20 ml | 40 ml | 50 ml | 80 ml | 100 ml |
| | High molecular weight surfactant | PVA | 500 mg | 1,000 mg | 2,000 mg | 2,500 mg | 4,000 mg | 5,000 mg |
| | Additive | - | - | - | - | - | - | - |
| Quenching medium | | Aqueous solution of 1% PVA | 250 ml | | | | | |

Example 30. Preparation of PLGA-based sustained-release particles by changing the ratio of dispersed phase/continuous phase

Controlled-release PLGA particles of Examples 30-A to 30-F were prepared in the same manner as in Example 1-A except that the content of the continuous phase was changed. Details of each component used in preparation of the particles and preparation conditions are shown in Table 40.

[Table 40]

| Examples 30-A to 30-F | | | A | B | C | D | E | F |
|---|---|---|---|---|---|---|---|---|
| Dispersed phase | Solvent | DCM | 2 ml | | | | | |
| | Polymer | Resomer RG502H (PLGA 50:50, acid, 7000-17000) | 160 mg | | | | | |
| Continuous phase | Purified water | D.W. | 10 ml | 20 ml | 40 ml | 50 ml | 80 ml | 100 ml |
| | High molecular weight surfactant | PVA | 500 mg | 1,000 mg | 2,000 mg | 2,500 mg | 4,000 mg | 5,000 mg |
| | Additive | - | - | - | - | - | - | - |
| Quenching medium | | Aqueous solution of 1% PVA | 250 ml | | | | | |

Example 31. Preparation of PLGA-based sustained-release particles by changing the emulsification method

Controlled-release PLGA particles of Example 31-A to 31-D were prepared in the same manner as in Example 1-C except that homogenizer conditions were changed in step 2. Details of each component used in preparation of the particles and preparation conditions are shown in Table 41.

[Table 41]

| Examples 31-A to 31-D | | A | B | C | D |
|---|---|---|---|---|---|
| Dispersed phase Solvent | DCM | 2 ml | | | |
| Dispersed phase Polymer | Lactel B6006-1 (PLGA 85:15, ester, 50000-75000) | 160 mg | | | |
| Dispersed phase Additive | - | - | | | |
| Emulsification | Homogenizer | 5,000 rpm, 5 min | 5,000 rpm, 8 min | 10,000 rpm, 5 min | 15,000 rpm, 5 min |
| | Sonicator | - | - | - | - |

Example 32. Preparation of PLGA-based sustained-release particles using ultrasonic irradiation

Controlled-release PLGA particles of Examples 32-A to 32-B were prepared in the same manner as in Example 1-B except that, in step 2, emulsification was performed using a sonicator instead of the homogenizer. Details of each component used in preparation of the particles and preparation conditions are shown in Table 42.

[Table 42]

| Examples 32-A to 32-B | | | A | B |
|---|---|---|---|---|
| Dispersed phase | Solvent | DCM | 2 ml | 2 ml |
| | Polymer | Resomer RG752H (PLGA 75:25, acid, 4000-15000) | 160 mg | 160 mg |
| | Additive | - | - | - |
| Emulsification | | Homogenizer | - | - |
| | | Sonicator | 40%, 3 min | 40%, 5 min |
| | | Freeze-drying | - | - |

Example 33. Preparation of PLGA-based sustained-release particles under changed vacuum drying conditions

Controlled-release particles of Examples 3 3-A to 33-D were prepared in the same manner as in Example 1-B except that vacuum drying conditions were changed. Details of each component used in preparation of the particles and preparation conditions are shown in Table 43.

[Table 43]

| Examples 33-A to 33-D | | | A | B | C | D |
|---|---|---|---|---|---|---|
| Drug | | OND-1 | 40 mg | 40 mg | 40 mg | 40 mg |
| Dispersed phase | Solvent | DCM | 2 ml | 2 ml | 2 ml | 2 ml |
| | Polymer | Resomer RG752H (PLGA 75:25, acid, 4,000-15,000) | 160 mg | 160 mg | 160 mg | 160 mg |
| | Additive | - | - | - | - | - |
| Drying | | Vacuum drying | 25°C, 24 hr | 25°C, 48 hr | 30°C, 48 hr | 35°C 48 hr |
| | | Freeze-drying | - | - | - | - |

Example 34. Preparation of PLGA-based sustained-release particles under changed vacuum drying conditions

Controlled-release particles of Examples 34-A to 34-D were prepared in the same manner as in Example 1-A except that vacuum drying conditions were changed. Details of each component used in preparation of the particles and preparation conditions are shown in Table 44.

[Table 44]

| Examples 34-A to 34-D | | | A | B | C | D |
|---|---|---|---|---|---|---|
| Drug | | OND-1 | 40 mg | 40 mg | 40 mg | 40 mg |
| Dispersed phase | Solvent | DCM | 2 ml | 2 ml | 2 ml | 2 ml |
| | Polymer | Resomer RG502H (PLGA 50:50, acid, 7,000-17,000) | 160 mg | 160 mg | 160 mg | 160 mg |
| | Additive | - | - | - | - | - |
| Drying | | Vacuum drying | 25°C 24 hr | 25°C 48 hr | 30°C 48 hr | 35°C 48 hr |
| | | Freeze-drying | - | - | - | - |

Example 35. Preparation of PLGA-based sustained-release particles under changed vacuum drying conditions

Controlled-release particles of Examples 35-A to 35-D were prepared in the same manner as in Example 1-C except that vacuum drying conditions were changed. Details of each component used in preparation of the particles and preparation conditions are shown in Table 45.

[Table 45]

| Examples 35-A to 35-D | | | A | B | C | D |
|---|---|---|---|---|---|---|
| Drug | | OND-1 | 40 mg | 40 mg | 40 mg | 40 mg |
| Dispersed phase | Solvent | DCM | 2 ml | 2 ml | 2 ml | 2 ml |
| | Polymer | Lactel B6006-1 (PLGA 85:15, ester, 50,000-75,000) | 160 mg | 160 mg | 160 mg | 160 mg |
| | Additive | - | - | - | - | - |

(continued)

| Examples 35-A to 35-D | | A | B | C | D |
|---|---|---|---|---|---|
| Drying | Vacuum drying | 25°C 24 hr | 25°C 48 hr | 30°C °C 48 hr | 35°C 48 hr |
| | Freeze-drying | | - - | - | - |

Example 36. Preparation of PLGA-based sustained-release particles under changed emulsification and freeze-drying conditions

Controlled-release particles of Examples 36-A to 36-D were prepared in the same manner as in Example 2-B except that homogenizer conditions or freeze-drying conditions were changed. Details of each component used in preparation of the particles and preparation conditions are shown in Table 46.

[Table 46]

| Examples 36-A to 36-D | | | A | B | C | D |
|---|---|---|---|---|---|---|
| Drug | | OND-1 | 40 mg | 40 mg | 40 mg | 40 mg |
| Dispersed phase | Solvent | DCM | 2 ml | 2 ml | 2 ml | 2 ml |
| | Polymer | Resomer RG752H (PLGA 75: 25, acid, 4,000-15,000) | 160 mg | 160 mg | 160 mg | 160 mg |
| | Additive | - | - | - | - | - |
| Emulsification | | Homogenizer | 5,000 rpm, 8 min | 5,000 rpm, 8 min | 15,000 rpm, 5 min | 5,000 rpm, 8 min |
| | | Sonicator | - | - | - | - |
| Drying | | Vacuum drying | - | - | - | - |
| | | Freeze-drying | -40°C 24 hr | -40°C 24 hr | -40°C 48 hr | -40°C 48 hr |

Example 37. Preparation of PLGA-based sustained-release particles under changed emulsification and freeze-drying conditions

Controlled-release particles of Examples 37-A to 37-D were prepared in the same manner as in Example 2-A except that homogenizer conditions or freeze-drying conditions were changed. Details of each component used in preparation of the particles and preparation conditions are shown in Table 47.

[Table 47]

| Examples 37-A to 37-D | | | A | B | C | D |
|---|---|---|---|---|---|---|
| Drug | | OND-1 | 40 mg | 40 mg | 40 mg | 40 mg |
| Dispersed phase | Solvent | DCM | 2 ml | 2 ml | 2 ml | 2 ml |
| | Polymer | Resomer RG502H (PLGA 50: 50, acid, 7,000-17,000) | 160 mg | 160 mg | 160 mg | 160 mg |
| | Additive | - | - | - | - | - |
| Emulsification | | Homogenizer | 5,000 rpm, 8 min | 5,000 rpm, 8 min | 15,000 rpm, 5 min | 5,000 rpm, 8 min |
| | | Sonicator | - | - | - | - |

(continued)

| Examples 37-A to 37-D | | A | B | C | D |
|---|---|---|---|---|---|
| Drying | Vacuum drying | - | - | - | - |
| | Freeze-drying | -40°C 24 hr | -40°C 24 hr | -40°C 48 hr | -40°C 48 hr |

Example 38: Preparation of PLGA-based sustained-release particles under changed emulsification and freeze-drying conditions

Controlled-release particles of Examples 38-A to 38-D were prepared in the same manner as in Example 2-C except that homogenizer conditions or freeze-drying conditions were changed. Details of each component used in preparation of the particles and preparation conditions are shown in Table 48.

[Table 48]

| Examples 38-A to 38-D | | | A | B | C | D |
|---|---|---|---|---|---|---|
| Drug | | OND-1 | 40 mg | 40 mg | 40 mg | 40 mg |
| Dispersed phase | Solvent | DCM | 2 ml | 2 ml | 2 ml | 2 ml |
| | Polymer | Lactel B6006-1 (PLGA 85:15, ester, 50,000-75,000) | 160 mg | 160 mg | 160 mg | 160 mg |
| | Additive | - | - | - | - | - |
| Emulsification | | | 5,000 rpm, 8 min | 5,000 rpm, 8 min | 15,000 rpm, 5 min | 5,000 rpm, 8 min |
| | | | - | - | - | - |
| Drying | | | - | - | - | - |
| | | Freeze-drying | -40°C 24 hr | -40°C 24 hr | -40°C 48 hr | -40°C 48 hr |

Example 39: Preparation of PLGA-based sustained-release particles containing [D-2-(4-methylbenzenthio)sarcosine$^3$]cyclosporine A

Controlled-release particles of Example 39-A to 39-C were prepared in the same manner as in Example 1 except that the drug was changed from OND-1 to [D-2-(4-methylbenzenthio)sarcosine$^3$]cyclosporine A. Details of each component used in preparation of the particles and preparation conditions are shown in Table 49.

[Table 49]

| Examples 39-A to 39-C | | | 39-A | 39-B | 39-C |
|---|---|---|---|---|---|
| Drug | | ([2-(4-methylbenzenethio) Sar$^3$]CsA | 40 mg | 40 mg | 40 mg |
| Dispersed phase | Solvent | DCM | 2 ml | 2 ml | 2 ml |
| | Polymer | Resomer RG502H (PLGA 50:50, acid, 7000-17000) | 160 mg | - | - |
| | | Resomer RG752H (PLGA 75:25, acid, 4,000-15,000) | - | 160 mg | - |
| | | Lactel B6006-1 (PLGA 85:15, ester, 50,000-75,000) | - | - | 160 mg |
| | Polymer | - | - | - | - |

Example 40. Preparation of PLGA-based sustained-release particles containing [N-methyl-aminobutyric acid$^3$]cyclosporine A

Controlled-release particles of Example 40-A to 40-C were prepared in the same manner as in Example 1 except that the drug was changed from OND-1 to [N-methyl-Abu$^3$]cyclosporine A. Details of each component used in preparation of the particles and preparation conditions are shown in Table 50.

[Table 50]

| Examples 40-A to 40-C | | | 40-A | 40-B | 40-C |
|---|---|---|---|---|---|
| Drug | | [N-methyl-Abu$^3$]CsA | 40 mg | 40 mg | 40 mg |
| Dispersed phase | Solvent | DCM | 2 ml | 2 ml | 2 ml |
| | Polymer | Resomer RG502H (PLGA 50:50, acid, 7,000-17,000) | 160 mg | - | - |
| | | Resomer RG752H (PLGA 75:25, acid, 4,000-15,000) | - | 160 mg | - |
| | | Lactel B6006-1 (PLGA 85:15, ester, 50,000-75,000) | - | - | 160 mg |
| | Additive | - | - | - | - |

Example 41. Preparation of PLGA-based sustained-release particles containing [N-methyl-Nva$^3$]cyclosporine A

Controlled-release particles of Example 41-A to 41-C were prepared in the same manner as in Example 1 except that the drug was changed from OND-1 to [N-methyl-Nva$^3$]cyclosporine A. Details of each component used in preparation of the particles and preparation conditions are shown in Table 51.

[Table 51]

| Examples 41-A to 41-C | | | 41-A | 41-B | 41-C |
|---|---|---|---|---|---|
| Drug | | [N-methyl-Nva$^3$]CsA | 40 mg | 40 mg | 40 mg |
| Dispersed phase | Solvent | DCM | 2 ml | 2 ml | 2 ml |
| | Polymer | Resomer RG502H (PLGA 50:50, acid, 7,000-17,000) | 160 mg | - | - |
| | | Resomer RG752H (PLGA 75:25, acid, 4,000-15,000) | - | 160 mg | - |
| | | Lactel B6006-1 (PLGA 85:15, ester, 50,000-75,000) | - | - | 160 mg |
| | Polymer | - | - | - | - |

Example 42. Preparation of PLGA-based sustained-release particles containing [D-MeAla$^3$]cyclosporine A

Controlled-release particles of Example 42-A to 42-C were prepared in the same manner as in Example 1 except that the drug was changed from OND-1 to [D-MeAla$^3$]cyclosporine A. Details of each component used in preparation of the particles and preparation conditions are shown in Table 52.

[Table 52]

| Examples 42-A to 42-C | | | 42-A | 42-B | 42-C |
|---|---|---|---|---|---|
| Drug | | [D-MeAla$^3$]CsA | 40 mg | 40 mg | 40 mg |
| Dispersed phase | Solvent | DCM | 2 ml | 2 ml | 2 ml |
| | Polymer | Resomer RG502H (PLGA 50:50, acid, 7000-17000) | 160 mg | - | - |
| | | Resomer RG752H (PLGA 75:25, acid, 4000-15000) | - | 160 mg | - |
| | | Lactel B6006-1(PLGA 85:15, ester,50000-75000) | - | - | 160 mg |
| | Additive | - | - | - | - |

Experimental Example 1. Evaluation of the size, morphology, and surface condition of PLGA particles

In this experimental example, the size, morphology, and surface condition of the PLGA particles prepared in Examples 1 to 42 were analyzed based on SEM images thereof to determine whether the PLGA particles had morphological features favorable for sustained drug release. The PLGA particles prepared in each example were dispersed in distilled water, followed by centrifugation, and then only the precipitated PLGA particles were vacuum-dried, followed by observation of an SEM image thereof.

FIG. 1 to FIG. 3 are SEM images of Examples 1-A to 1-C prepared through vacuum drying, respectively.

Referring to FIG. 1, the PLGA particles of Example 1-A exhibit a relatively uniform particle size distribution of about 5 μm to 30 μm and have a spherical shape with a smooth surface.

Referring to FIG. 2, the PLGA particles of Example 1-B exhibit a relatively uniform particle size distribution of about 20 μm to 40 μm and have a spherical shape with a smooth surface.

Referring to FIG. 3, the PLGA particles of Example 1-C exhibit a relatively uniform particle size distribution of about 10 μm to 30 μm and have a spherical shape with a smooth surface.

FIG. 4 to FIG. 6 are SEM images of Examples 2-A to 2-C prepared through freeze-drying, respectively.

Referring to FIG. 4, the PLGA particles of Example 2-A exhibit a relatively uniform particle size distribution of about 5 μm to 10 μm and have a spherical shape with a smooth surface.

Referring to FIG. 5, the PLGA particles of Example 2-B exhibit a polydisperse particle size distribution of about 2 μm to 20 μm and have a spherical shape with a smooth surface.

Referring to FIG. 6, the PLGA particles of Example 2-C exhibit a polydisperse particle size distribution of about 5 μm to 60 μm and have a spherical shape with a smooth surface.

FIG. 7 to FIG. 10 are SEM images of Examples 4-1-A to 4-1-D, respectively.

Referring to FIG. 7, the PLGA particles of Example 4-1-A exhibit a relatively uniform particle size distribution of about 3 μm to 8 μm and have a spherical shape with a smooth surface.

Referring to FIG. 8, the PLGA particles of Example 4-1-B exhibit a polydisperse particle size distribution of about 0.2 μm to 2 μm and have a spherical shape.

Referring to FIG. 9, the PLGA particles of Example 4-1-C exhibit a particle size distribution of about 5 μm to 25 μm and have a spherical shape. In addition, one month after in-vitro release, the PLGA particles of Example 4-1-C exhibit a particle size distribution of 3 μm to 10 μm and have a spherical shape.

Referring to FIG. 10, the PLGA particles of Example 4-1-D (#73-1) exhibit a relatively uniform particle size distribution of about 2 μm to 8 μm and have a spherical shape with a smooth surface. In addition, one month after in-vitro release, the PLGA particles of Example 4-1-D are melted at the surface thereof and have a hollow inside.

Referring to FIG. 11, the PLGA particles of Examples 31-B to 31-D have a spherical shape with a smooth surface despite changes in emulsification conditions.

Referring to FIG. 12 and FIG. 13, the PLGA particles of Examples 39-B, 40-B, 41-B, and 42-B prepared using different position 3-substituted cyclosporine derivatives have a spherical shape with a smooth surface.

FIG. 1 to FIG. 13 suggest that position 3-substituted cyclosporine derivative-containing PLGA particles have a smooth surface morphology and thus exhibit morphological features that enable stable and sustained drug release without an initial burst due to external pores.

Experimental Example 2. Particle size analysis of controlled-release PLGA formulations.

SEM images were taken of samples at random positions, followed by particle size analysis using an image analysis program to determine the particle size distribution. The number of particles to be measured was set to at least 300, and the size of the particles was measured in diameter.

2.1. Particle size analysis of vacuum-dried controlled-release PLGA formulations as a function of LA:GA ratio

FIG. 14 to FIG. 16 show results of particle size analysis of Examples 1-A to 1-C.

Example 1-A (#125), Example 1-B (#126), and Example 1-C (#127) had average particle sizes of 16.8 μm, 15.4 μm, and 9.44 μm, respectively.

2.2. Particle size analysis of vacuum-dried controlled-release PLGA formulations as a function of emulsification rate.

FIG. 17 to FIG. 19 show results of particle size analysis of Examples 31-B to 31-D.

Examples 31-B (#120), 31-C (#121), and 31-D (#122) had average particle sizes of 20.9 μm, 0.74 μm, and 0.61 μm, respectively, and the average particle size thereof had a tendency to decrease as the stirring speed of the homogenizer was increased from 5,000 rpm to 10,000 rpm to 15,000 rpm. In particular, when the stirring speed was increased from 5,000 rpm to 10,000 rpm, the average particle size thereof decreased sharply. For example, it can be seen that a preferred stirring speed of the homogenizer for preparing particles having an average particle size of about 20 μm is 5,000 rpm, and a preferred stirring speed of the homogenizer for preparing particles having an average particle

size of 1 μm or less is 10,000 rpm or more.

Experimental Example 3: measurement of the drug encapsulation efficiency and yield of microsphere of controlled-release PLGA formulations

Drug-loading capacity is calculated by dividing the amount of drug entrapped by the amount of microspheres recovered. Encapsulation efficiency may be expressed as a ratio of the amount of drug entrapped to the amount of drug fed. The amount of drug entrapped was measured by HPLC analysis (HPLC pump & column oven method). Details of the HPLC analysis method and conditions are as follows.

[Table 53] /

| Inject volume (μl) | Total time (min) | Flow rate (μl/min) | ACN (%) | D.W (%) | Column oven (°C) |
|---|---|---|---|---|---|
| 20 | 8.00 | 2000 | 75 | 25 | 70 |

[Analysis method]

1 mg of PLGA microspheres was dissolved in 1 ml of 75% ACN, followed by vortex for 30 seconds and sonication for 3 minutes. Thereafter, the resulting supernatant was filtered through a 0.22 μm syringe filter, followed by analysis by HPLC. Drug-loading capacity, encapsulation efficiency, and yield of microsphere may be calculated according to Equations 1 to 3, respectively.

[Equation 1]

Drug-loading capacity (%) = Weight (mg) of drug entrapped in microspheres ÷ weight (mg) of microspheres recovered × 100

[Equation 2]

Encapsulation efficiency (%) = Weight (mg) of drug entrapped in microspheres ÷ weight (mg) of drug fed × 100

[Equation 3]

Yield of microsphere (%) = Weight (mg) of microspheres recovered ÷ (weight (mg) of drug fed + polymer) × 100

The encapsulation efficiency and yield of microsphere of OND-1-containing controlled-release PLGA particles are shown in Table 54.

[Table 54]

| | Drug-loading capacity (%) | Encapsulation efficiency (%) | Yield of microsphere (%) |
|---|---|---|---|
| Example 1-A | 18.9 | 94.6 | 62.3 |
| Example 1-B | 18.9 | 94.6 | 67.3 |
| Example 1-C | 19.9 | 99.4 | 49.0 |
| Example 3-A | 19.7 | 98.3 | 71.3 |
| Example 3-B | 17.4 | 87.1 | 90.8 |
| Example 3-C | 17.7 | 88.7 | 68.0 |

(continued)

| | Drug-loading capacity (%) | Encapsulation efficiency (%) | Yield of microsphere (%) |
|---|---|---|---|
| Example 4-A | 19.9 | 99.5 | 75.0 |
| Example 4-B | 17.6 | 88.2 | 65.0 |
| Example 4-C | 19.6 | 97.9 | 79.0 |
| Example 5-A | 19.6 | 97.9 | 85.5 |

Experimental results of measuring the drug-loading capacity, encapsulation efficiency, and yield of microsphere of controlled-release PLGA particles containing various position 3-substituded cyclosporine A derivatives are shown in Table 55 and Table 56.

[Table 55]

| 50:50 | Drug-loading capacity (%) | Encapsulation efficiency (%) | Yield of microsphere (%) |
|---|---|---|---|
| Example 39-A | 19.0 | 96.5 | 59.2 |
| Example 39-B | 18.8 | 99.9 | 58.2 |
| Example 39-C | 19.2 | 98.8 | 76.6 |
| Example 40-A | 19.9 | 95.3 | 74.2 |
| Example 40-B | 18.1 | 88.5 | 80.3 |
| Example 40-C | 17.4 | 93.8 | 66.7 |

[Table 56]

| 75:25 | Drug-loading capacity (%) | Encapsulation efficiency (%) | Yield of microsphere (%) |
|---|---|---|---|
| Example 41-A | 17.4 | 91.8 | 56.1 |
| Example 41-B | 18.3 | 99.0 | 81.2 |
| Example 41-C | 17.9 | 91.6 | 75.8 |
| Example 42-A | 17.5 | 89.5 | 61.1 |
| Example 42-B | 19.2 | 93.2 | 73.0 |
| Example 42-C | 18.1 | 92.3 | 54.0 |

Experimental Example 4: Measurement of zeta-potential of controlled-release PLGA formulations

Interparticle aggregation was analyzed by measuring the zeta potential of the PLGA formulations. A zeta potential out of the range of ±30 mV indicates that the particles do not aggregate due to the presence of repulsive force therebetween, whereas, a zeta potential within the range of ±10 mV indicates that the particles can aggregate. Accordingly, a zeta potential of -30 mV or less or ±30 mV or greater indicates that a corresponding dispersion is stable.

In this experiment, the surface charge of the sustained-release PLGA formulations of Examples 1, 3, 4, 5, 14, 17, and 20 prepared by vacuum drying was measured using a Zetasizer (model name: Nano ZS 90; manufacturer: Malvern Panalytical Ltd.; dispersant: D.W.; temperature: 25°C; equilibration time: 120 sec). Zeta potential measurements at three positions were averaged and are shown in Tables 57 and 58.

[Table 57]

| | Zeta potential (mV) | St Dev (mV) |
|---|---|---|
| Example 1-A (50:50, A7K) | -41.5 | 5.86 |
| Example 1-B (75:25, A4K) | -37.2 | 5.43 |
| Example 1-C (85:15, E50K) | -31.1 | 4.62 |

(continued)

| | Zeta potential (mV) | St Dev (mV) |
|---|---|---|
| Example 3-A (50:50, A38K) | -27.2 | 5.97 |
| Example 3-B (50:50, E7K) | -33.2 | 5.49 |
| Example 3-C (50:50, E38K) | -28.3 | 6.25 |
| Example 4-A (75:25, A38K) | -38.7 | 5.01 |
| Example 4-B (75:25, E4K) | -35.0 | 6.27 |
| Example 4-C (75:25, E38K) | -31.5 | 5.08 |
| Example 5-A (85:15, E190K) | -27.3 | 6.01 |

[Table 58]

| | Zeta potential (mV) | St Dev (mV) |
|---|---|---|
| Example 14-E (Eudragit) | +46.9 | 2.4 |
| Example 14-F (chitosan) | +82.2 | 2.3 |
| Example 14-H (gelatin A) | +12.6 | 1.91 |
| Example 17-A (lecithin) | -3.52 | 0.94 |
| Example 17-B (poloxamer) | -46.23 | 6.38 |
| Example 17-C (tween 80) | +1.46 | 1.85 |
| Example 20-A (span 80) | -2.78 | 1.39 |

As can be seen from Tables 57 and 58, both Example 1 and Example 4 exhibited a zeta potential of -30 mV or less, indicating good dispersion stability.

Experimental Example 5. In-vitro release testing

(1) Experimental Method

Delayed drug release depends on the time for PLGA to degrade. When PBS with tween 80 0.2% (pH: 7.4), commonly used in in-vitro drug release testing, is used as a release medium, no drug is released for a minimum of two weeks and a maximum of four weeks. This is different from in-vivo release results.

Accordingly, an in-vitro release test was conducted using rat plasma as a release medium to mimic in-vivo conditions and to establish an optimal in vitro-in vivo correlation (IV-IVC). As the rat plasma, a supernatant obtained by dispensing blood from rats into heparinized tubes, followed by centrifugation in a centrifuge at 3,000 rpm for 10 minutes, was used. To 2 ml of the obtained rat plasma, the particles prepared in each example were added at a concentration of 50 μg/ml relative to OND-1 and stored in an incubator at 37°C with shaking at 90 rpm. Then, sampling was performed hourly, followed by analysis by HPLC to measure the quantity of drug released. Here, HPLC conditions are the same as those shown in Table 52. The HPLC analysis method is as follows.

[HPLC analysis method]

After a vial to be sampled was vortexed for 30 seconds, each 100 μl sample was dispensed from the vial, followed by centrifugation at 13,000 rpm for 30 minutes. Thereafter, 90 μl of the separated supernatant was dispensed, followed by addition of 270 μl of ACN (Sample A). To the remaining pellets, 360 μl of 75% ACN was added (Sample B). Then, Samples A and B were subjected to sonication for 3 minutes, followed centrifugation at 13,000 rpm for 30 minutes. Theater, 300 μl of the separated supernatant of Samples A and B was dispensed and filtered through a 0.22 μm syringe filter, followed by analysis by HPLC. Results of drug release profile analysis are shown in FIG. 20 to FIG. 27.

The above results demonstrate that the controlled-release PLGA formulations according to the present invention can exhibit sustained release properties. Furthermore, based on the experimental results, a drug exhibiting an appropriate

drug release profile can be prepared as needed. For example, the controlled-release PLGA particles according to the present invention may be formulated to exhibit a higher initial drug release rate for patients with severe hair loss, to allow slow drug release for patients with mild hair loss or those in need of hair loss prevention, and to exhibit a different drug release profile for women.

Specific results and details of each experiment are as follows.

5.1. Comparison of drug release profiles depending on the LA:GA ratio of PLGA

Drug (OND-1) release profiles of Examples 1-A to 1-C are shown in FIG. 20. Referring to FIG. 20, the rate of drug release was higher in the sequence of Example 1-A (LA:GA ratio of PLGA: 50:50) > Example 1-B (LA:GA ratio of PLGA: 75:25) > Example 1-C (LA:GA ratio of PLGA: 85:15). On day 7, Examples 1-A to 1-C exhibited drug release rates of 38%, 17%, and 0%, respectively, and, on day 14, Examples 1-A to 1-C exhibited drug release rates of 53%, 32%, and 9%, respectively. Examples 1-A, 1-B, and 1-C all showed no initial burst, indicating a good drug release profile as a sustained-release formulation.

SEM images of the particles on day 7 of drug release were observed and are shown in FIG. 21. Referring to FIG. 21, the particles of Example 1-A (drug release rate: 38%) almost lost the spherical shape thereof and exhibited an aggregate morphology. The particles of Example 1-B (drug release rate: 17%) retained the spherical shape thereof, but were observed to have a rough surface and many holes therein. The particles of Example 1-C (drug release rate: 0%) were observed to have a spherical shape with a smooth surface. The above in-vitro release results are consistent with the results of morphological observation.

According to the existing literature, PLGA microsphere formulations containing cyclosporine A (CsA) are known to exhibit a biphasic in-vitro drug release profile with an initial burst of drug release followed by slow drug release. Numerous papers have pointed out an initial burst phenomenon of cyclosporine A-containing PLGA microspheres (for example, J. Nanotechnology 2014, Article ID 683153 reported that approximately 70% to 92% of the drug is released within 24 hours, and another paper (J of Con Release 151 (2011) 286-294) reported that 75% to 90% of the drug is released within 24 hours.

Although not clearly understood, such a biphasic release profile with an initial burst of drug release followed by slow drug release is presumed due to: the internal structure of nanoparticles formed by freeze-drying; CsA remaining on the surface of the particles during a preparation process; rapid increase in solubility due to transformation of CsA from a crystalline structure to an amorphous structure during freeze-drying; rapid increase in solubility due to formation of an amorphous solid dispersion of CsA and PLGA; and the like. The inventors of the present invention found that the most important cause of the biphasic release profile is weak binding between CsA and PLGA and relatively high hydrophilicity of CsA.

CsA has a solubility in water of 27 μg/ml and a partition coefficient (log P) of 2.92 (Ismailos et al. 1991, Czogalla et al. 2009), whereas, OND-1, which is a derivative of CsA, has a solubility in water of 10 μg/ml and a partition coefficient (log P) of 3.84. That is, OND-1 can be released in a sustained and controlled manner due to its higher hydrophobicity than CsA, greater partition coefficient than CsA, and delayed release from microspheres in the body. Accordingly, PLGA microspheres containing CsA, which has a smaller partition coefficient than OND-1, can exhibit an initial burst, whereas PLGA microspheres containing OND-1, which is a more hydrophobic molecule than CsA, can provide sustained drug release without an initial burst.

In conclusion, the experimental results indicate that, unlike conventional CsA-containing PLGA microspheres, the OND-1-containing PLGA microspheres according to the present invention exhibit no in-vivo initial burst due to high in-vivo stability and good controlled release profile thereof and thus have good in-vivo controlled drug release properties.

Furthermore, FT-IR results of Experimental Example 7 described below show that, unlike CsA, OND-1 showed a shift of the amide I peak and strong interaction with PLGA. In addition, results of Experimental Example 8 show that the OND-1-containing PLGA microspheres according to the present invention showed a stable morphology since OND-1 was strongly bound to PLGA due to higher hydrophobicity than CsA and Tg of the OND-1-containing microspheres disappeared by PLGA. Accordingly, the stable drug release profile of the OND-1-containing PLGA microspheres may be attributed to the difference in chemical properties between CsA and a position 3-substituded derivative thereof.

5.2. Comparison of drug release profiles as a function of the type of end group and molecular weight of PLGA (LA:GA=50:50)

Drug release profiles of Examples 1-A and 3-A to 3-C are shown in FIG. 22. Referring to FIG. 22, the rate of drug release was higher in the sequence of Example 1-A > Example 3-A > Example 3-C > Example 3-B, that is, in the sequence of acid-7K > acid-38K > ester-38K > ester-7K. Examples 1-A, 3-A, 3-C, and 3-B had drug release rates of 37%, 32%, 21%, and 12%, respectively, as measured on day 8, and drug release rates of 54%, 45%, 29%, and 19%, respectively, as measured on day 14. All the examples showed a good sustained release profile without an initial burst.

5.3. Comparison of drug release profiles as a function of the type of end group and molecular weight of PLGA (LA:GA=75:25)

Drug release profiles of Examples 1-B and 4-A to 4-C are shown in FIG. 23. Referring to FIG. 23, the rate of drug release was higher in the sequence of Example 1-B > Example 4-B > Example 4-A > Example 4-C, that is, in the sequence of acid-4K > ester-4K > acid-38K > ester-38K. Examples 1-B, 4-B, 4-A, and 4-C had drug release rates of 17%, 11%, 0%, and 0%, respectively, as measured on day 8, and drug release rates of 33%, 17%, 9%, and 4%, respectively, as measured on day 14. All the examples showed a good drug release profile as a sustained-release formulation without an initial burst.

5.4. Comparison of drug release profiles as a function of the type of end group and molecular weight of PLGA (LA:GA=85:15)

Drug release profiles of Examples 1-C and 5-A are shown in FIG. 24. Referring to FIG. 24, the rate of drug release was higher in the sequence of Example 1-C > Example 5-A, that is, in the sequence of ester-50K > ester-190K. Example 1-C and Example 5-A had drug release rates of 9% and 1%, respectively, as measured on day 14, Both Example 1-C and Example 5-A showed a good drug release profile as a sustained-release formulation without an initial burst.

5.5. Analysis of drug release profiles as a function of drug content

Drug release profiles of Examples 26-A to 26-C are shown in FIG. 25. Referring to FIG. 25, the rate of drug release was higher in the sequence of Example 26-C > Example 26-B > Example 26-A, that is, in the sequence of OND-1 content: 60% > OND-1 content: 40% > OND-1 content: 20%. Examples 26-C, 26-B, and 26-A had drug release rates of 36%, 24%, and 15%, respectively, as measured on day 14. All the examples showed a good drug release profile as a sustained-release formulation without an initial burst.

5.6. Analysis of drug release profiles as a function of the type of modulator

Drug release profiles of Examples 23-A to 23-F are shown in FIG. 26. Referring to FIG. 26, the drug release rate was higher in the sequence of Example 23-B > Example 23-C > Example 23-F > Example 23-E > Example 23-A > Example 23-D, that is, in the sequence of span 20 > poloxamer P124 > Mrij 52 > Brij 58 > tween 80 > PEG 400 as the modulator. Examples 23-B, 23-C, 23-F, 23-E , 23-A, and 23-D had drug release rates of 96%, 53%, 48%, 39%, 34%, and 12%, respectively, as measured on day 14. All the examples showed a good drug release profile as a sustained-release formulation without an initial burst.

5.7. Release profiles of position 3-substituted cyclosporine derivative-containing drugs

Referring to FIG. 27, position 3-substituted cyclosporine derivatives, [D-2-(4-methylbenzenethio)-Sar$^3$]cyclosporine A, [N-methyl-D-Abu$^3$]cyclosporine A, [N-methyl-D-Nva$^3$]cyclosporine A, and [D-MeAla$^3$]cyclosporine A all showed a good drug release profile as a sustained-release formulation without an initial burst, similar to OND-1. [369]

Experimental Example 6. Difference in crystallinity between OND-1 and CsA: XRD and DSC results

A drug may exist in crystalline form, amorphous form, or a combination thereof. Crystallinity of the drug is one of the important physicochemical properties that affect the rate of drug release.

6.1. Determination of crystallinity by X-ray diffraction (XRD)

XRD measurement conditions and a crystallization method used in this experiment are as follows. Measurement results are shown in FIG. 28 and FIG. 29.

[Measurement conditions]

- X-ray diffractometer (model name: MiniFlex, Rigaku, Tokyo, Japan/- X-ray generator: graphite monochromatized Cu K$\alpha$ radiation ($\lambda$=1.5418 Å, 2$\theta$ range: from 10° to 30° with a step size of 0.02°/- Scanning rate: 1.0°/min)

[Crystallization Method]

- Evaporation crystallization: 20 mg of CsA or OND-1 is dissolved in 5 ml of ACN, followed by slow and complete evaporation of ACN at room temperature over 24 hours or more to obtain crystals.
- Drowning-out crystallization: 20 mg of CsA or OND-1 is dissolved in 5 ml of ACN, which, in turn, was added to 50 mL of D.W., followed by filtration to obtain crystals.

Referring to FIG. 28, cyclosporine A exhibited a crystalline XRD pattern regardless of preparation conditions. Results of XRD measurement of raw cyclosporine A powder show sharp peaks characteristic of crystalline form, indicating that the raw cyclosporine A raw powder is crystalline.

Conversely, referring to FIG. 29, OND-1, which is a position 3-substituted cyclosporine derivative, always exhibited an amorphous XRD pattern regardless of preparation conditions. OND-1 also exhibited an amorphous XRD pattern after recrystallization by evaporation or drowning-out to induce crystallinity. This difference suggests that OND-1 as the position 3-substituted cyclosporine derivative may exhibit very different release characteristics than cyclosporine A.

6.2. Determination of crystallinity by differential scanning calorimetry (DSC)

Differential scanning calorimetry (DSC) was used to identify a difference in crystallinity between cyclosporine A and OND-1 by determining the melting point, glass transition temperature, and recrystallization of the drugs. DSC measurement conditions were as follows. Results of DSC measurement are shown in FIG. 30 and FIG. 31.

[Measurement conditions]

DSC (model name: DSC Q2000; manufacturer: TA instruments; purging gas: $N_2$ gas (flow: 70 mL/min); heating rate: 5 °C/min; isothermal temperature: 10 °C/min; cycle number: 1 (from 20°C to 200°C)

Referring to FIG. 30, in the case of cyclosporine A raw powder, the melting temperature (Tm) appeared at about 112°C in the first heating curve, indicating that cyclosporine A is crystalline.

Conversely, referring to FIG. 31, in the case of raw OND-1 powder, Tm (112°C) of crystalline cyclosporine A disappeared in the first and second heating curves and the glass transition temperature or a phase transition due to formation of thermotropic liquid crystals appeared at about 125°C, indicating that OND-1 is amorphous.

Experimental Example 7. Evaluation of intraparticle drug-polymer interaction of controlled-release PLGA formulations (FT-IR)

Preparation of stable particles with controlled drug release properties requires that phase separation and recrystallization do not occur during storage, and that an initial burst is suppressed. This requires close interaction between a drug and PLGA. In order to study interaction between a drug and PLGA, FT-IR spectroscopic analysis of the amide I band, which is the absorption region of four amide groups in the molecule of cyclosporine A, and the absorption band of an ester bond of PLGA was conducted.

In particular, the amide I band (1,600 $cm^{-1}$ to 1,700 $cm^{-1}$) of cyclosporine A is a region that exhibits structural changes of four amide bonds in the molecule of cyclosporine A, and is an important indicator of how a secondary structure of cyclosporine A is altered by binding to the surrounding solvent or polymer. For example, in crystallized cyclosporine A, four amide groups are involved in formation of an intramolecular hydrogen bond. However, when cyclosporine A contacts a polar solvent or polymer, the amide groups can form a hydrogen bond with the external solvent or polymer instead of forming the intramolecular hydrogen bond. Accordingly, crystallized cyclosporine A exhibits a peak at 1,624 $cm^{-1}$, which is characteristic of intramolecular hydrogen bonding, but, when bound to a polar solvent or polymer, shows a peak at 1,630 $cm^{-1}$, which is characteristic of intermolecular hydrogen bonding. Results of determination of the amide I band shift of OND-1 as a function of solvent polarity showed that the peak of the amide I band is shifted from 1622 $cm^{-1}$ to 1634 $cm^{-1}$ as the polarity of the solvent increases, like CsA.

In order to evaluate chemical interaction between CsA or OND-1 and a PLGA network based on this FT-IR peak shift, shifts of spectroscopic absorption peaks in the amide I band and the ester bond region of PLGA can be utilized as evidence of the chemical interaction (Quimica Nova 35.4 (2012): 723-727, Current Drug Delivery, 2006, 3, 343-349, The AAPS Journal 2007; 9 (2)).

In this experiment, the formulations of Example 26 were analyzed by Fourier-transform infrared spectroscopy (FT-IR) to evaluate interaction between the position 3-substituted cyclosporine derivative and the PLGA polymer in the

PLGA particles. In addition, samples prepared by physical mixing (PM) of OND-1 and PLGA in mass ratios of 2:8, 3:7, 4:6, 5:5, and 8:2 were used as a control group. FT-IR measurement conditions are as follows. Results of FT-IR measurement are shown in FIG. 32 to FIG. 35.

[FT-IR measurement conditions]

FT-IR (model name: Nicolet iS 5; manufacturer: Thermo Fisher Scientific; range: 800 $cm^{-1}$ to 3,000 $cm^{-1}$; interval: 4 $cm^{-1}$; crystal type: diamond (pH of 1 to 12))

Referring to FIG. 32 and FIG. 33, the peak of the amide I band (amide C=O) of OND-1 appeared at 1,622 $cm^{-1}$, and the peak of the ester bond (ester C=O) of PLGA appeared at 1,746 $cm^{-1}$.

Referring to FIG. 34, in the control group prepared by physical mixing of OND-1 and PLGA in mass ratios of 2:8, 3:7, 4:6, 5:5, and 8:2 (OND-LPLGA), the peak of the amide I band of OND-1 appeared at 1622 $cm^{-1}$ without any peak shift, indicating that there was no binding between the two materials.

Conversely, referring to FIG. 35, the nanoparticles according to the present invention showed a significant shift of the amide I band peak of OND-1 from 1,622 $cm^{-1}$ to 1,633 $cm^{-1}$ to 1,634 $cm^{-1}$. In particular, nanoparticles with a mass ratio of OND-1:PLGA of 2:8 (Example 26-A) and 3:7 (Example 26-B) showed a strong shift of the amide I band peak of OND-1, indicating strong binding between OND-1 and PLGA. In addition, nanoparticles with a mass ratio of OND-1:PLGA of 4:6, 5:5, and 8:2 also showed a certain trend of peak shift. As the weight ratio of OND-1 to PLGA increased, the peak shift decreased due to decrease in binding of OND-1 to PLGA.

According to previous research results, no shift in the amide I band was observed in the FT-IR spectra of PLGA microsphere formulations containing cyclosporine (CsA). This seems to indicate that there is no or weak chemical interaction between CsA and PLGA (Quimica Nova 35.4 (2012): 723-727, Current Drug Delivery, 2006, 3, 343-349, Materials Research, Vol. 11, No. 2, 207-211, 2008, International Journal of Pharmaceutics 389 (2010) 186-194). Therefore, the initial burst and low formulation stability of CsA-containing PLGA microspheres are thought to be due to low chemical interaction between CsA and PLGA.

Previous studies could not predict that, when a position 3-substituded cyclosporine derivative and PLGA form particles, intramolecular amide groups are strongly bounded to PLGA. This may be due to the fact that OND-1 undergoes property changes due to addition of a methylthio group at position 3, despite being a derivative of cyclosporine A. According to the experimental examples of the present invention, unlike CsA, which is mostly crystalline, OND-1 was found to be amorphous under varying crystallization conditions, indicating that OND-1-containing PLGA nanoparticles can exhibit completely different properties than conventional CsA-based PLGA nanoparticles.

In conclusion, it was found that, unlike CsA-containing PLGA microspheres, OND-1-containing PLGA microspheres exhibit strong interaction between OND-1 and PLGA due to a shift of the amide I peak of OND-1. These experimental results support that the controlled-release formulation according to the present invention is highly stable and has good sustained in-vivo release properties without an initial burst. [401]

Experimental Example 8. Evaluation of intraparticle stability of drug and polymer of controlled-release PLGA formulations (DSC)

Intermolecular interaction between a drug and a polymeric carrier is required to prevent phase separation or recrystallization of drug-loaded particles during storage. In this experiment, the melting point or glass transition temperature and recrystallization of a drug were determined by differential scanning calorimetry (DSC). DSC measurement conditions are the same as in Experimental Example 6-2. Measurement results are shown in FIG. 36 to FIG. 38.

Referring to FIG. 36, the glass transition temperature (Tg) of OND-1 or phase transition of OND-1 due to formation of thermotropic liquid crystals was observed at 125°C, and the Tg of PLGA was observed at 51°C.

FIG. 37 to FIG. 38 show measurement results for nanoparticles of Example 26-A. Referring to FIG. 37, the Tg (or the temperature at which a phase transition due to formation of thermotropic liquid crystals occurs) of OND-1 is at 125°C, whereas, in NP containing 20% of OND-1, the Tg of OND-1 did not appear. In addition, referring to FIG. 38, NP containing 50% or 80% of OND-1 had a Tg lower than the Tg of OND-1. This may be due to the fact that interaction between OND-1 and PLGA eliminated heat-induced phase transition or molecular mobility of OND-1 molecules in the particles, or due to the fact that PLGA allows OND-1 to be maintained in a stable amorphous state.

Experimental Example 9. In-vitro skin penetration of position 3-substituted cyclosporine derivative.

A percutaneous absorption diffusion test (Franz diffusion cell) was conducted to determine skin penetration of position 3-substituted cyclosporine derivatives by topical skin application.

Specifically, frozen porcine skin was thawed, followed by removal of subcutaneous fat from the dermis, and then the skin was cut into a disc shape with a diameter of 3 cm. Then, the skin was mounted in a skin permeation device

(Franz diffusion, receptor volume: 5 ml, diffusion area: 0.785 $cm^2$) with the stratum corneum facing an upper donor chamber and the dermis facing a lower receptor chamber. Then, 0.2 ml of olive oil containing 200 μg of OND-1 was added to the donor chamber, followed by incubation under conditions of 37°C and 600 rpm. Hourly, 500 μl of a receiving solution (PBS containing 0.2% Tween 80, pH: 7.4) was sampled, followed by replenishment with an equal volume of fresh one.

The position 3-substituted cyclosporine derivative in the sampled solution was subjected to quantitative analysis by HPLC, followed by calculation of the average value of five replicates to determine the permeation amount of the position 3-substituted cyclosporine derivative. Results are shown in Table 58.

After 24 hours of use, the skin was removed from the skin permeation device, washed several times with ethanol, dried, and tape-stripped (10 times with scotch tape) to completely remove any residual drug from the surface thereof. The collected skin samples were lysed by incubation with collagenase type I (Gibco®) solution at 37°C for 24 hours. Thereafter, samples were obtained by a liquid-liquid phase extraction method using methyl tert-butyl ether (MTBE), followed by quantitative analysis of the position 3-substituted cyclosporine derivative by HPLC to calculate the quantity of drug deposited in the skin.

Details of the liquid-liquid extraction method are as follows. A 200 μl to 400 μl aliquot of each of the lysed skin samples was dispensed, followed by addition of 1.2 ml of MTBE. After high-speed shaking for 10 minutes, an organic solvent layer (1 ml) was separated by centrifugation (13,000 rpm, 3 minutes) and then dried under nitrogen flow for 10 minutes. Thereafter, the organic solvent layer was dissolved with a reconstituted solvent (75% acetonitrile) and filtered through a syringe filter to obtain a sample for analysis.

[HPLC Analytical Conditions]

Analytical system: Agilent 1100 series, CSA 4000; analytical column: Kromasil 100-5-C18, 4.6×150, 70°C; mobile phase: mobile phase A (water), mobile phase B (acetonitrile); flow rate of mobile phase: 2 ml/min; sample injection volume: 20 μl.

[Table 59]

| Time | 2 hr | 6 hr | 10 hr | 14 hr | 20 hr | 24 hr |
|---|---|---|---|---|---|---|
| Penetration rate (μg/$cm^2$) | Not detected | 1.3 | Not detected | 1.1 | 0.2 | 1.1 |

As shown in Table 59, the amount of dermal penetration (transdermal systemic absorption) of the position 3-substituted cyclosporine derivative was found to be extremely small. In addition, the quantity of the position 3-substituted cyclosporine derivative deposited in the skin was about 0.3 μg/$cm^2$ to 1.1 μg/$cm^2$. The experimental results showed that drug delivery by intralesional mesotherapy or the like was required to ensure that the position 3-substituted cyclosporine derivative provides a hair growth effect.

Experimental Example 10. Determination of hair growth effect by topical drug delivery (intralesional mesotherapy)

10-1. Determination of hair growth effect upon administration by topical injection

It was determined whether the position 3-substituted cyclosporine derivative has an effect of promoting hair follicle development and hair growth when administered by topical injection.

Specifically, hair was removed from the back of 42- to 49-day-old mice (C57BL/6, female) and the mice were weighed and divided into groups to be evenly distributed in terms of body weight. Thereafter, a depot solution containing the particles of Example 1-B was administered topically by intralesional mesotherapy (0.25 mm (31G)×8 mm insulin syringe) to determine the presence of a hair growth effect.

Referring to FIG. 39, the site of topical administration showed strong hair follicle formation and hair growth, whereas surrounding sites did not show hair follicle formation. Therefore, it was confirmed that topical administration of the position 3-substituted cyclosporine derivative promotes strong hair growth.

10-2. Determination of drug level in blood upon administration by topical injection.

In this experiment, it was determined whether the hair growth effect of the topically administered cyclosporine in Experimental Example 10-1 was due to systemic action or due to topical action.

Specifically, the drug level in blood was determined by a liquid-liquid extraction method using methyl tert-butyl ether (MTBE) and LC/MS analysis. Preparation of skin samples at a targeted site and a non-administration site and the

liquid-liquid-phase extraction method using MTBE were the same as in Experimental Example 9. LC/MS analysis conditions were as follows: HPLC analytical system: Agilent 1100 series, CSA 4000; column: Unison UK-C8 (3 μm), 2.0×50, 70°C; mobile phase: mobile phase A (water and formic acid (100:0.1, v/v)) and mobile phase B (methanol and formic acid (100:0.1, v/v)). After initially increasing the proportion of mobile phase B to 20% for 3 minutes to equilibrate the mobile phase, the flow rate of the mobile phase was 0.4 ml/min and the sample injection volume was 3 μl. In addition, as a mass spectrometer, a 4000 Q TRAP Mass Spectrometer system from Applied Bioscience was used.

The quantities of drug remaining at the administration site and the non-administration site measured by LC/MS analysis are shown in Table 60.

[Table 60]

| Test subject | Analysis of OND-1 level in systemic blood (μg/ml) | Quantity of OND-1 remaining at administration site (μg/g) | Quantity of OND-1 remaining at non-administration site (μg/g) |
|---|---|---|---|
| 1 | 0.005 | 98 | 0.04 |
| 2 | 0.001 | 120 | Not detected |
| 3 | Not detected | 109 | Not detected |
| 4 | 0.003 | 123 | 0.03 |
| 5 | Not detected | 132 | 0.01 |

Referring to Table 60, OND-1 was highly detected in the skin at the site of topical administration, whereas OND-1 was rarely detected in the surrounding skin or the systemic blood. Therefore, it was confirmed that the hair growth effect of the position 3-substituted cyclosporine derivative is due to topical action rather than systemic action, and that the position 3-substituted cyclosporine derivative can provide hair growth results when topically administered to the site of hair loss.

Experimental Example 11. Determination of drug loss into systemic circulation upon topical (intradermal or intralesional) injection of PLGA depot.

If a topically administered drug is easily lost into the systemic circulation, this can cause systemic toxicity or low effectiveness. This experiment was conducted to determine the extent of drug loss due to diffusion into the systemic circulation upon administration by topical injection of the PLGA depot formulation according to the present invention.

Rats (Sprague Dawley®, female) aged 4 to 5 weeks were prepared, hair was removed from the back of each rat before drug administration, and the experimental animals were weighed and divided into groups to be evenly distributed in terms of body weight. After a one-day acclimatization period, the OND-1-containing drug formulation (#126) of Example 1-B and a OND-1 solution (PG:EtOH:DW=6:2:2) as a control were administered by intradermal injection (ID) to six locations on the hairless back of each rat. The volume of injection administered was about 50 μl, and a 0.25 mm (31G)×8 mm insulin syringe was used.

After drug administration, about 300 μl to 400 μl of blood was collected from the subclavian vein of each experimental animal at planned time intervals (1, 3, 6, 9, 12, 15, 18, 21, 24, 28, 32, 36, 40, 44, 48, 54, 72, 78, 144, 168, and 192 hours after drug administration). The collected blood samples were treated by a liquid-liquid phase extraction method using methyl tert-butyl ether (MTBE), followed by measurement of drug levels in blood by LC/MS analysis. (For the MTBE liquid-liquid phase extraction method and LC/MS analysis method, see Experimental Example 10)

Results of the experiment are shown in Table 61 and FIG. 40.

[Table 61]

| | PG:EtOH:DW (=6:2:2) | PLGA #126 |
|---|---|---|
| $AUC_{(0-t)}$ (μg/L*h) | 3362 | 406 |
| $T_{max}$ (h) | 1 | 36 |
| $C_{max}$ (μg/L) | 152.3 | 15.3 |

Referring to Table 61 and FIG. 40, an experimental animal group administered the drug-loaded PLGA particle formulation according to the present invention showed low systemic drug exposure compared to an experimental animal group administered the general solution formulation. Quantitative comparison of calculated area under curve (AUC)

values revealed that the drug level in blood in the animal group administered the general solution formulation was approximately eight times higher than that in the animal group administered the PLGA particle formulation.

These experimental results indicate that topical administration of position 3-substituted cyclosporine derivative-containing PLGA particles can suppress systemic side effects due to increased drug levels in blood that may occur when using conventional injectable solution formulations, while ensuring moderate and long-lasting maintenance of the drug level (therapeutic window) at the site of hair loss, thereby providing enduring hair loss prevention and hair growth promotion. Comparison of conditions of the experimental animals before and during the experiment showed that no unusual clinical symptoms were found in all treatment groups.

Experimental Example 12. Determination of the quantity of drug remaining in the skin upon administration by topical (intradermal or intralesional) injection of PLGA depot.

In order for a topically administered drug to show a good effect, the drug is required to remain at the site of topical administration for a certain period of time. This experiment was conducted to determine the quantity of drug remaining in the skin over time upon topical administration.

Rats (Sprague Dawley®, female) aged 4 to 5 weeks were prepared and hair was removed from the back of each rat. Then, the experimental animals were weighed, divided into groups to be evenly distributed in terms of body weight, and allowed to acclimate for one day. An injection formulation containing the particles of Example 1-B was administered by intradermal injection (ID) to the hairless back of each animal (using a 0.25 mm (31G) ×8 mm insulin syringe).

At the end of observation, a skin sample was collected from the back of each experimental animal, followed by quantitative analysis of the quantity of drug remaining in the skin by a liquid-liquid extraction method using methyl tert-butyl ether (MTBE) and HPLC. (For details, see Experimental Example 9)

Results of HPLC analysis are shown in FIG. 41. Referring to FIG. 41, the PLGA depot showed an AUC more than 4 times larger than that of the general solution, indicating that a significantly larger amount of drug remained in the skin. This indicates that topical delivery of the position 3-substituted cyclosporine derivative to the skin in the form of PLGA particles can minimize rapid increase in drug level in the systemic blood and resulting systemic side effects that may occur when using conventional injectable solution formulations, while ensuring moderate and long-lasting maintenance of the drug level (therapeutic window) at the site of hair loss, thereby providing sustained hair loss prevention and hair growth promotion.

Experimental Example 13. Evaluation of the quantity of drug remaining in the skin as a function of the LA:GA ratio of PLGA.

Rats (Sprague Dawley®, female) aged 4 to 5 weeks were prepared, hair was removed from the back of each rat, and the experimental animals were weighed and divided into groups to be evenly distributed in terms of body weight. After a one-day acclimatization period, the OND-1-containing PLGA particles with different LA:GA ratios of Example 1-A (#125), Example 1-B (#126), and Example 1-C (#127) were administered by intradermal injection (ID) (injection solution: about 50 μl of saline) into several locations on the hairless back of each experimental animal, starting from the bottom thereof (left side: Example 1-A, center: Example 1-B, right side: Example 1-C), at planed time intervals (1, 4, 8, 10, 15, 16, and 17 days before the end of the experiment, for a total of 7 doses) using a 0.25 mm (31G)×8 mm insulin syringe.

At the end of observation, skin samples of similar size were taken from the back of each experimental animal, followed by quantitative analysis of the quantity of drug remaining in the skin by a liquid-liquid extraction using MTBE and HPLC. (See Experimental Example 9)

Results of the experiment are shown in FIG. 42. Referring to FIG. 42, the three formulations with different LA to GA ratios showed differences in terms of the rate of drug appearance from the administration site over time.

In order to confirm this numerically, area under curve (AUC) values of the drug retention curve over time were plotted into a graph. The AUC values of the three formulations with different LA to GA ratios were statistically significant to each other (t-test, $^*p<0.05$, $^{**}p<0.01$, $^{***}p<0.001$).

The above results showed that PLGA particles loaded with the position 3-substituted cyclosporine derivative drug can exhibit significant differences in terms of the extent of drug disappearance from the administration site depending on the type of PLGA polymer (PLA:PGA: 50:50, 75:25, and 85:15). This suggests that a sustained-release formulation with a controlled drug release rate can be prepared by changing the type of PLGA polymer as needed. Comparison of conditions of the experimental animals before and during the experiment showed that no unusual clinical symptoms were found in any of the treatment groups.

## Claims

1. Controlled-release particles comprising a position 3-substituted cyclosporine derivative represented by Formula 1 and a biodegradable polymer.

[Formula 1]

MeBmt-Abu-**C**-MeLeu-Val-MeLeu-Ala-DAla-MeLeu-MeLeu-MeVal

where C is an amino acid represented by -N($R_1$)-CH($R_2$)-C(=O)-,
$R_1$ is hydrogen or a methyl group,
$R_2$ is X-R' or R', X being oxygen (O) or sulfur (S), R' being: hydrogen; a linear or branched $C_1$-$C_6$ alkyl group, alkenyl group, or alkynyl group; or an aryl group, and
each carbon in the alkyl group, the alkenyl group, or the alkynyl group is substituted or unsubstituted with one to three substituents selected from the group consisting of an amino group, a hydroxyl group, a halogen group, a haloalkyl group, an ester group, an alkoxy group, a cyano group, a nitro group, an alkylamino group, and a dialkylamino group.

**2.** The controlled-release particles according to claim 1, wherein X is sulfur (S).

**3.** The controlled-release particles according to claim 1, wherein the position 3-substituted cyclosporine derivative is:

[2-methylthio-sarcosine$^3$]cyclosporine A;
[2-dimethylamino-ethylthio-sarcosine$^3$]cyclosporine A;
[2-(4-methylbenzentio)sarcosine$^3$]cyclosporine A;
[N-methyl-aminobutyric acid$^3$]cyclosporine A;
[N-methyl-norvaline$^3$]cyclosporine A;
[2-(methylamino)-hexa-4-ynoyl$^3$]cyclosporine A;
[2-(methylamino)-pent-4-ynoyl$^3$]cyclosporine A;
[N-methyl-O-propenyl-serine$^3$]cyclosporine A;
[N-methyl-serine$^3$]cyclosporine A; or
[N-methyl-alanine$^3$]cyclosporine A.

**4.** The controlled-release particles according to claim 1, wherein the position 3-substituted cyclosporine derivative is [2-methylthio-sarcosine$^3$]cyclosporine A.

**5.** The controlled-release particles according to claim 1, wherein the biodegradable polymer is a natural polymer or a synthetic polymer.

**6.** The controlled-release particles according to claim 5, wherein the natural polymer comprises at least one selected from the group consisting of carboxymethylcellulose, algin, alginic acid, alginate, hyaluronic acid, polypeptides, proteins, gelatin, collagen, albumin, dextran, starch, casein, chitin derivatives, chitosan, and small intestinal submucosa tissue.

**7.** The controlled-release particles according to claim 5, wherein the synthetic polymer comprises at least one selected from the group consisting of polyethylene (PEA), polyethylene glycol (PEG), polycaprolactone (PCL), polyalkylcarbonate, polyamino acid, polyhydroxybutyric acid, polyorthoester, polyanhydride, Pluronic (polyethylene oxide)poly(propylene oxide)poly(ethylene oxide)), polylactide (PLA), polyglycolide (PGA), polybutylene succinate (PBS), poly(3-hydroxybutyrate-co-3-hydroxyvalerate) (PHBV), polybutylene terephthalate (PBT), polytrimethylene terephthalate (PTT), polyethylene naphthalate (PEN), polylactide-co-glycolide (poly(D,L-lactic-co-glycolic acid), PLGA), polylactide-co-glycolide-glucose (PLGA-glucose), and methoxy polyethylene glycol-(polycaprolactone-co-polylactide) (MPEG-(PCL-co-PLLA)).

**8.** The controlled-release particles according to claim 1, wherein the biodegradable polymer is: polylactide-co-glycolide (poly(lactic-co-glycolic acid), PLGA); or a di- or tri-block copolymer having polylactide-co-glycolide as a hydrophobic block.

**9.** The controlled-release particles according to claim 8, wherein the polylactide-co-glycolide (poly(lactic-co-glycolic acid), PLGA) has a monomeric molar ratio of lactide to glycolide of 90:10 to 40:60.

**10.** The controlled-release particles according to claim 8, wherein the polylactide-co-glycolide has an ester (neutral charge) or a carboxylic acid (negative charge) as an end group.

**11.** The controlled-release particles according to claim 8, wherein the polylactide-co-glycolide has a molecular weight of 4,000 Da to 240,000 Da.

**12.** The controlled-release particles according to claim 8, wherein the polylactide-co-glycolide is a cationic graft copolymer with poly-L-lysine introduced thereinto.

**13.** The controlled-release particles according to claim 1, wherein a weight ratio of the position 3-substituted cyclosporine derivative to the biodegradable polymer ranges from 5:95 to 90:10.

**14.** The controlled-release particles according to claim 1, wherein the controlled-release particles have an average diameter of 0.01 μm to 300 μm.

**15.** The controlled-release particles according to claim 1, wherein the controlled-release particles have an average zeta potential of +2 mV or greater or -2 mV or less.

**16.** The controlled-release particles according to claim 1, comprising 5% to 40% (w/w) of the position 3-substituted cyclosporine derivative.

**17.** A composition for prevention of hair loss or promotion of hair growth, comprising the controlled-release particles according to claim 1.

**18.** The composition according to claim 17, wherein hair loss comprises at least one selected from the group consisting of androgenetic alopecia (AGA), female-pattern hair loss, autoimmune alopecia areata, telogen effluvium, and cicatricial alopecia.

**19.** The composition according to claim 17, wherein the composition is administered intralesionally into a subject's skin, intradermally into a subject's scalp, or subcutaneously into a patient's scalp.

**20.** The composition according to claim 17, wherein the composition is formulated as a topical injection, a depot injection, or a dermal implant.

**21.** The composition according to claim 17, further comprising at least one selected from the group consisting of an aqueous vehicle for injection, an isotonic agent, and a suspending agent.

**22.** The composition according to claim 21, wherein the aqueous vehicle for injection comprises at least one selected from the group consisting of saline, sterile water, a Ringer's solution, buffered saline, an albumin injectable solution, a dextrose solution, a maltodextrin solution, glycerol, and ethanol.

**23.** The composition according to claim 21, wherein the isotonic agent comprises at least one selected from the group consisting of D-mannitol, maltitol, sorbitol, lactitol, xylitol, and sodium chloride.

**24.** The composition according to claim 21, wherein the suspending agent comprises at least one selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, and xanthan gum.

**25.** A method of preparing position 3-substituted cyclosporine derivative-loaded controlled-release particles, comprising:

a) preparing a drug-polymer dispersed phase by dissolving a position 3-substituted cyclosporine derivative and a biodegradable polymer in a solvent;
b) preparing an emulsion by mixing the dispersed phase with a continuous phase comprising a surfactant; and
c) producing position 3-substituted cyclosporine derivative-loaded particles by mixing the prepared emulsion with a quenching medium, followed by stirring to remove the solvent of the dispersed phase.

**26.** The method according to claim 25, wherein the biodegradable polymer is: polylactide-co-glycolide (poly(lactic-co-glycolic acid), PLGA); or a di- or tri-block copolymer having polylactide-co-glycolide as a hydrophobic block.

**27.** The method according to claim 25, wherein the solvent of the dispersed phase comprises at least one selected from the group consisting of dichloromethane, chloroform, acetonitrile, dimethyl sulfoxide (DMSO), dimethylformaldehyde, ethyl acetate, butyl acetate, ethyl formate, isopropyl acetate, isopropyl formate, diethyl ether, and glycofurol.

**28.** The method according to claim 25, wherein the solvent of the dispersed phase further comprises a co-solvent, the co-solvent comprising at least one co-solvent selected from the group consisting of methanol, ethanol, acetone, isopropanol, diethyl ether, chloroform, ethyl acetate, DMSO, and acetonitrile.

**29.** The method according to claim 25, wherein the dispersed phase in step a) further comprises a release modifier.

**30.** The method according to claim 29, wherein the release modifier comprises at least one hydrophilic release modifier selected from the group consisting of polyoxyethylene sorbitan fatty acid esters, glyceryl monooleate, sorbitan fatty acid esters, polyvinyl alcohol, poloxamers, polyethylene glycol, glyceryl palmitostearate, benzyl benzoate, ethyl oleate, $\alpha$-cyclodextrin, $\beta$-cyclodextrin, $\gamma$-cyclodextrin, hydroxypropyl $\beta$-cyclodextrin, tween 80, span 20, PEG 400, Mrij 52, Brij 58, poloxamer P124, and SLS.

**31.** The method according to claim 29, wherein the release modifier comprises at least one lipophilic release modifier selected from the group consisting of cottonseed oil, lecithin, castor oil, corn oil, sunflower oil, sesame oil, soybean oil, and peanut oil.

**32.** The method according to claim 25, wherein the surfactant in the continuous phase comprises at least one selected from the group consisting of polyethylene glycol, methylcellulose, hydroxypropyl methylcellulose, polyvinylpyrrolidone, lecithin, gelatin, chitosan, Eudragit, polyvinyl alcohol, polyoxyethylene sorbitan fatty acid esters, poloxamers, span, polyoxyethylene castor oil derivatives, Cremophor, Myrj 52, Brij 58, cyclodextrins, sodium lauryl sulfate, sodium stearate, ester amines, linear diamines, and fatty amines.

**33.** The method according to claim 25, wherein the surfactant in the continuous phase is polyvinyl alcohol.

**34.** The method according to claim 25, wherein the surfactant in the continuous phase comprises at least one selected from the group consisting of span, Labrasol, Rheodol MO-60, glycerol monooleate, and triacetin.

**35.** The method according to claim 25, wherein the surfactant is present in an amount of 0.1% to 20% (w/v) in the continuous phase.

**36.** The method according to claim 25, wherein, in step b), the dispersed phase and the continuous phase are mixed in a volume ratio of 1:5 to 1:50.

**37.** The method according to claim 25, wherein the quenching medium is water, methanol, ethanol, propanol, ethyl acetate, polyvinyl alcohol (PVA), or a combination thereof.

**38.** The method according to claim 25, wherein the quenching medium is mixed with the emulsion in an amount 1 to 100 times higher than that of the emulsion.

**39.** The method according to claim 25, wherein step c) is performed by an emulsification method selected from among mechanical agitation, high pressure emulsification, fluid-bed emulsification, static-motor emulsification, ultrasonic emulsification, membrane emulsification, nozzle spraying, and dripping.

**40.** The method according to claim 25, further comprising:

after step c),
d) separating the produced position 3-substituted cyclosporine derivative-loaded particles.

**41.** The method according to claim 40, further comprising:

after step d),

e) subjecting the separated particles to vacuum drying or freeze-drying.

**42.** The method according to claim 41, wherein vacuum drying is performed at a temperature of 30°C to 40°C and freeze-drying is performed at a temperature of - 30°C to -40°C.

【FIG. 1】

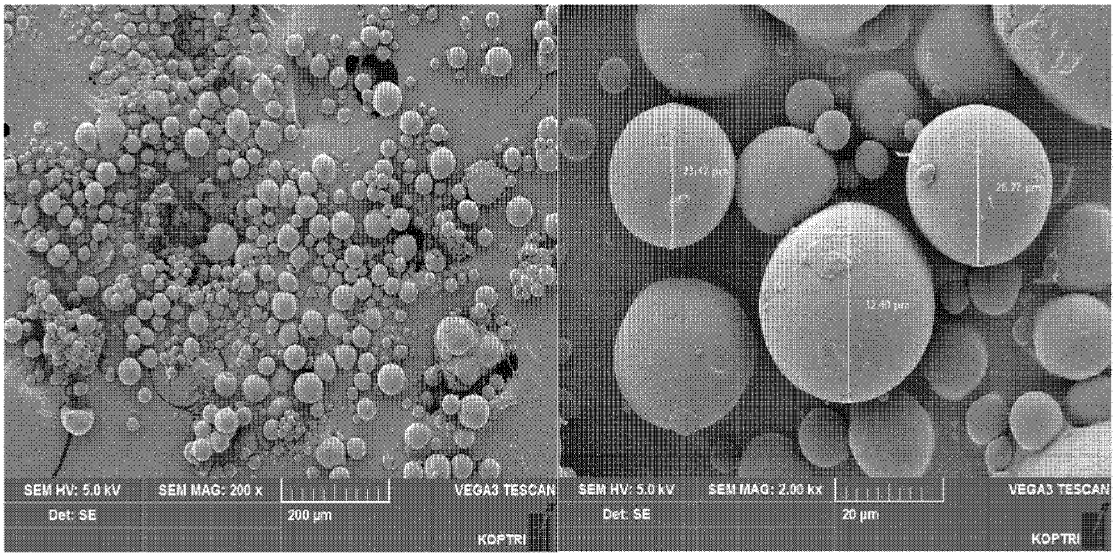
SEM HV: 5.0 kV
SEM MAG: 200 x
VEGA3 TESCAN
Det: SE
200 μm
KOPTRI
SEM HV: 5.0 kV
SEM MAG: 2.00 kx
VEGA3 TESCAN
Det: SE
20 μm
KOPTRI

【FIG. 2】

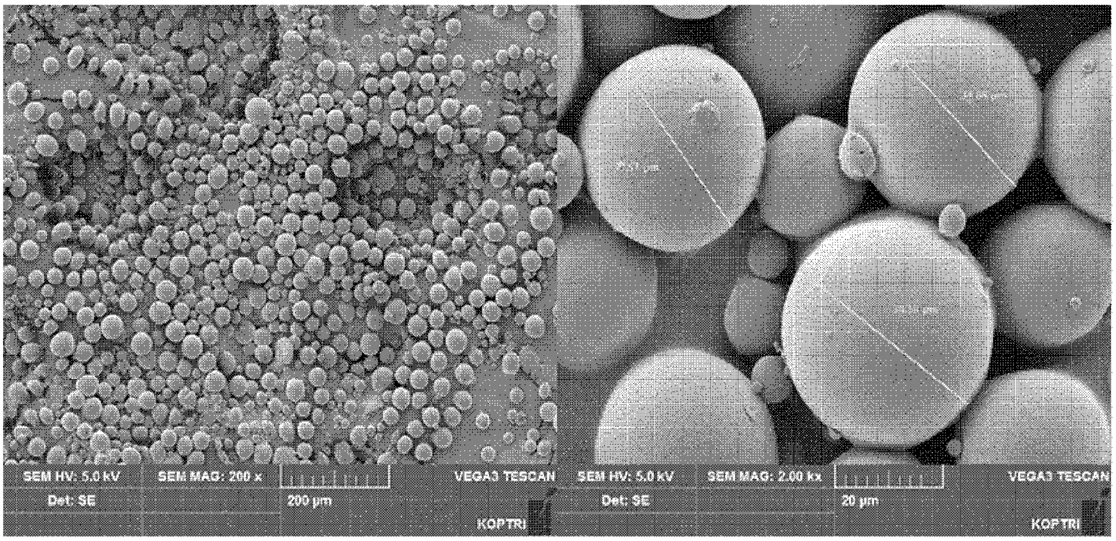
SEM HV: 5.0 kV
SEM MAG: 200 x
VEGA3 TESCAN
Det: SE
200 μm
KOPTRI
SEM HV: 5.0 kV
SEM MAG: 2.00 kx
VEGA3 TESCAN
Det: SE
20 μm
KOPTRI

【FIG. 3】

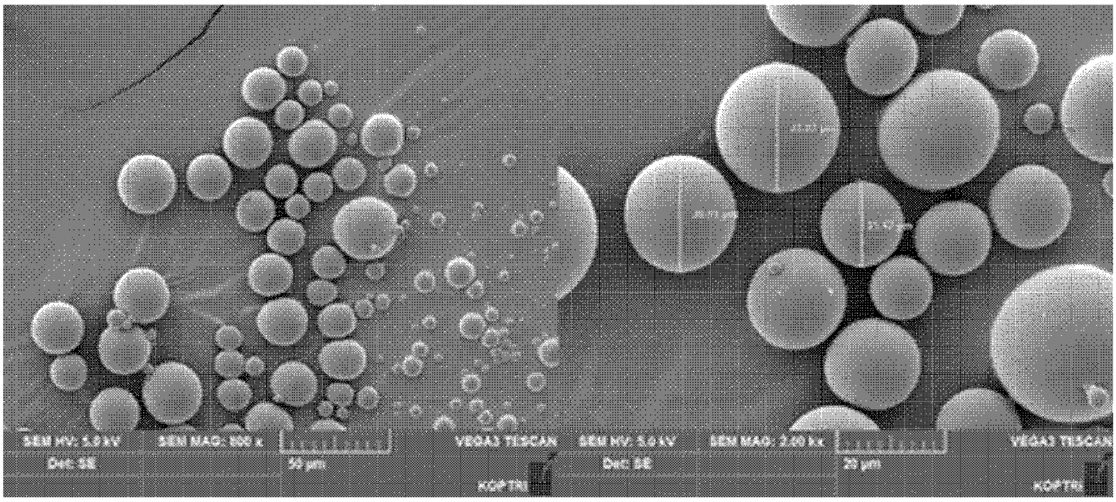
SEM HV: 5.0 kV
Det: SE
50 µm
VEGA3 TESCAN
KOPTRI
20 µm
VEGA3 TESCAN
KOPTRI

【FIG. 4】

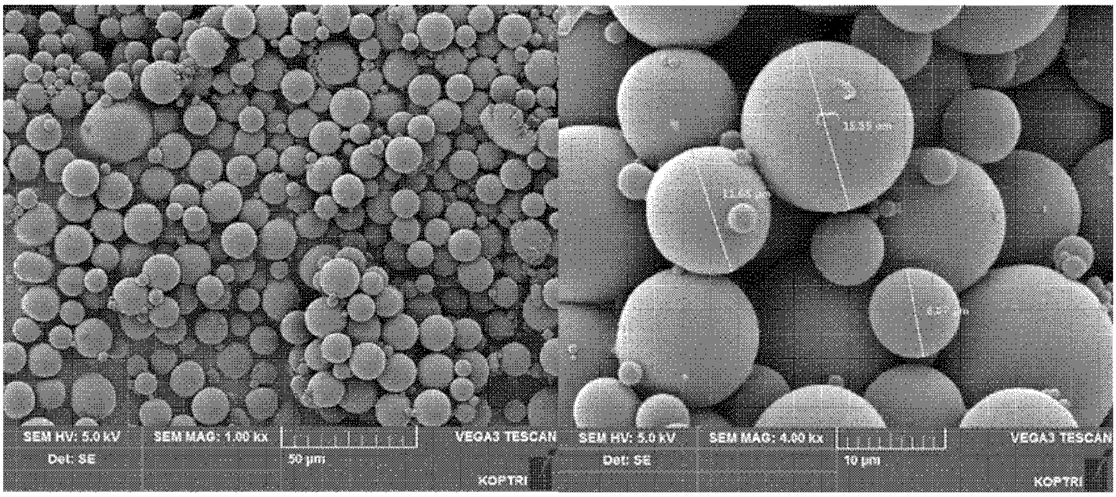
SEM HV: 5.0 kV
Det: SE
SEM MAG: 1.00 kx
50 µm
VEGA3 TESCAN
KOPTRI
SEM HV: 5.0 kV
Det: SE
SEM MAG: 4.00 kx
10 µm
VEGA3 TESCAN
KOPTRI

【FIG. 5】

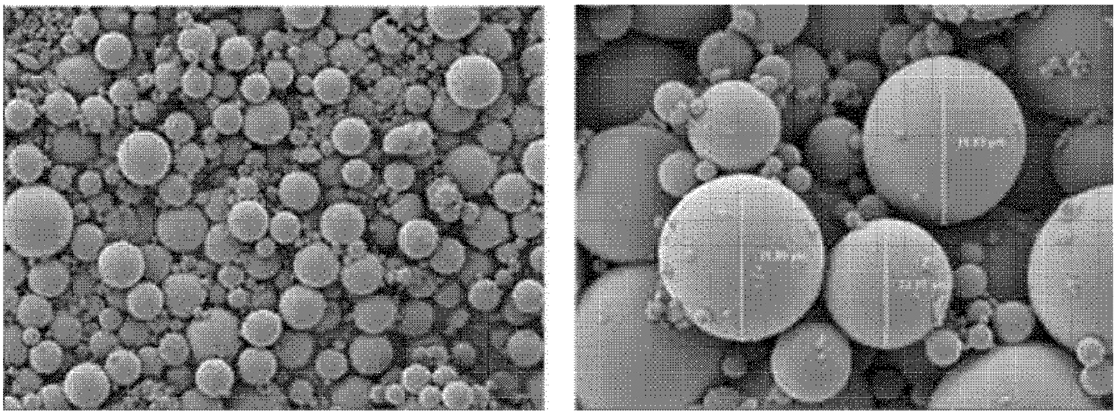

【FIG. 6】

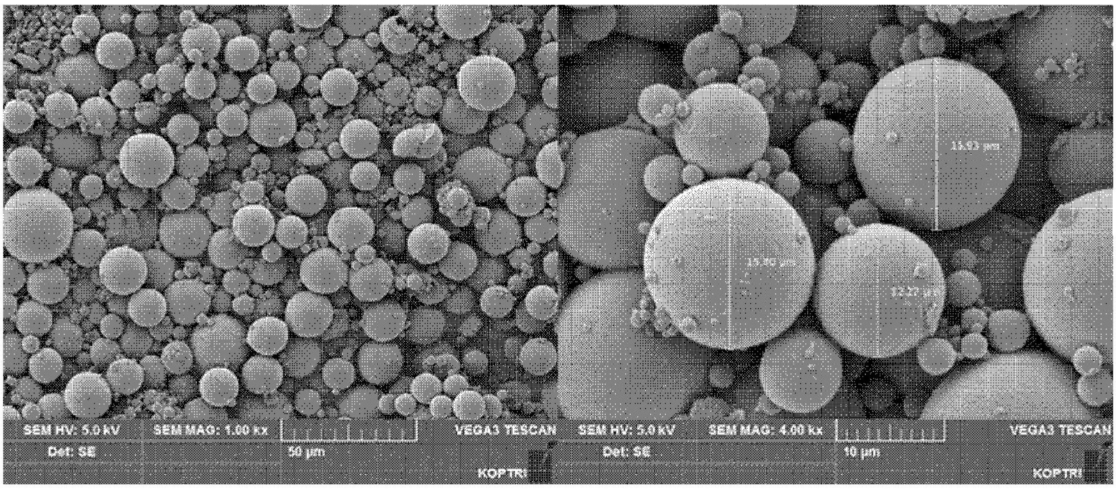
SEM HV: 5.0 kV
SEM MAG: 1.00 kx
VEGA3 TESCAN
Det: SE
50 µm
KOPTRI
SEM HV: 5.0 kV
SEM MAG: 4.00 kx
VEGA3 TESCAN
Det: SE
10 µm
KOPTRI

【FIG. 7】

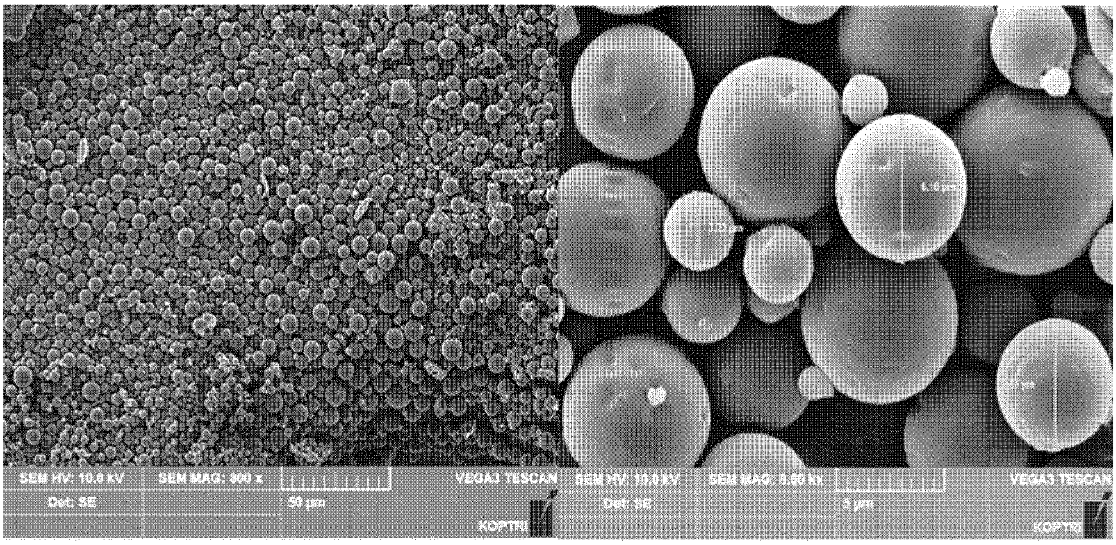
SEM HV: 10.0 kV
Det: SE
50 μm
VEGA3 TESCAN
KOPTRI
SEM HV: 10.0 kV
Det: SE
5 μm
VEGA3 TESCAN
KOPTRI

【FIG. 8】

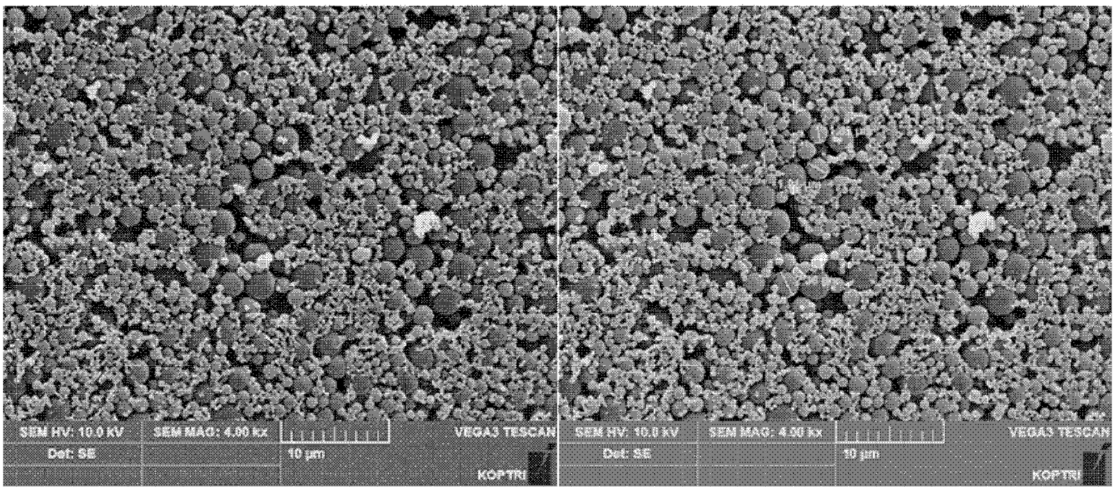
SEM HV: 10.0 kV
SEM MAG: 4.00 kx
Det: SE
10 μm
VEGA3 TESCAN
KOPTRI
SEM HV: 10.0 kV
SEM MAG: 4.00 kx
Det: SE
10 μm
VEGA3 TESCAN
KOPTRI

【FIG. 9】

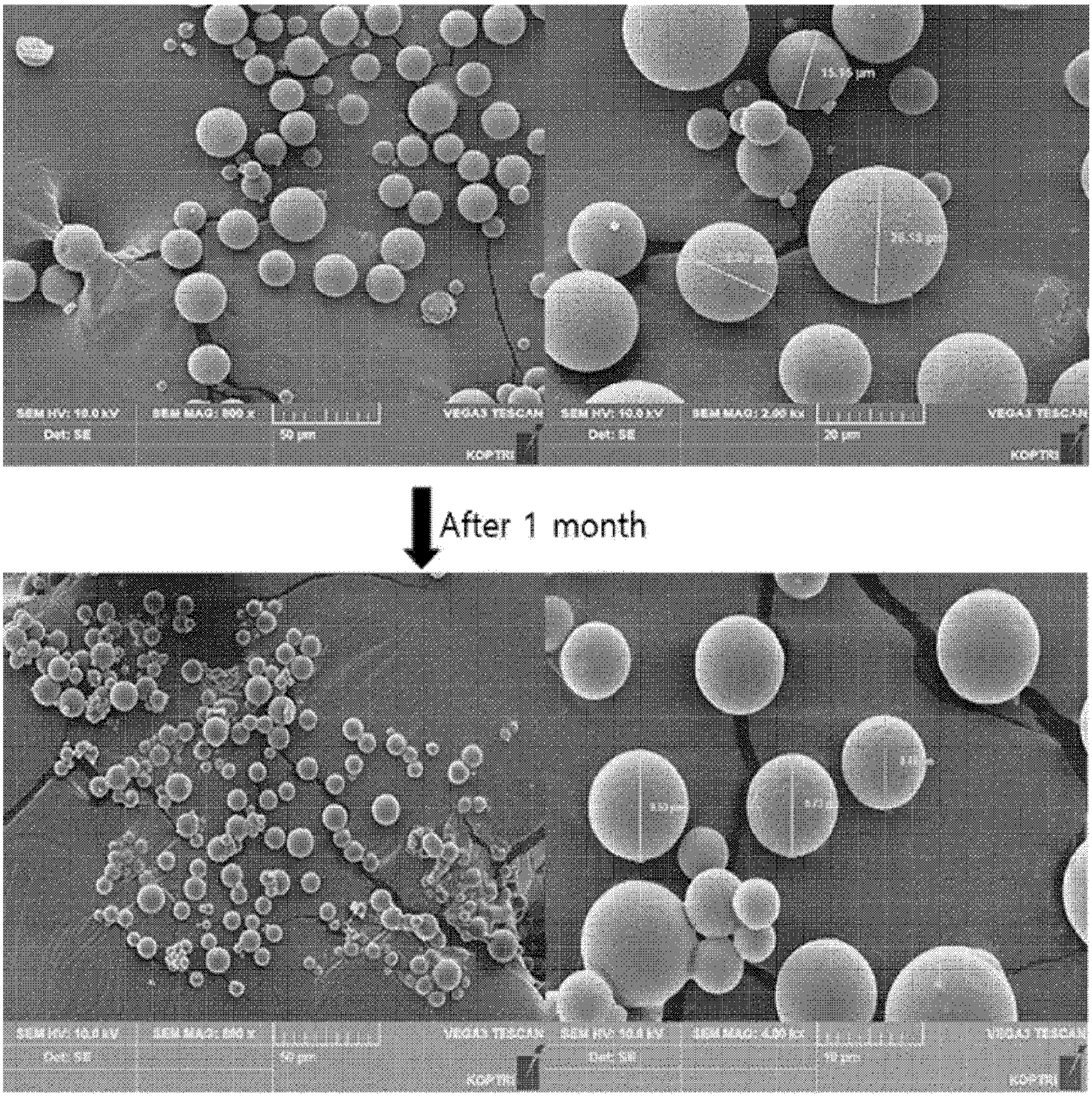

【FIG. 10】

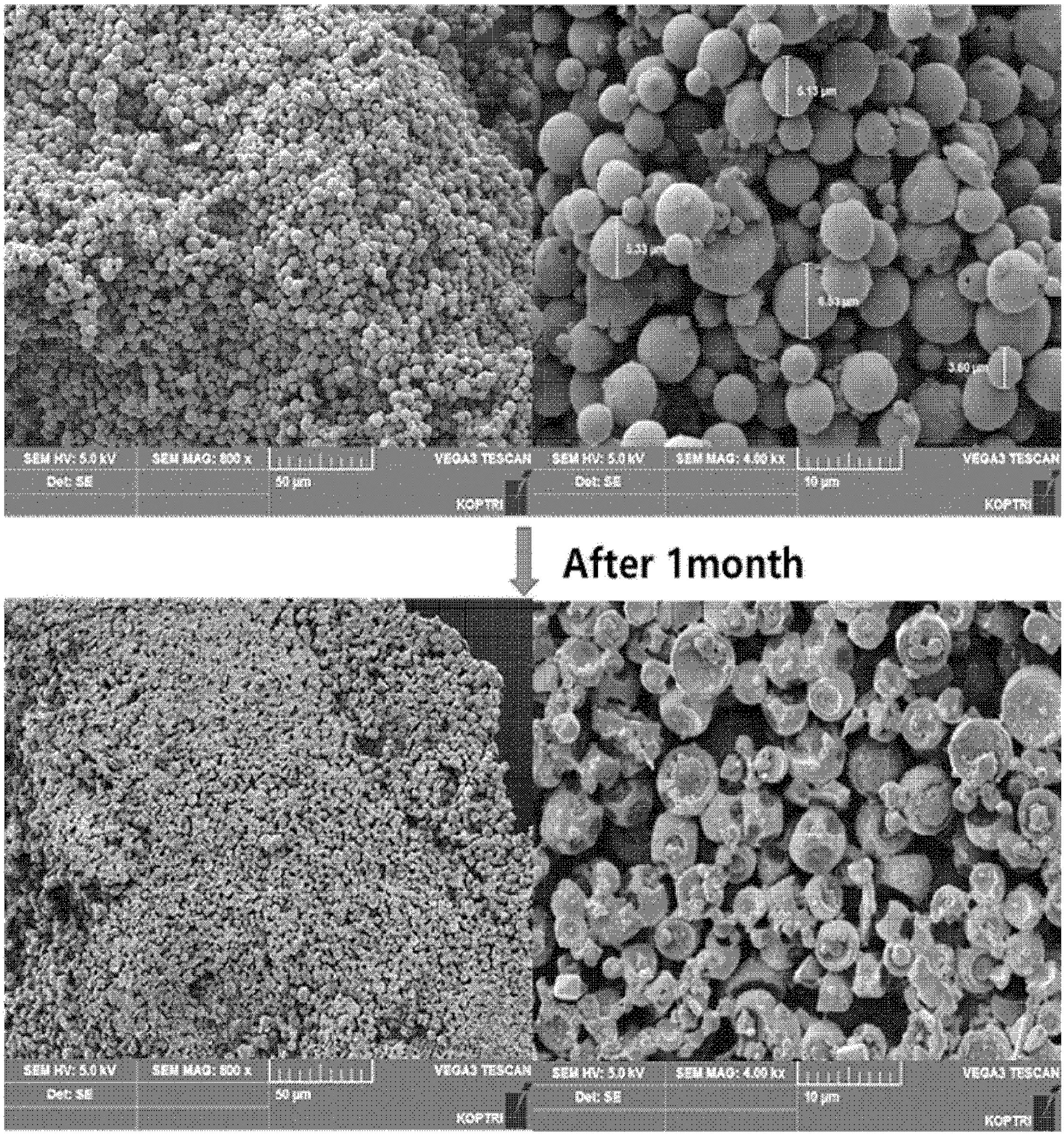
SEM HV: 5.0 kV
SEM MAG: 800 x
VEGA3 TESCAN
Det: SE
50 μm
KOPTRI
SEM HV: 5.0 kV
SEM MAG: 4.00 kx
VEGA3 TESCAN
Det: SE
10 μm
KOPTRI
After 1month
SEM HV: 5.0 kV
SEM MAG: 800 x
VEGA3 TESCAN
Det: SE
50 μm
KOPTRI
SEM HV: 5.0 kV
SEM MAG: 4.00 kx
VEGA3 TESCAN
Det: SE
10 μm
KOPTRI

【FIG. 11】

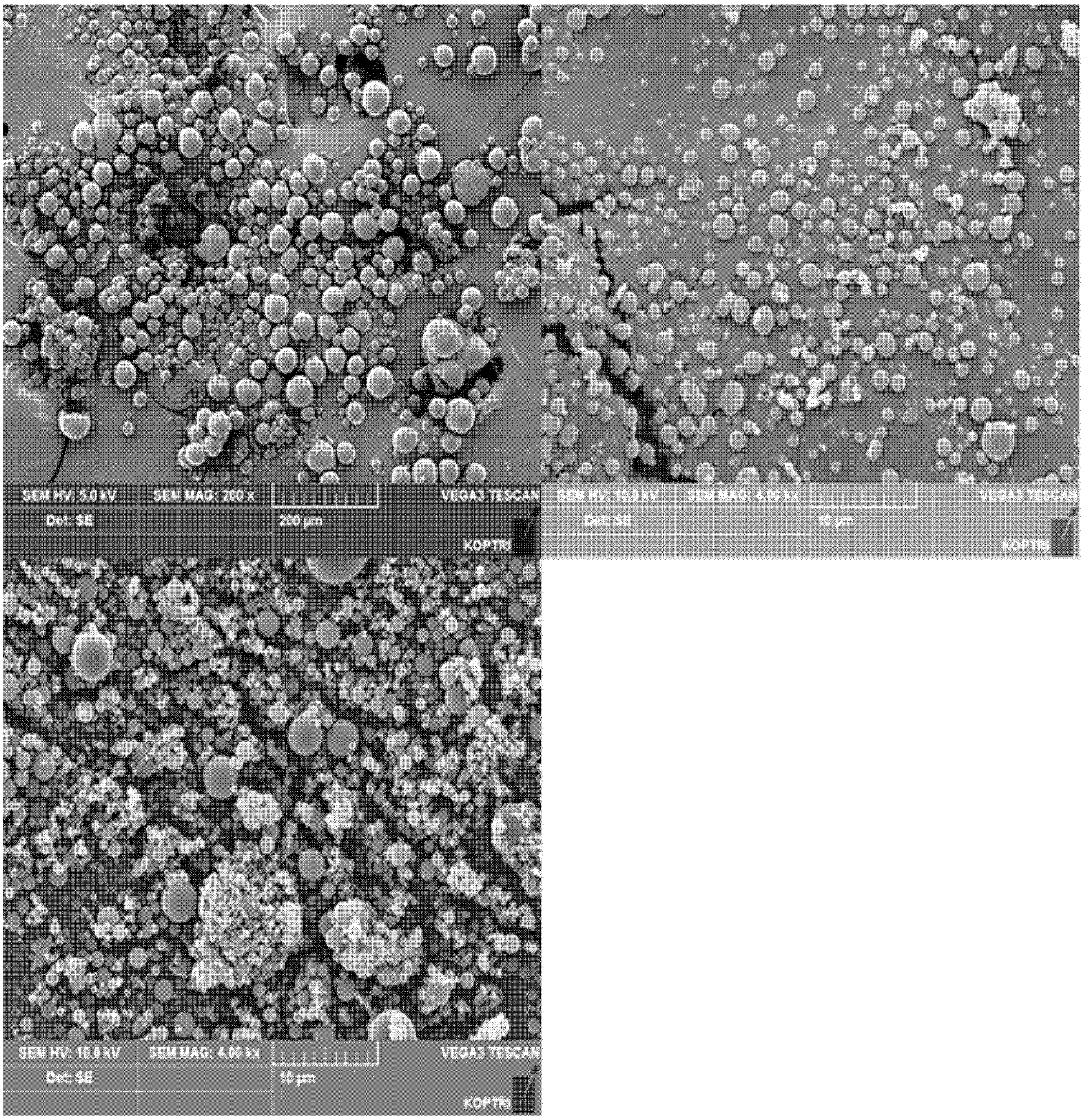
SEM HV: 5.0 kV
SEM MAG: 200 x
VEGA3 TESCAN
Det: SE
200 μm
KOPTRI
SEM HV: 10.0 kV
SEM MAG: 4.00 kx
VEGA3 TESCAN
Det: SE
10 μm
KOPTRI
SEM HV: 10.0 kV
SEM MAG: 4.00 kx
VEGA3 TESCAN
Det: SE
10 μm
KOPTRI

【FIG. 12】

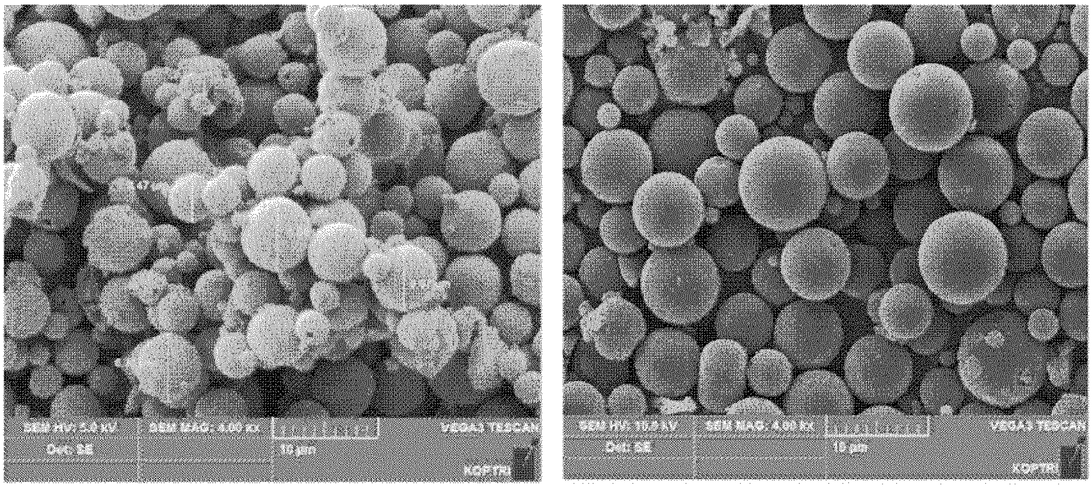

Example 39-B    Example 40-B

【FIG. 13】

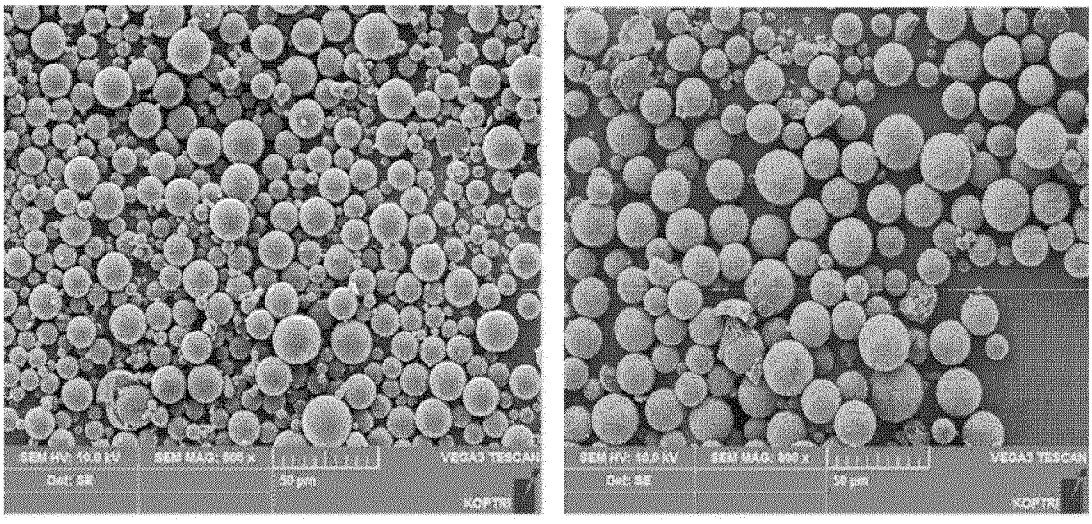

Example 41-B    Example 42-B

【FIG. 14】

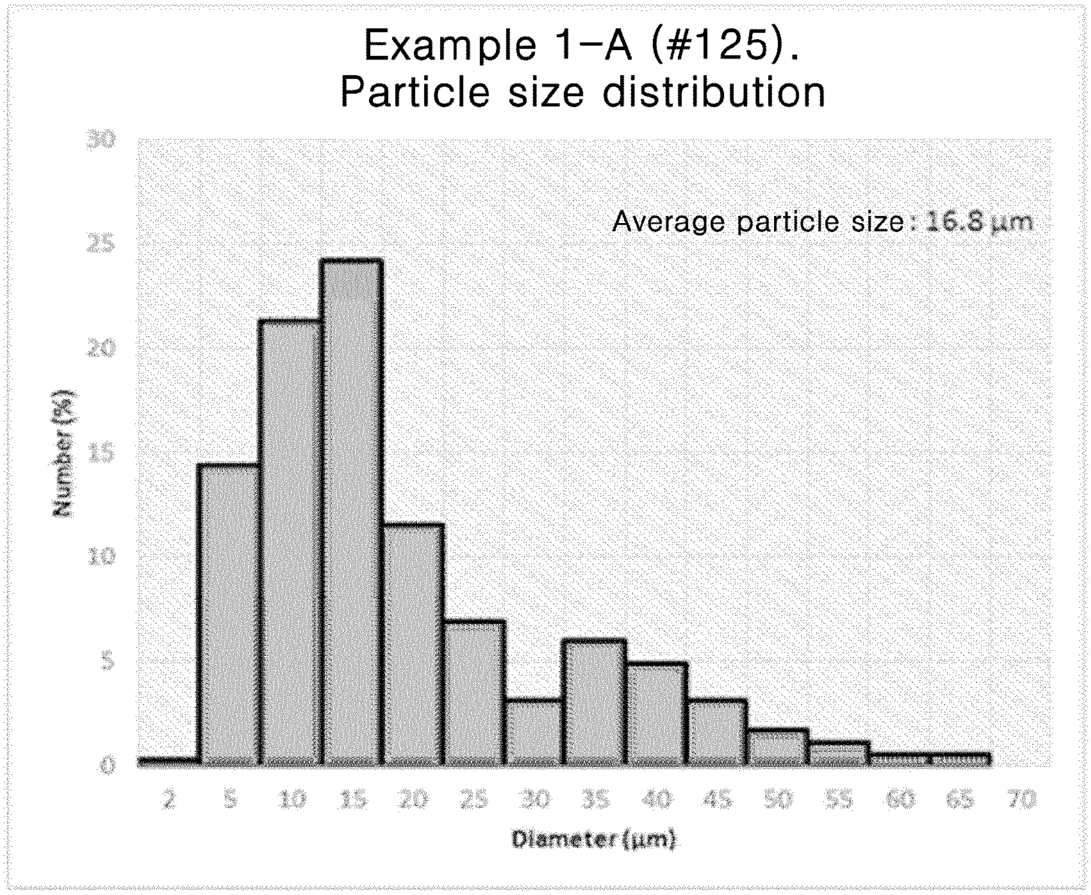
Example 1-A (#125).
Particle size distribution
Average particle size: 16.8 μm
Number (%)
0
5
10
15
20
25
30
Diameter (μm)
2
5
10
15
20
25
30
35
40
45
50
55
60
65
70

【FIG. 15】

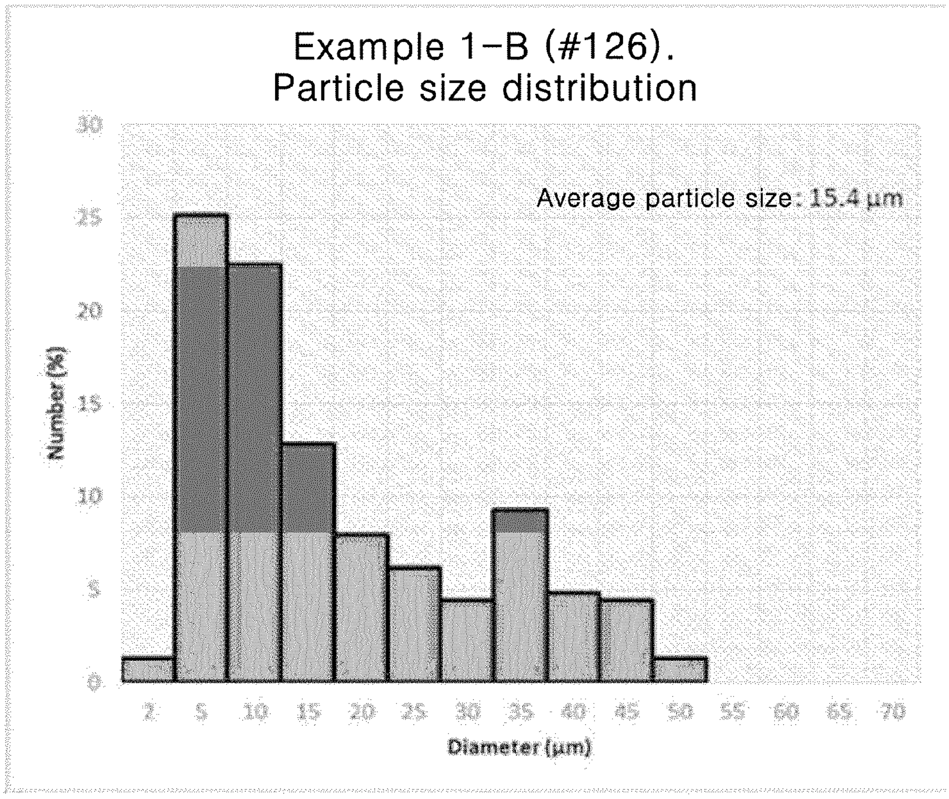
Example 1-B (#126).
Particle size distribution
Average particle size: 15.4 μm
Number (%)
30
25
20
15
10
5
0
2
5
10
15
20
25
30
35
40
45
50
55
60
65
70
Diameter (μm)

【FIG. 16】

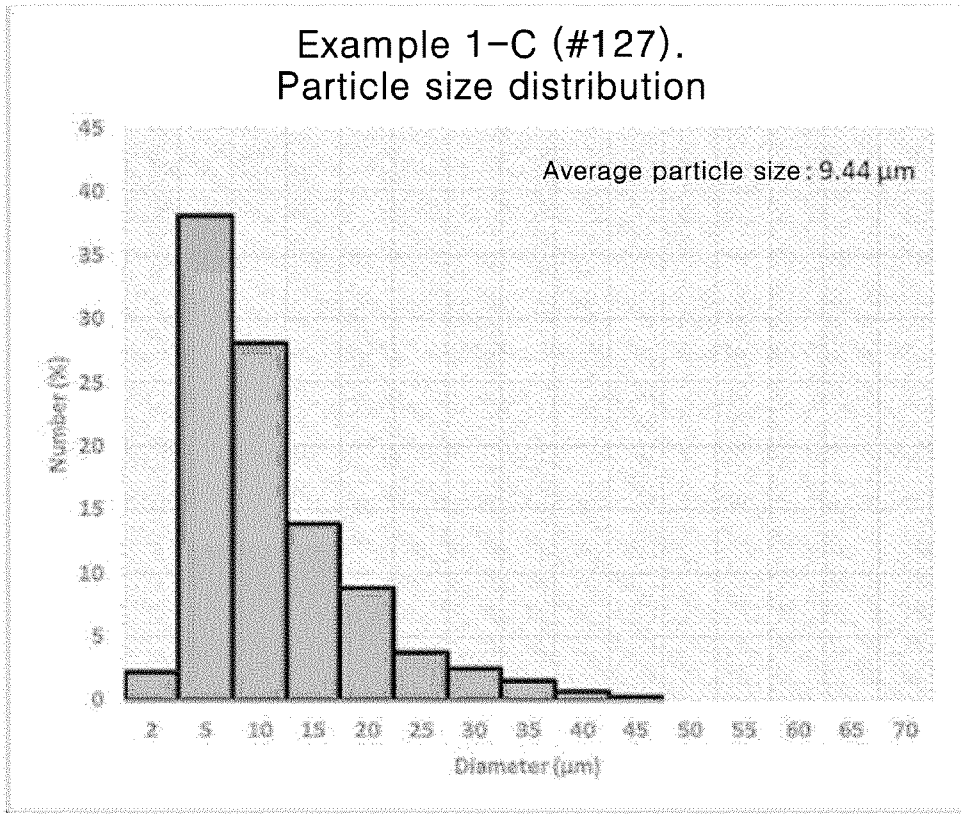
Example 1-C (#127).
Particle size distribution
Average particle size : 9.44 μm
Number (%)
0
5
10
15
20
25
30
35
40
45
2
5
10
15
20
25
30
35
40
45
50
55
60
65
70
Diameter (μm)

【FIG. 17】

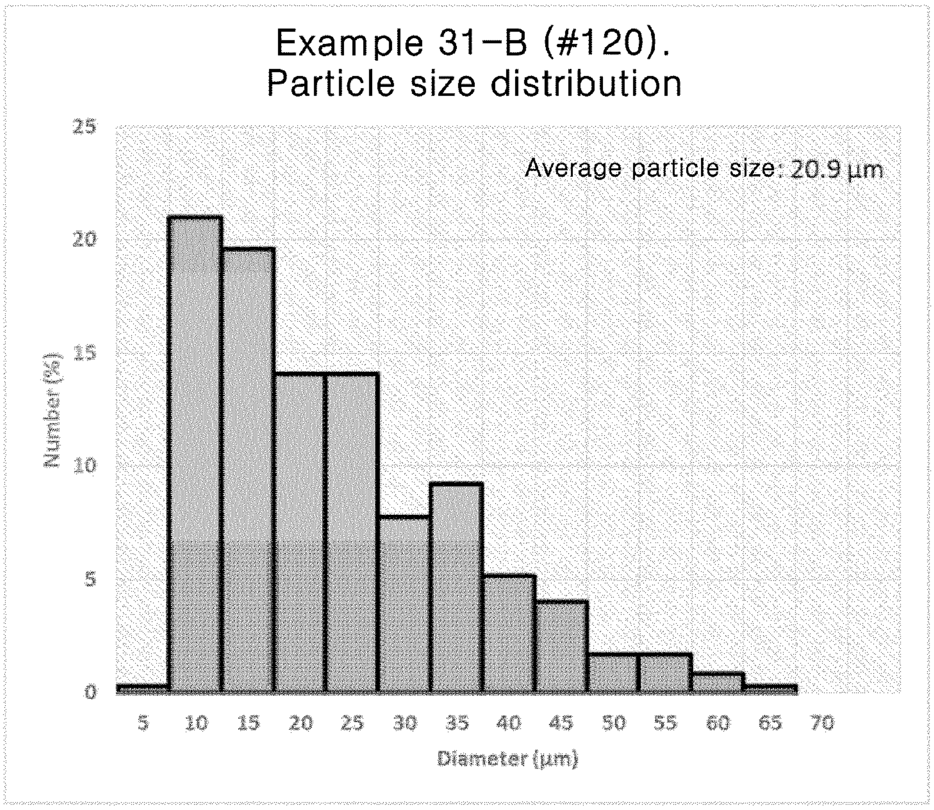
Example 31-B (#120).
Particle size distribution
Average particle size: 20.9 μm
Number (%)
25
20
15
10
5
0
5 10 15 20 25 30 35 40 45 50 55 60 65 70
Diameter (μm)

【FIG. 18】

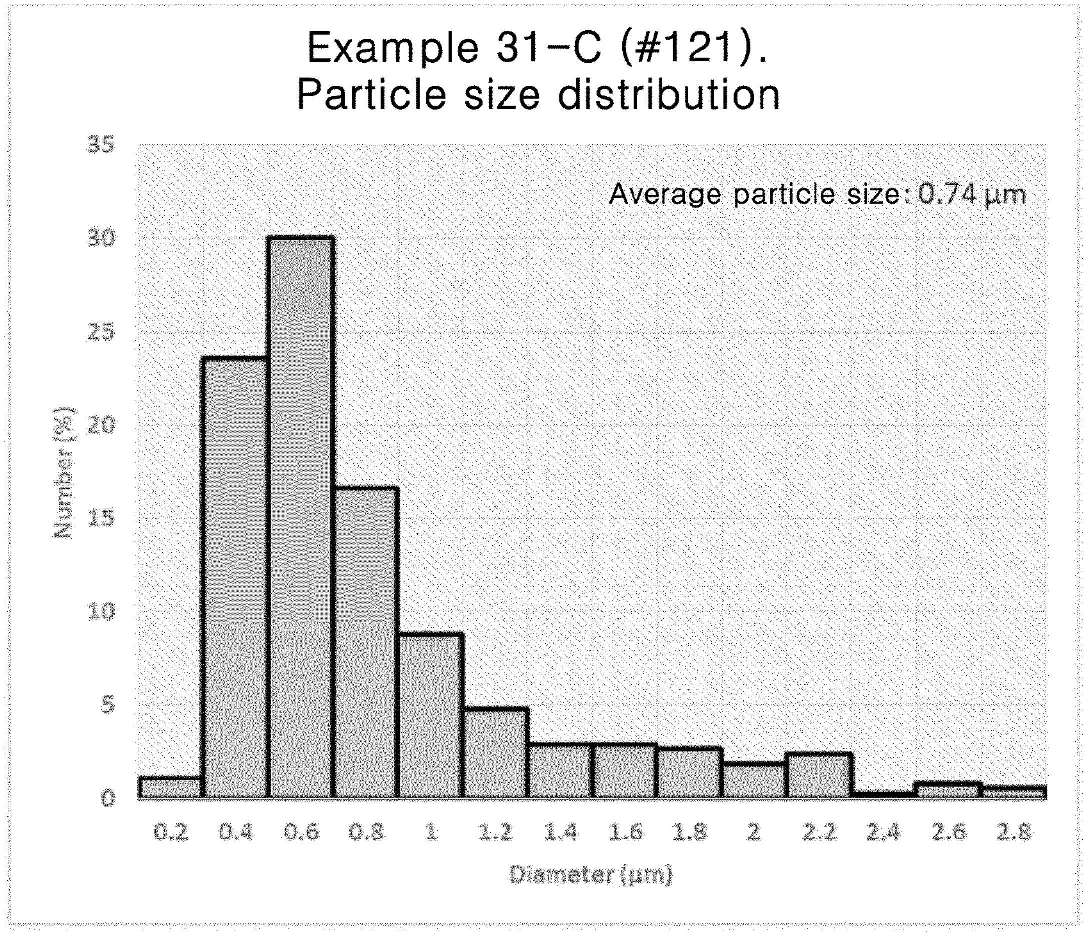
Example 31-C (#121).
Particle size distribution
Average particle size: 0.74 μm
Number (%)
0
5
10
15
20
25
30
35
0.2
0.4
0.6
0.8
1
1.2
1.4
1.6
1.8
2
2.2
2.4
2.6
2.8
Diameter (μm)

【FIG. 19】

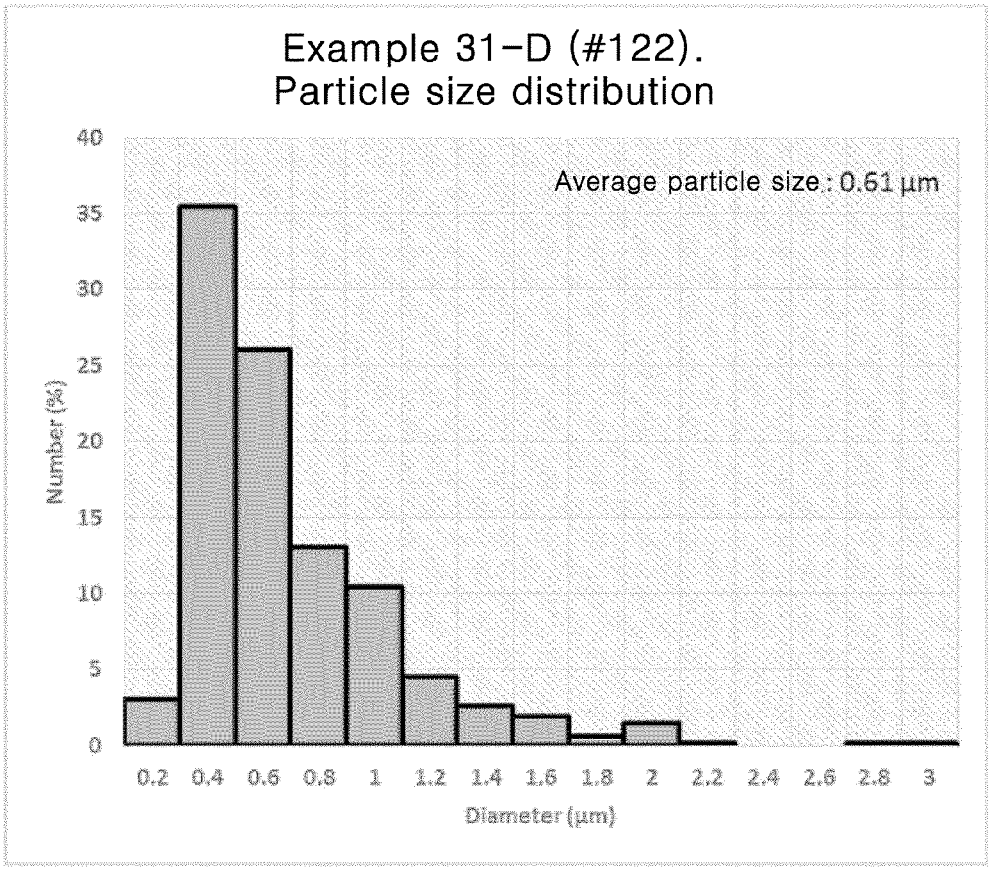
Example 31-D (#122).
Particle size distribution
Average particle size : 0.61 µm
Number (%)
40
35
30
25
20
15
10
5
0
0.2 0.4 0.6 0.8 1 1.2 1.4 1.6 1.8 2 2.2 2.4 2.6 2.8 3
Diameter (µm)

【FIG. 20】

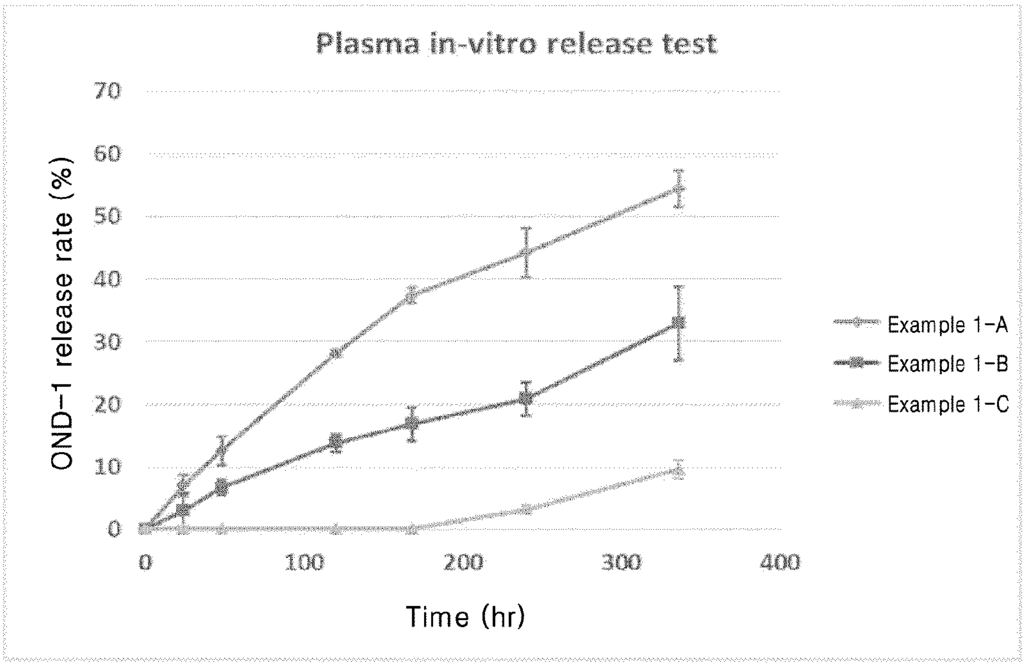
Plasma in-vitro release test
OND-1 release rate (%)
70
60
50
40
30
20
10
0
0
100
200
300
400
Time (hr)
Example 1-A
Example 1-B
Example 1-C

【FIG. 21】

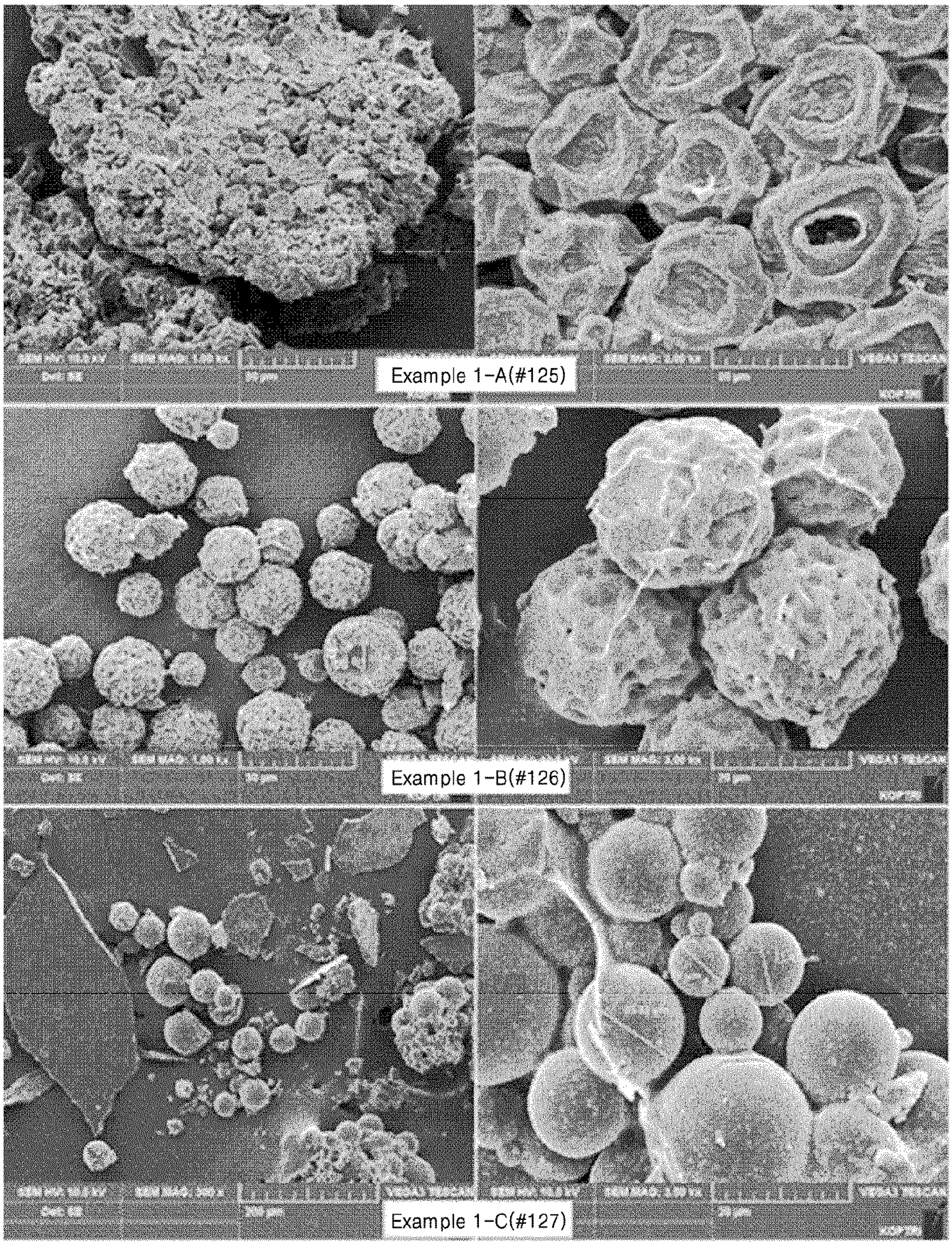

【FIG. 22】

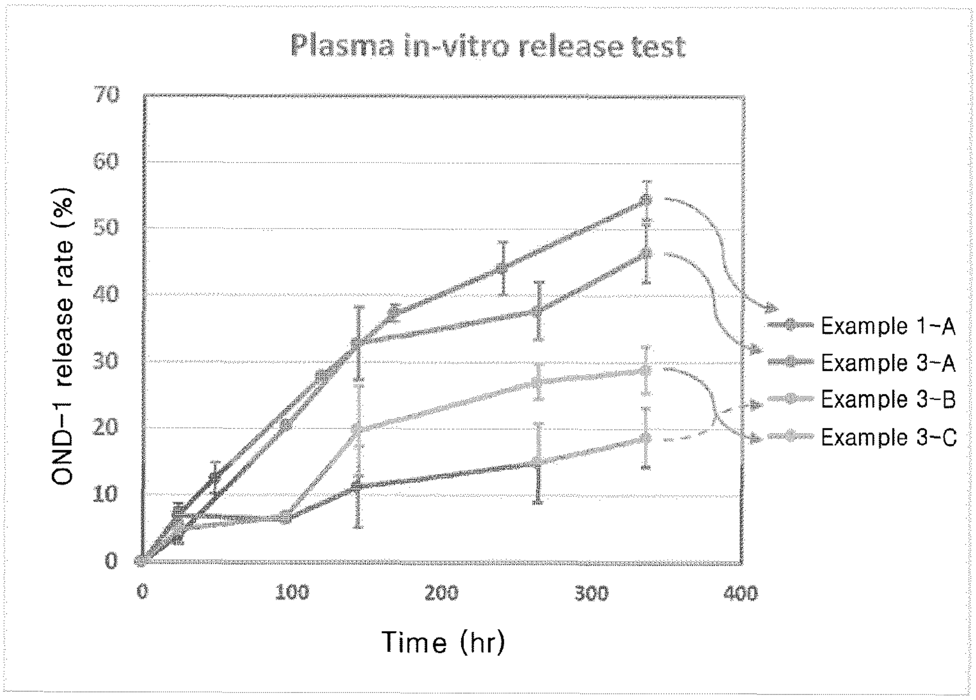
Plasma in-vitro release test
OND-1 release rate (%)
70
60
50
40
30
20
10
0
0
100
200
300
400
Time (hr)
Example 1-A
Example 3-A
Example 3-B
Example 3-C

【FIG. 23】

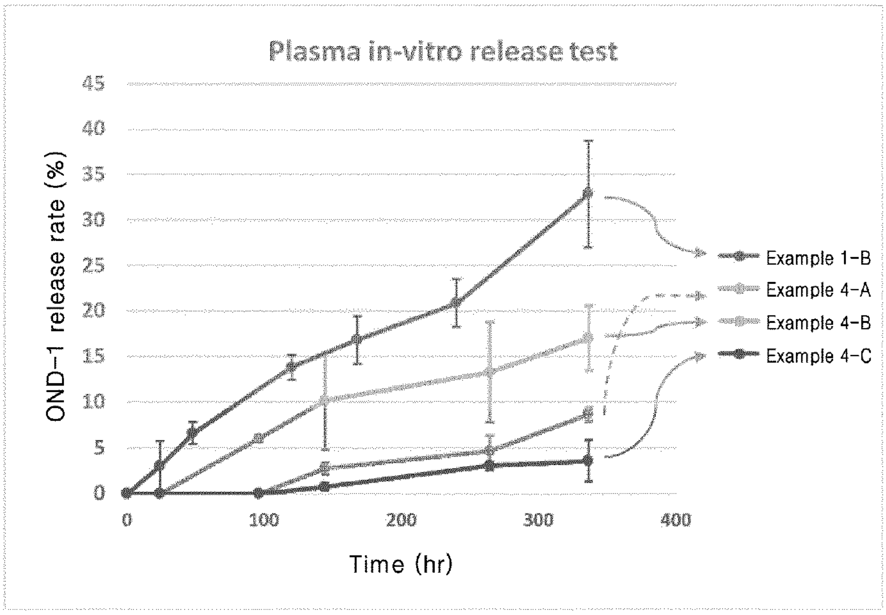
Plasma in-vitro release test
OND-1 release rate (%)
45
40
35
30
25
20
15
10
5
0
0
100
200
300
400
Time (hr)
Example 1-B
Example 4-A
Example 4-B
Example 4-C

【FIG. 24】

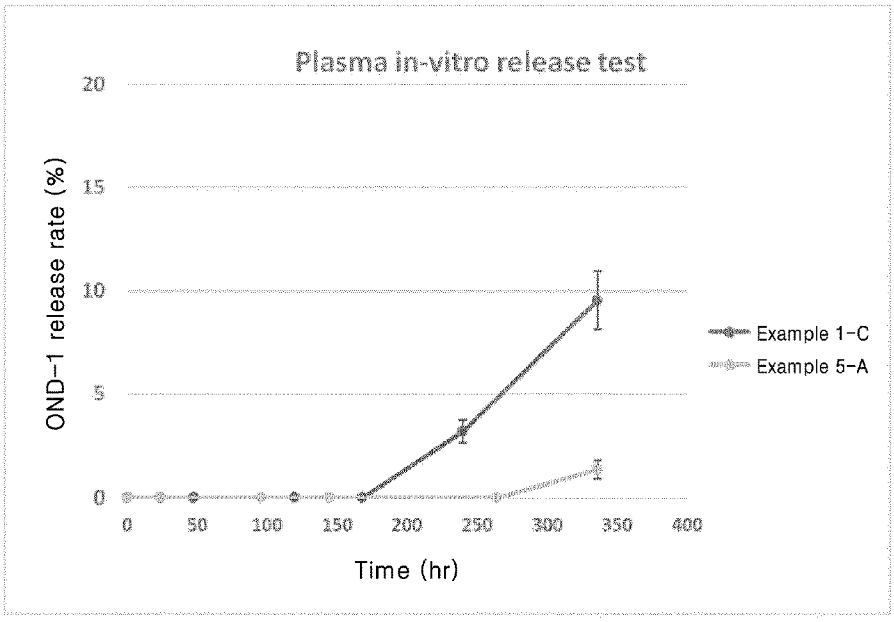
Plasma in-vitro release test
OND-1 release rate (%)
20
15
10
5
0
0
50
100
150
200
250
300
350
400
Time (hr)
Example 1-C
Example 5-A

【FIG. 25】

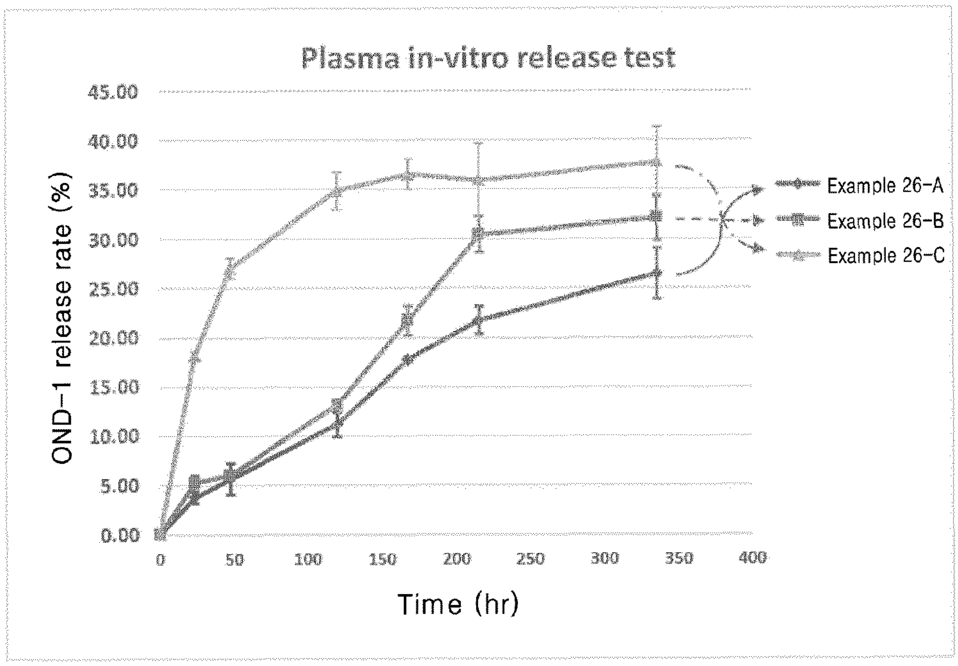
Plasma in-vitro release test
45.00
40.00
35.00
30.00
25.00
20.00
15.00
10.00
5.00
0.00
0
50
100
150
200
250
300
350
400
Time (hr)
OND-1 release rate (%)
Example 26-A
Example 26-B
Example 26-C

【FIG. 26】

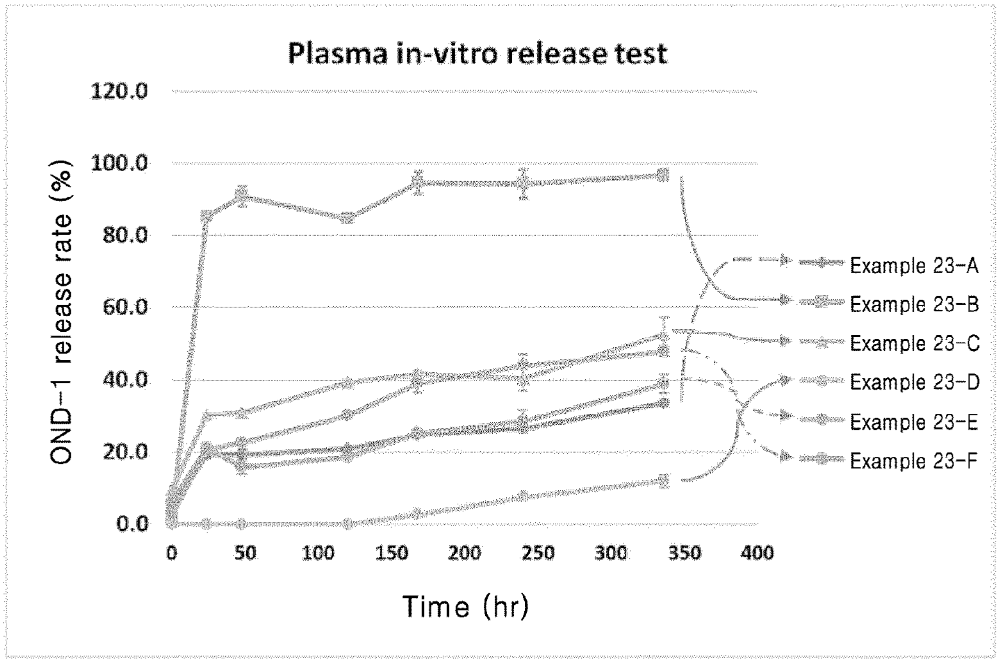

Plasma in-vitro release test
120.0
100.0
80.0
60.0
40.0
20.0
0.0
OND-1 release rate (%)
0
50
100
150
200
250
300
350
400
Time (hr)
Example 23-A
Example 23-B
Example 23-C
Example 23-D
Example 23-E
Example 23-F

【FIG. 27】

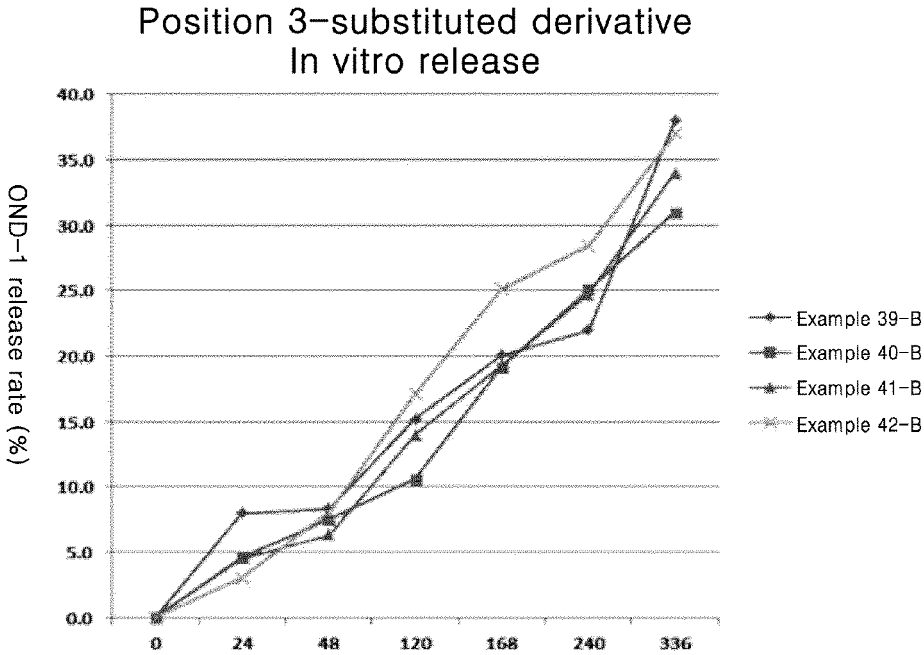
Position 3-substituted derivative
In vitro release
OND-1 release rate (%)
40.0
35.0
30.0
25.0
20.0
15.0
10.0
5.0
0.0
0
24
48
120
168
240
336
Example 39-B
Example 40-B
Example 41-B
Example 42-B

【FIG. 28】

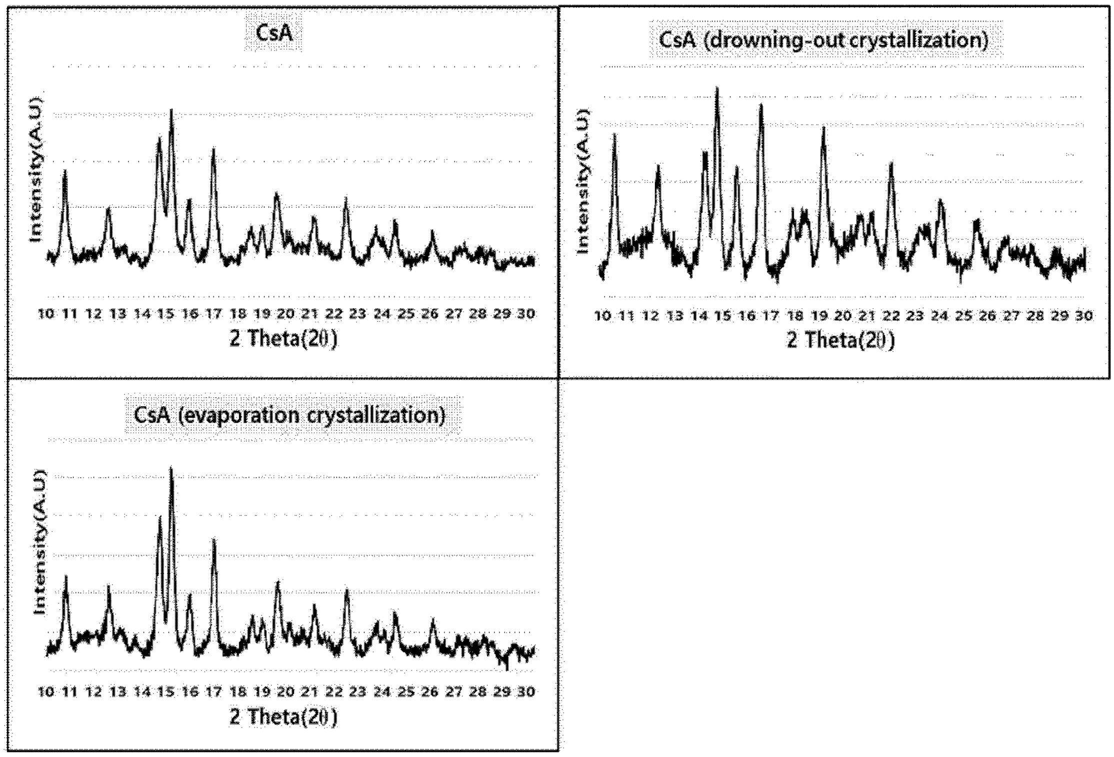
CsA
Intensity(A.U)
10 11 12 13 14 15 16 17 18 19 20 21 22 23 24 25 26 27 28 29 30
2 Theta(2θ)
CsA (drowning-out crystallization)
Intensity(A.U)
10 11 12 13 14 15 16 17 18 19 20 21 22 23 24 25 26 27 28 29 30
2 Theta(2θ)
CsA (evaporation crystallization)
Intensity(A.U)
10 11 12 13 14 15 16 17 18 19 20 21 22 23 24 25 26 27 28 29 30
2 Theta(2θ)

【FIG. 29】

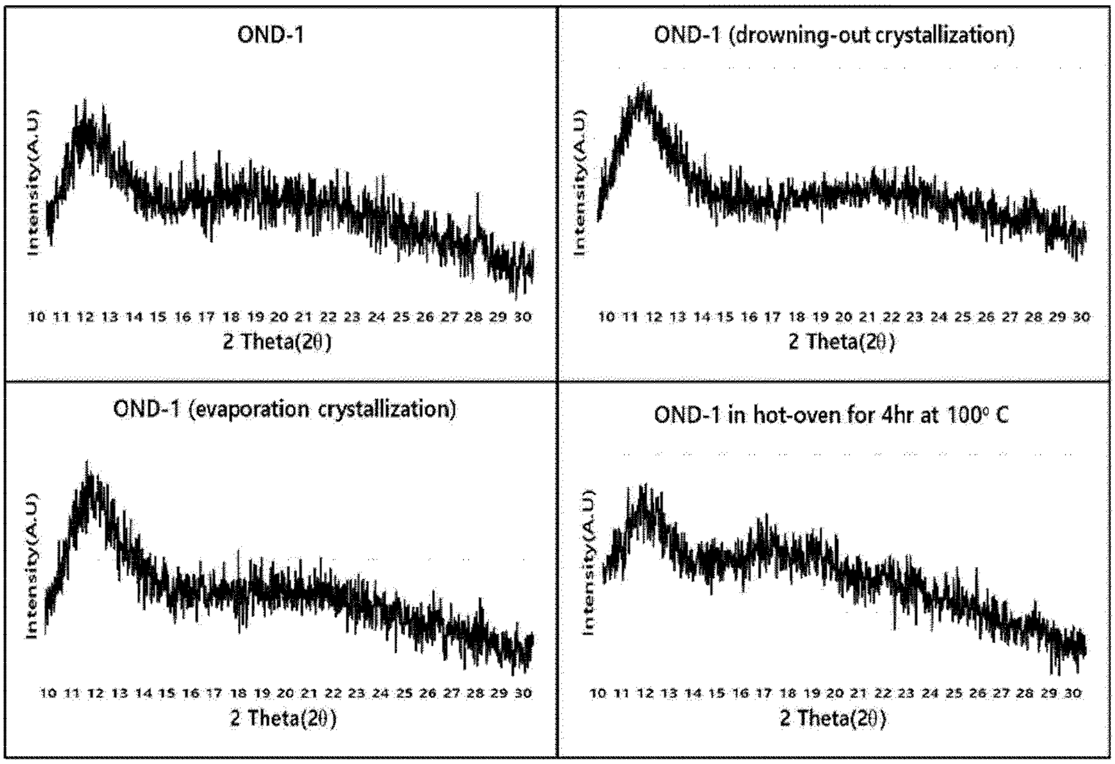
OND-1
Intensity(A.U)
10 11 12 13 14 15 16 17 18 19 20 21 22 23 24 25 26 27 28 29 30
2 Theta(2θ)
OND-1 (drowning-out crystallization)
Intensity(A.U)
10 11 12 13 14 15 16 17 18 19 20 21 22 23 24 25 26 27 28 29 30
2 Theta(2θ)
OND-1 (evaporation crystallization)
Intensity(A.U)
10 11 12 13 14 15 16 17 18 19 20 21 22 23 24 25 26 27 28 29 30
2 Theta(2θ)
OND-1 in hot-oven for 4hr at 100° C
Intensity(A.U)
10 11 12 13 14 15 16 17 18 19 20 21 22 23 24 25 26 27 28 29 30
2 Theta(2θ)

【FIG. 30】

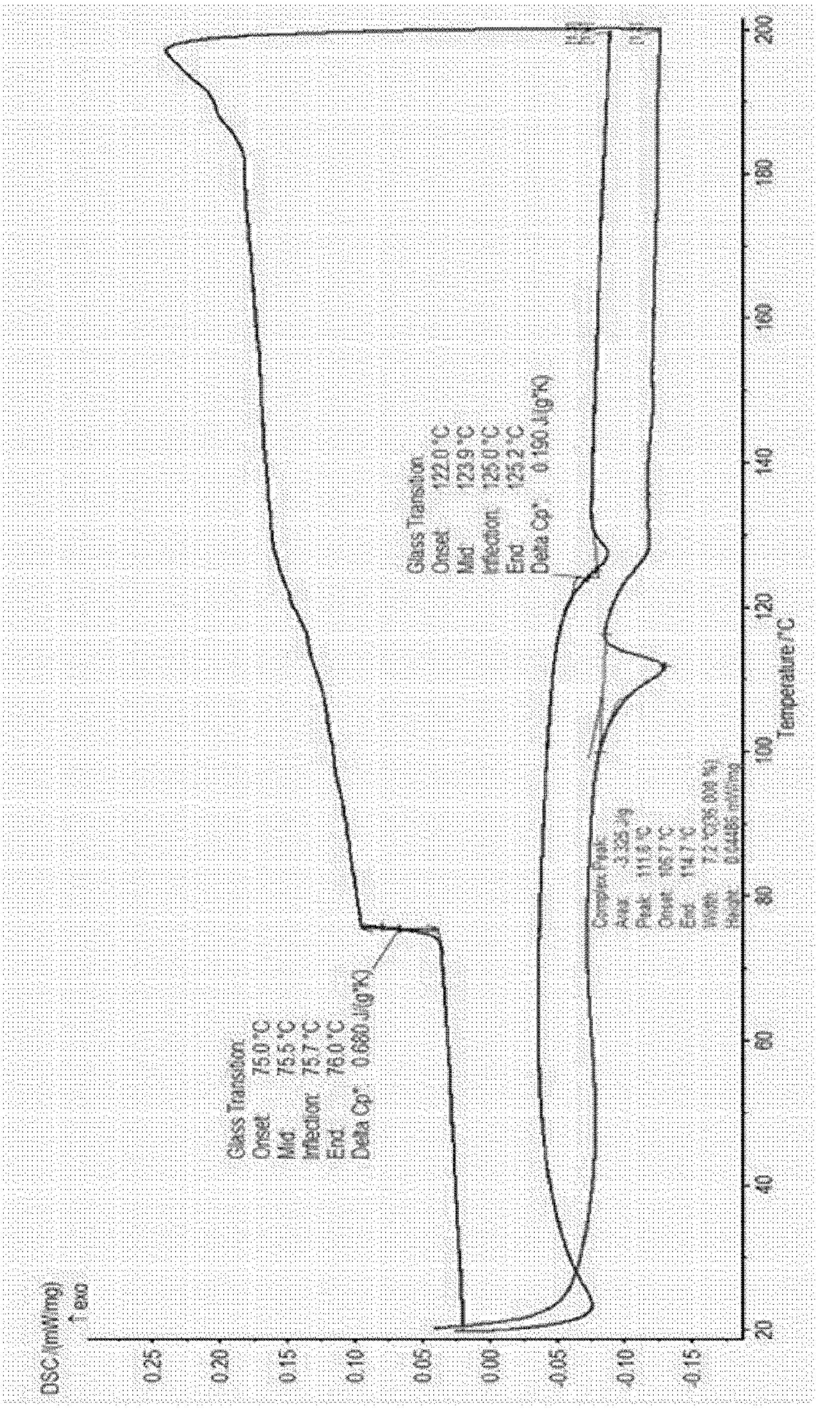
DSC /(mW/mg)
↑ exo
Glass Transition:
Onset: 75.0 °C
Mid: 75.5 °C
Inflection: 75.7 °C
End: 76.0 °C
Delta Cp*: 0.680 J/(g*K)
Glass Transition:
Onset: 122.0 °C
Mid: 123.9 °C
Inflection: 125.0 °C
End: 125.2 °C
Delta Cp*: 0.190 J/(g*K)
Complex Peak:
Area: -3.326 J/g
Peak: 111.8 °C
Onset: 105.7 °C
End: 114.7 °C
Temperature /°C

【FIG. 31】

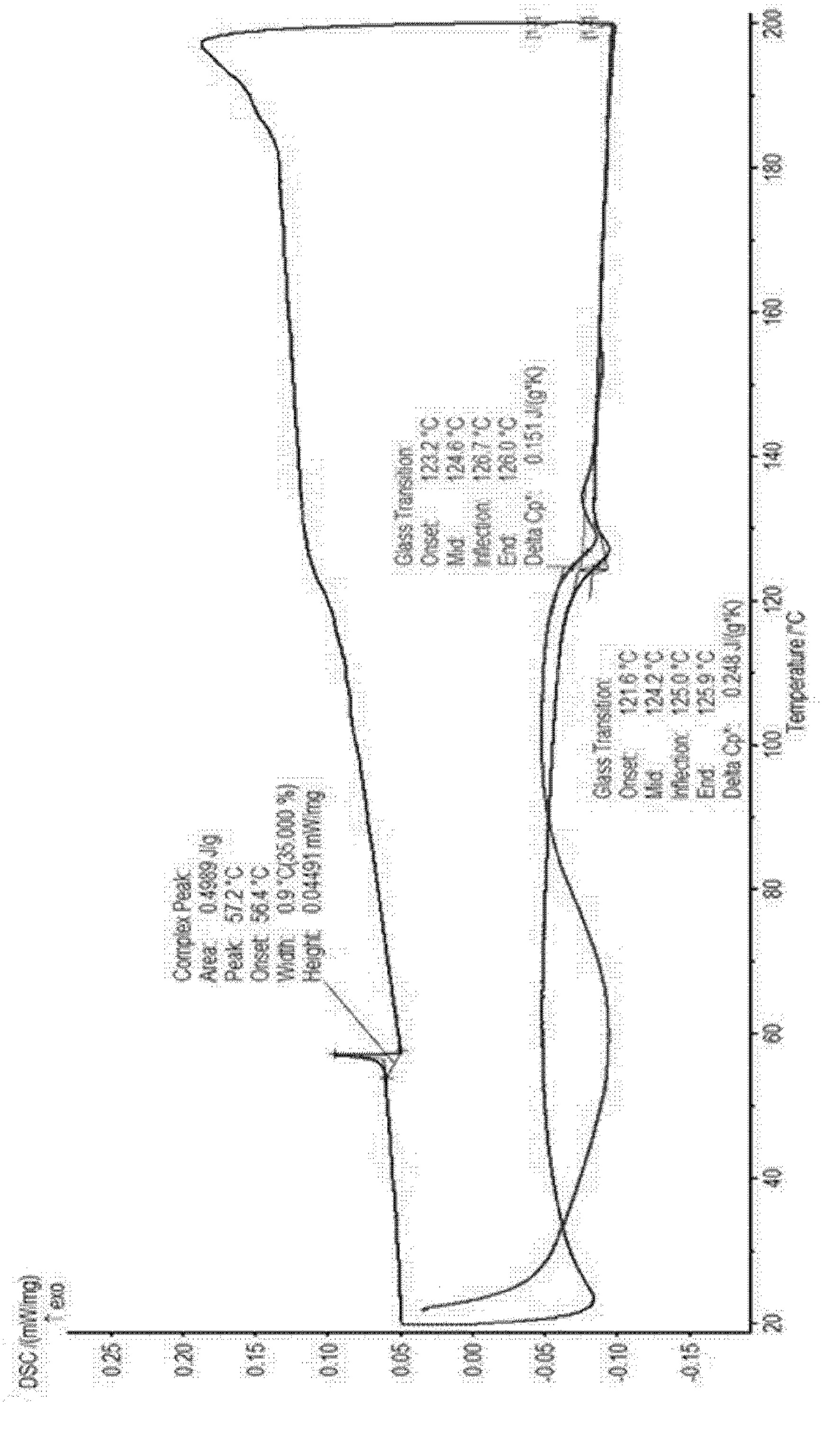
DSC /(mW/mg)
↑ exo
0.25
0.20
0.15
0.10
0.05
0.00
-0.05
-0.10
-0.15
20
40
60
80
100
120
140
160
180
200
Temperature /°C
Complex Peak:
Area: 0.4989 J/g
Peak: 57.2 °C
Onset: 56.4 °C
Width: 0.9 °C(35.000 %)
Height: 0.04491 mW/mg
Glass Transition:
Onset: 121.6 °C
Mid: 124.2 °C
Inflection: 125.0 °C
End: 125.9 °C
Delta Cp*: 0.248 J/(g*K)
Glass Transition:
Onset: 123.2 °C
Mid: 124.6 °C
Inflection: 126.7 °C
End: 126.0 °C
Delta Cp*: 0.151 J/(g*K)

【FIG. 32】

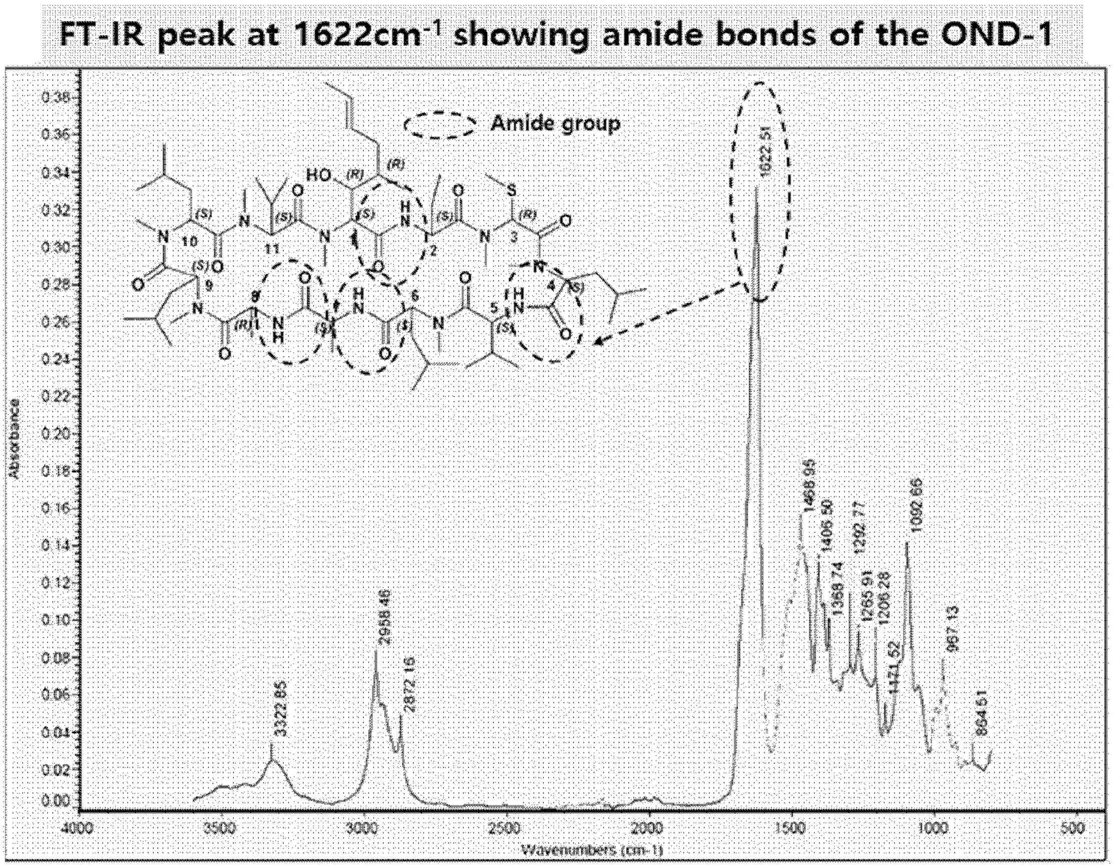

FT-IR peak at 1622cm-1 showing amide bonds of the OND-1
Amide group
Absorbance
Wavenumbers (cm-1)
1622.51
1468.95
1406.50
1368.74
1292.77
1265.91
1206.28
1171.52
1092.66
967.13
864.51
3322.85
2958.46
2872.16

【FIG. 33】

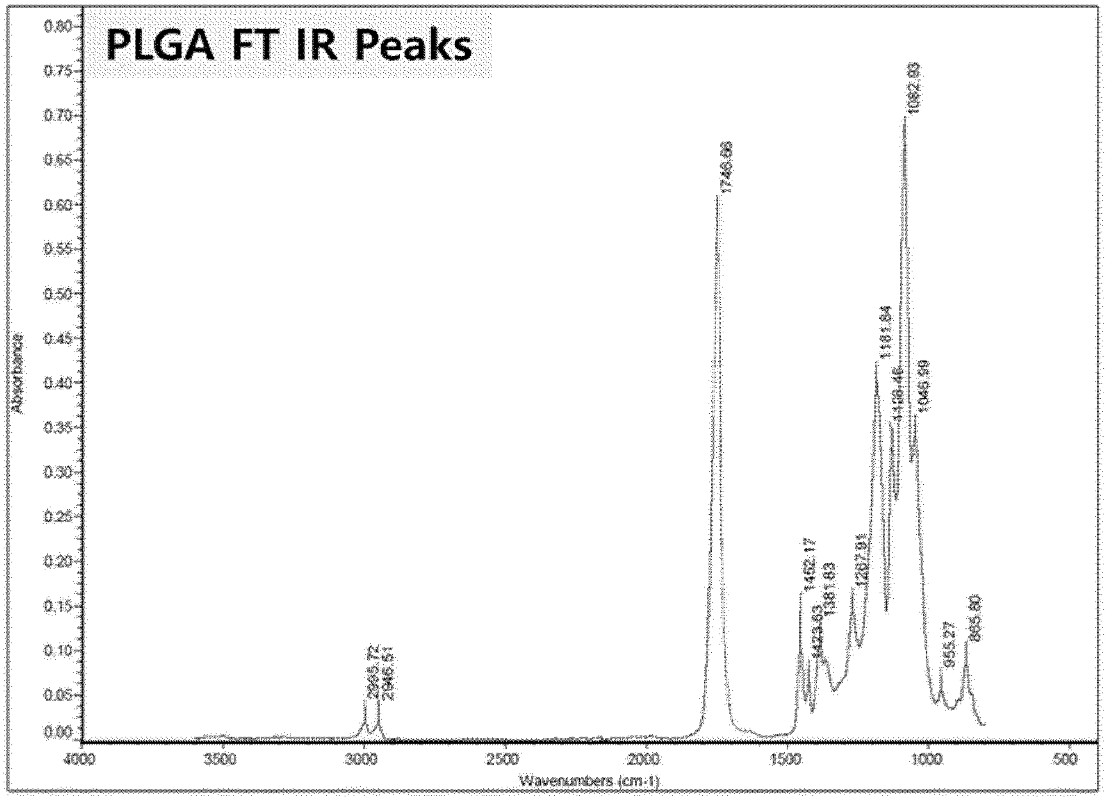
PLGA FT IR Peaks
Absorbance
Wavenumbers (cm-1)
1746.86
1082.93
1181.84
1128.46
1046.99
1452.17
1423.63
1381.83
1267.91
955.27
865.80
2995.72
2946.51

【FIG. 34】

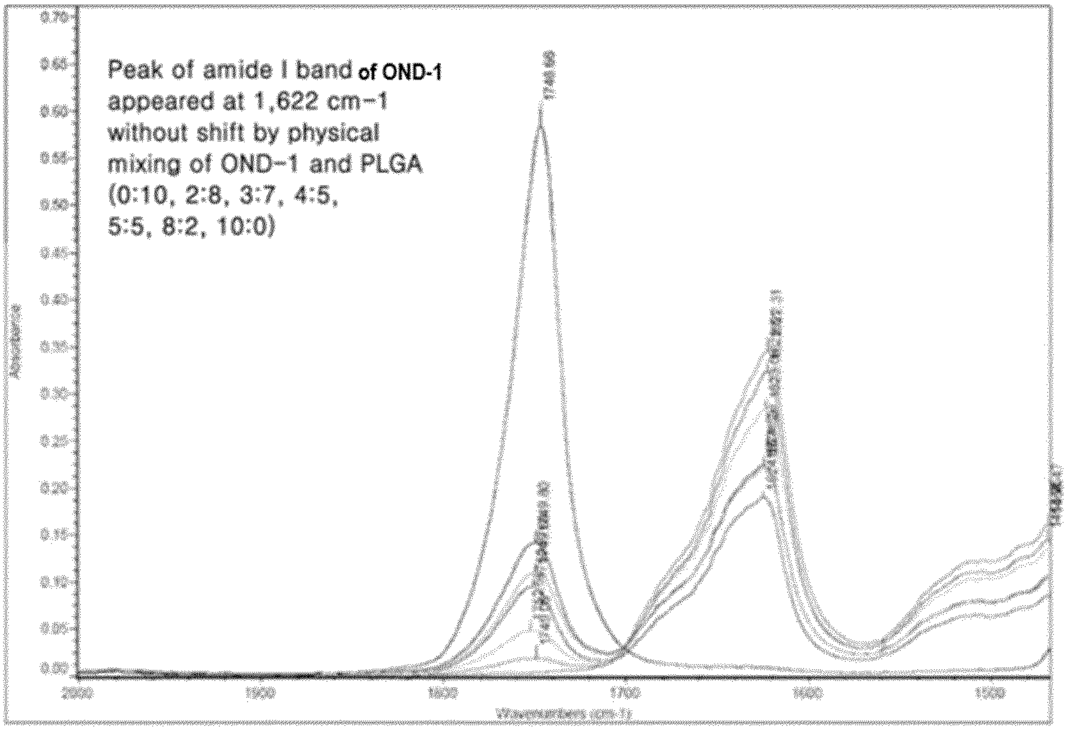
Peak of amide I band of OND-1
appeared at 1,622 cm−1
without shift by physical
mixing of OND-1 and PLGA
(0:10, 2:8, 3:7, 4:5,
5:5, 8:2, 10:0)
Absorbance
Wavenumbers (cm-1)

【FIG. 35】

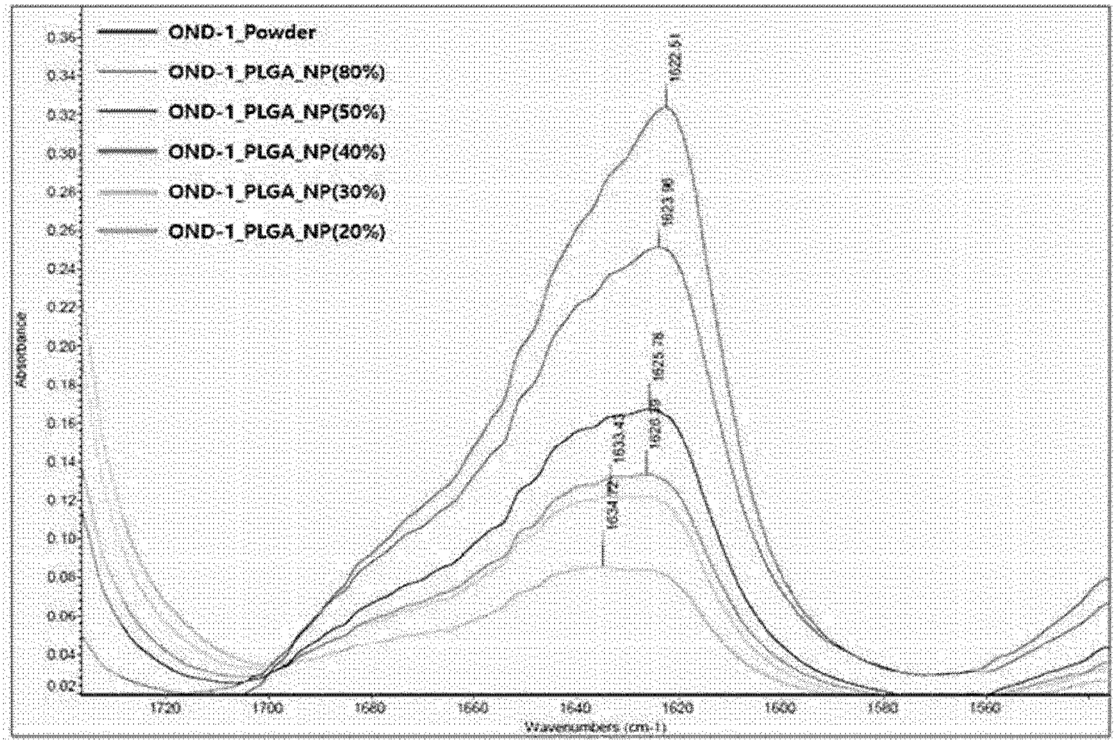
OND-1_Powder
OND-1_PLGA_NP(80%)
OND-1_PLGA_NP(50%)
OND-1_PLGA_NP(40%)
OND-1_PLGA_NP(30%)
OND-1_PLGA_NP(20%)
1622.51
1623.96
1625.78
1626.29
1633.43
1634.72
Absorbance
Wavenumbers (cm-1)

【FIG. 36】

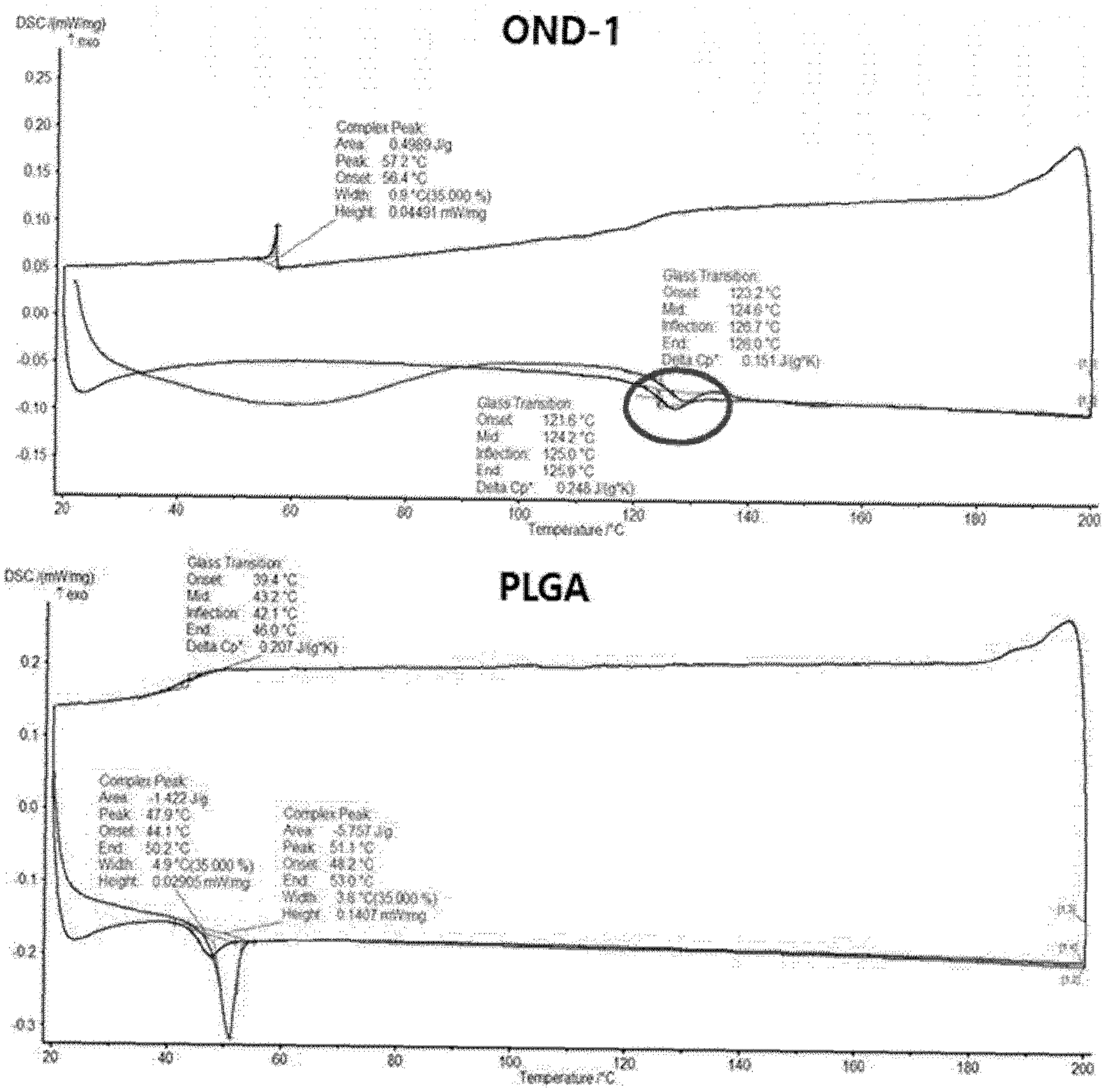
OND-1
DSC /(mW/mg)
↑ exo
Complex Peak:
Area: 0.4969 J/g
Peak: 57.2 °C
Onset: 56.4 °C
Width: 0.9 °C(35.000 %)
Height: 0.04491 mW/mg
Glass Transition:
Onset: 123.2 °C
Mid: 124.6 °C
Inflection: 126.7 °C
End: 126.0 °C
Delta Cp*: 0.151 J/(g*K)
Glass Transition:
Onset: 121.6 °C
Mid: 124.2 °C
Inflection: 125.0 °C
End: 125.9 °C
Delta Cp*: 0.248 J/(g*K)
Temperature /°C
PLGA
DSC /(mW/mg)
↑ exo
Glass Transition:
Onset: 39.4 °C
Mid: 43.2 °C
Inflection: 42.1 °C
End: 46.0 °C
Delta Cp*: 0.207 J/(g*K)
Complex Peak:
Area: -1.422 J/g
Peak: 47.9 °C
Onset: 44.1 °C
End: 50.2 °C
Width: 4.9 °C(35.000 %)
Height: 0.02905 mW/mg
Complex Peak:
Area: -5.757 J/g
Peak: 51.1 °C
Onset: 48.2 °C
End: 53.0 °C
Width: 3.6 °C(35.000 %)
Height: 0.1407 mW/mg
Temperature /°C

【FIG. 37】

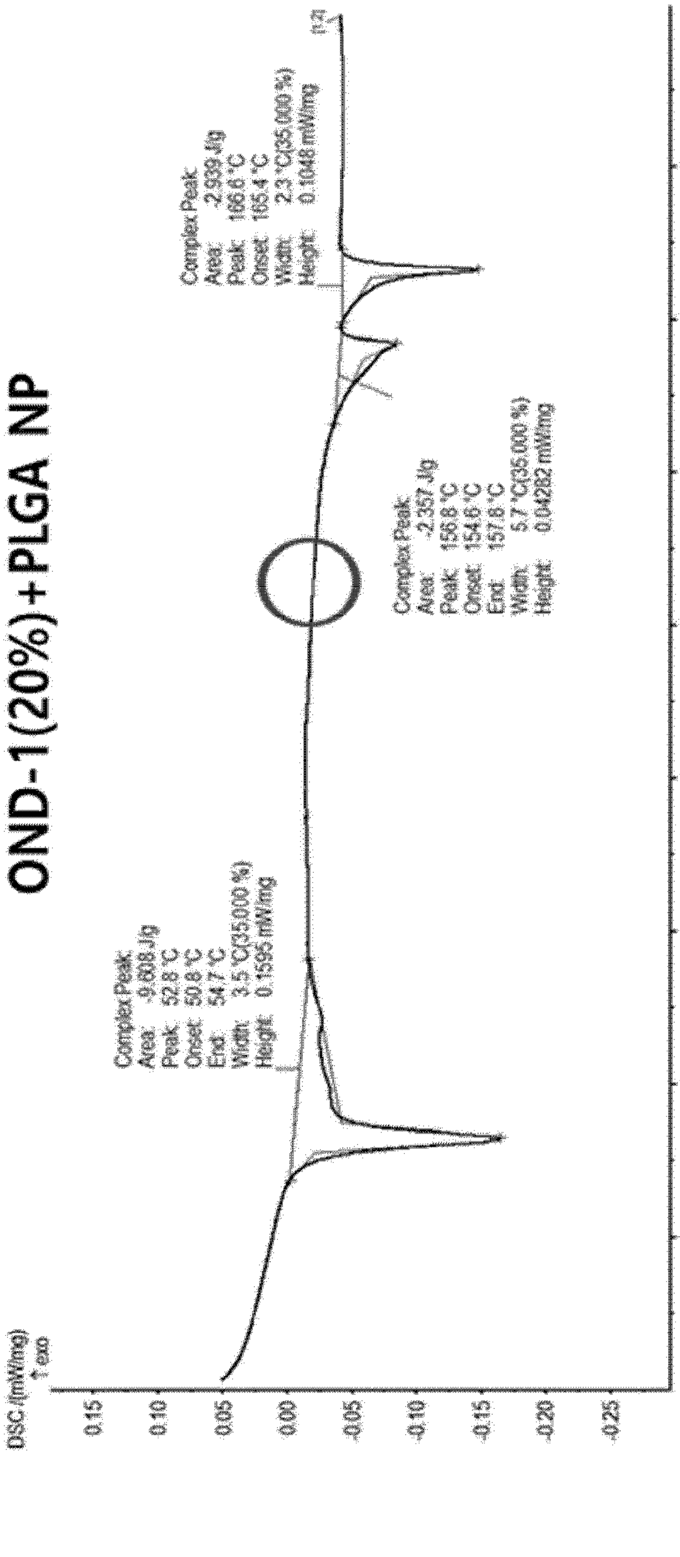
OND-1(20%)+PLGA NP
DSC /(mW/mg)
↑ exo
0.15
0.10
0.05
0.00
-0.05
-0.10
-0.15
-0.20
-0.25
40
60
80
100
120
140
160
180
Temperature /°C
Complex Peak:
Area: -9.608 J/g
Peak: 52.8 °C
Onset: 50.8 °C
End: 54.7 °C
Width: 3.5 °C(35.000 %)
Height: 0.1595 mW/mg
Complex Peak:
Area: -2.357 J/g
Peak: 156.8 °C
Onset: 154.6 °C
End: 157.8 °C
Width: 5.7 °C(35.000 %)
Height: 0.04282 mW/mg
Complex Peak:
Area: -2.939 J/g
Peak: 166.6 °C
Onset: 165.4 °C
Width: 2.3 °C(35.000 %)
Height: 0.1048 mW/mg

【FIG. 38】

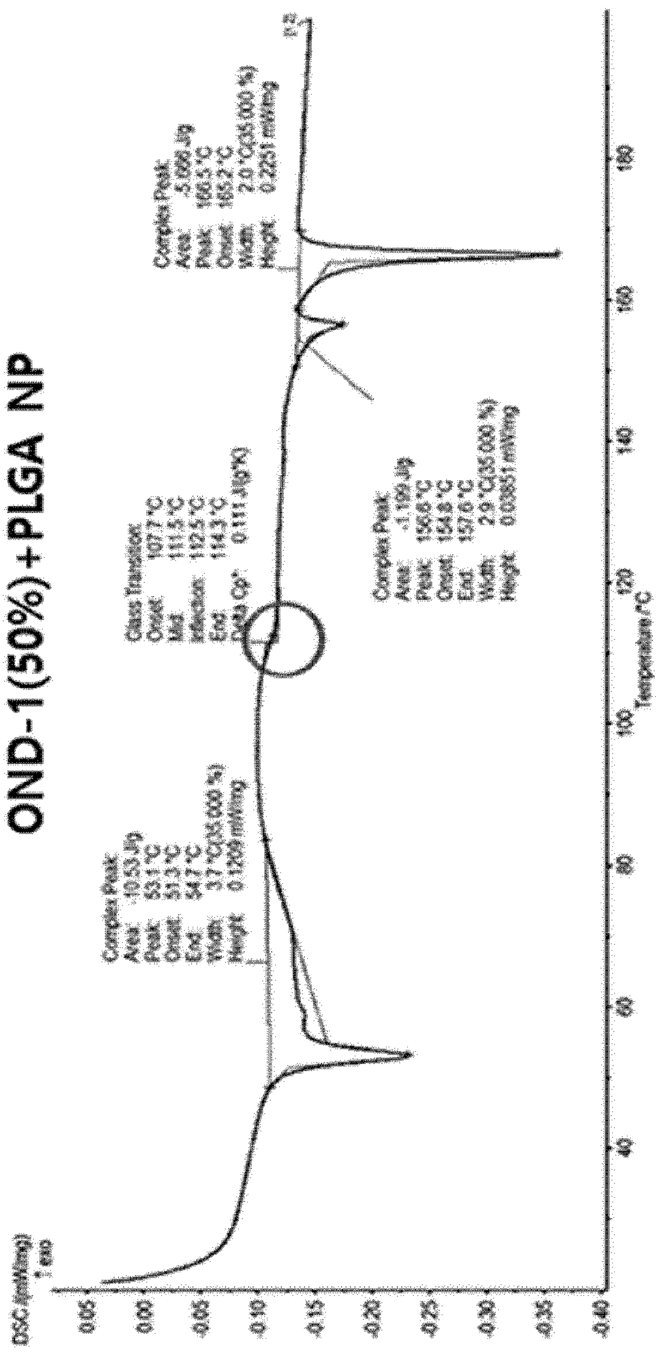
OND-1(50%)+PLGA NP
DSC /(mW/mg)
↑ exo
0.05
0.00
-0.05
-0.10
-0.15
-0.20
-0.25
-0.30
-0.35
-0.40
Complex Peak:
Area: -10.53 J/g
Peak: 53.1 °C
Onset: 51.3 °C
End: 54.7 °C
Width: 3.7 °C(35.000 %)
Height: 0.1206 mW/mg
Glass Transition:
Onset: 107.7 °C
Mid: 111.5 °C
Inflection: 112.5 °C
End: 114.3 °C
Delta Cp*: 0.111 J/(g*K)
Complex Peak:
Area: -1.199 J/g
Peak: 156.6 °C
Onset: 154.8 °C
End: 157.6 °C
Width: 2.9 °C(35.000 %)
Height: 0.03851 mW/mg
Complex Peak:
Area: -5.086 J/g
Peak: 166.5 °C
Onset: 165.2 °C
Width: 2.0 °C(35.000 %)
Height: 0.2251 mW/mg
40
60
80
100
120
140
160
180
Temperature /°C

【FIG. 39】

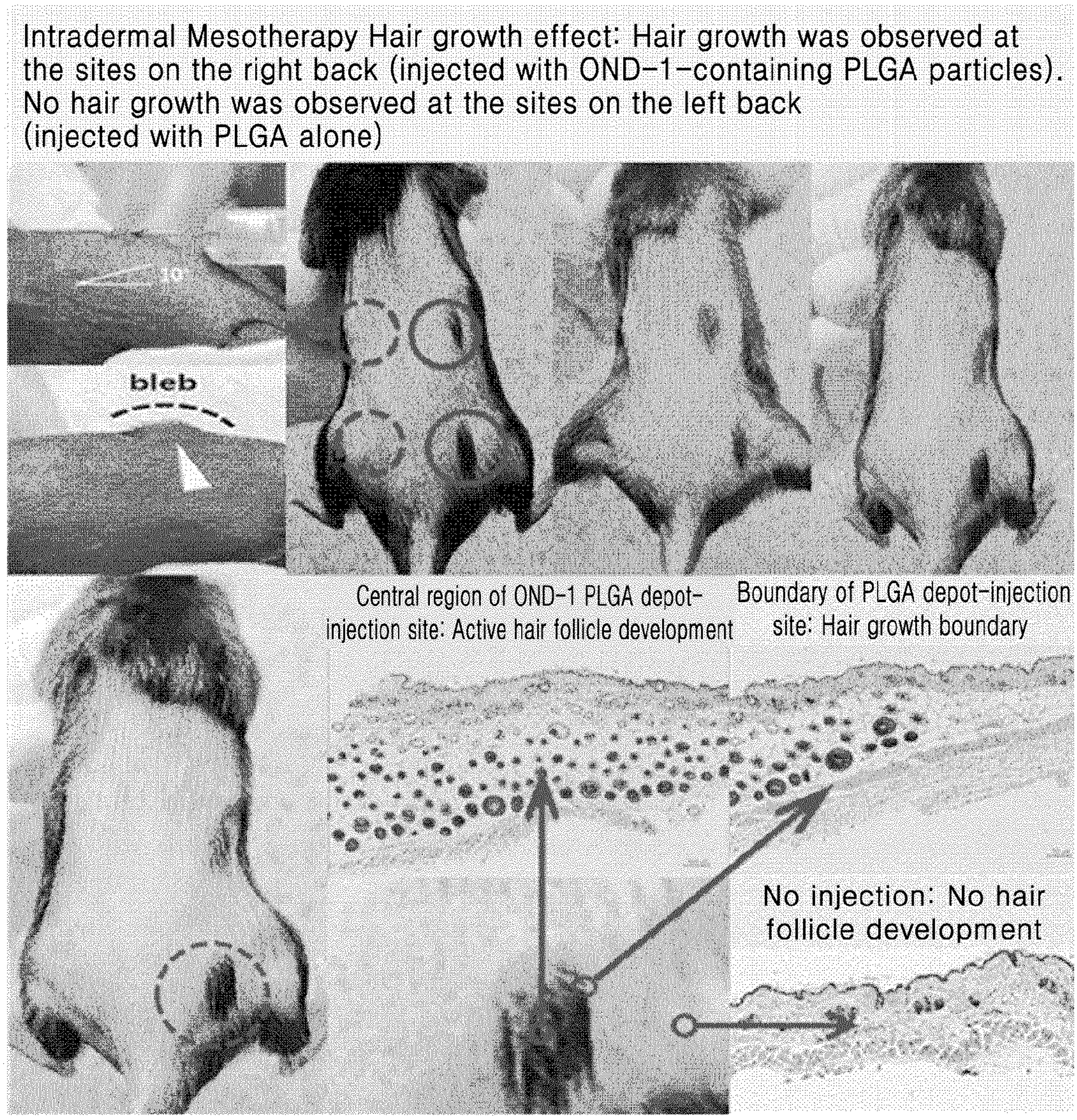
Intradermal Mesotherapy Hair growth effect: Hair growth was observed at the sites on the right back (injected with OND-1-containing PLGA particles). No hair growth was observed at the sites on the left back (injected with PLGA alone)
10°
bleb
Central region of OND-1 PLGA depot-injection site: Active hair follicle development
Boundary of PLGA depot-injection site: Hair growth boundary
No injection: No hair follicle development

【FIG. 40】

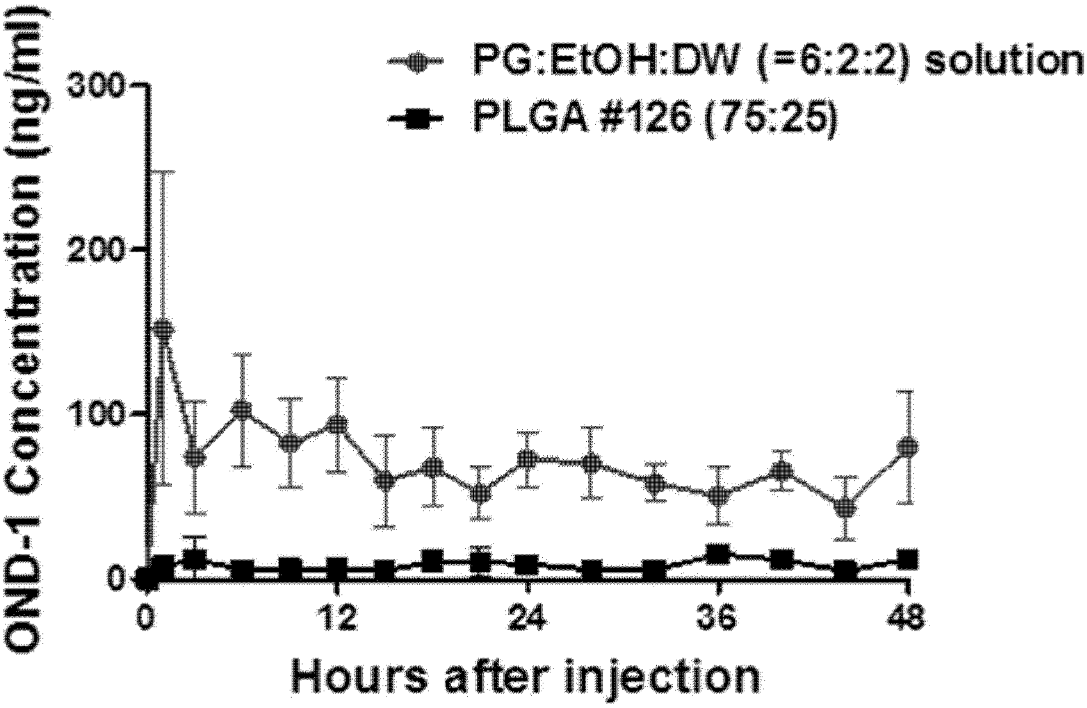
OND-1 Concentration (ng/ml)
300
200
100
0
PG:EtOH:DW (=6:2:2) solution
PLGA #126 (75:25)
0
12
24
36
48
Hours after injection

【FIG. 41】

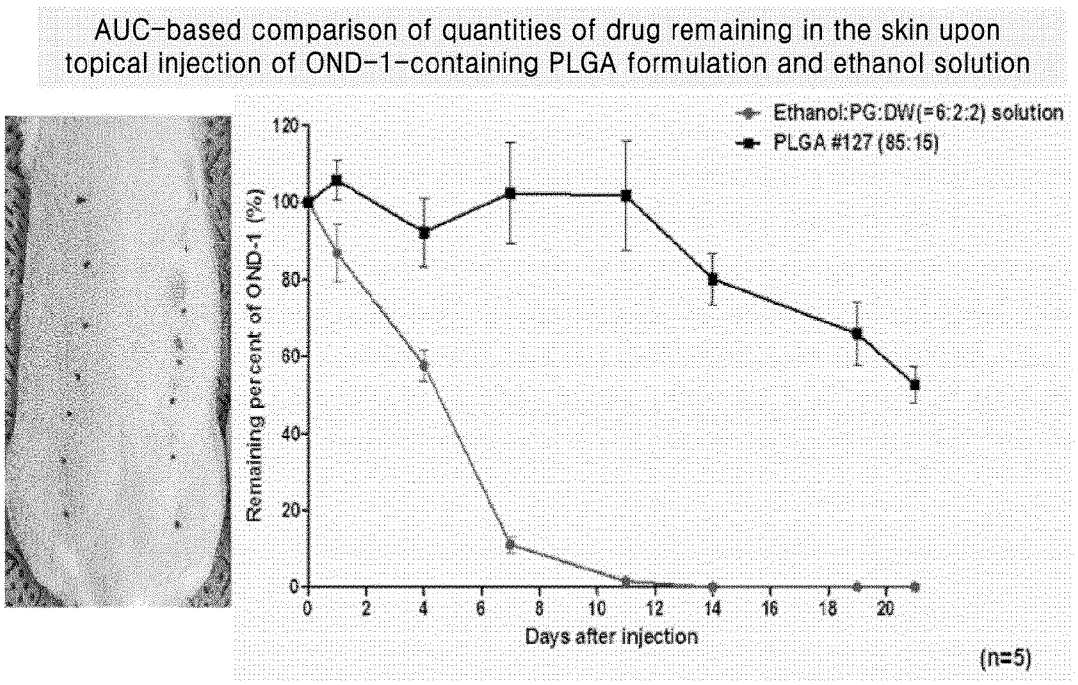
AUC-based comparison of quantities of drug remaining in the skin upon topical injection of OND-1-containing PLGA formulation and ethanol solution
Ethanol:PG:DW(=6:2:2) solution
PLGA #127 (85:15)
Remaining percent of OND-1 (%)
120
100
80
60
40
20
0
0
2
4
6
8
10
12
14
16
18
20
Days after injection
(n=5)

【FIG. 42】

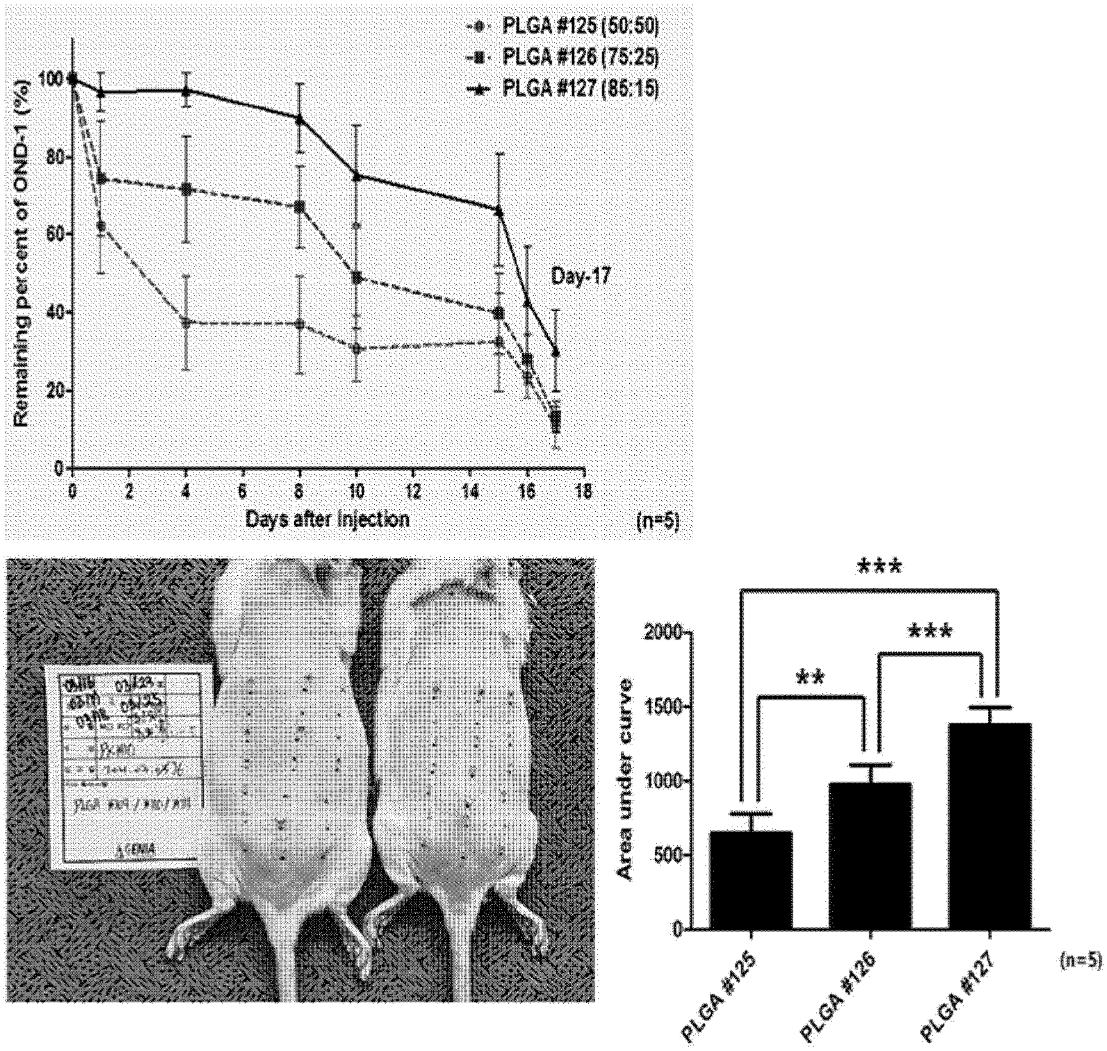
PLGA #125 (50:50)
PLGA #126 (75:25)
PLGA #127 (85:15)
Remaining percent of OND-1 (%)
Day-17
Days after injection
(n=5)
Area under curve
***
**
***
PLGA #125
PLGA #126
PLGA #127
(n=5)

**INTERNATIONAL SEARCH REPORT**

International application No.
**PCT/KR2022/095079**

**A. CLASSIFICATION OF SUBJECT MATTER**

**A61K 9/16**(2006.01)i; **A61K 38/13**(2006.01)i; **A61K 9/00**(2006.01)i; **A61P 17/14**(2006.01)i; **A61K 47/34**(2006.01)i; **A61K 47/32**(2006.01)i; **A61K 38/12**(2006.01)i

According to International Patent Classification (IPC) or to both national classification and IPC

**B. FIELDS SEARCHED**

Minimum documentation searched (classification system followed by classification symbols)

A61K 9/16(2006.01); A61K 38/00(2006.01); A61K 38/13(2006.01); A61K 47/14(2006.01); A61K 8/44(2006.01); A61K 9/00(2006.01); A61Q 7/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 사이클로스포린 A(cyclosporin A), 발모(hair growth), 방출 조절(release control), 약물 전달(drug delivery), 폴리락타이드-co-글리콜라이드(poly(DL-lactic acid-co-glycolic acid, PLGA)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2004-0033380 A (LG HOUSEHOLD & HEALTH CARE LTD.) 28 April 2004 (2004-04-28)<br>See claims 1-23; and paragraphs [0021]-[0086] and [0102]-[0251]. | 1-42 |
| Y | KR 10-0465012 B1 (LG HOUSEHOLD & HEALTH CARE LTD.) 13 January 2005 (2005-01-13)<br>See claim 11; and paragraphs [0049]-[0104]. | 1-24 |
| Y | MENSAH, R. A. et al. Optimising poly(lactic-co-glycolic acid) microparticle fabrication using a Taguchi orthogonal array design-of-experiment approach. PLoS ONE. 26 September 2019 (publication date), vol. 14, article no. e0222858, inner pp. 1-22.<br>See abstract; and inner pages 5-6. | 25-42 |
| Y | JP 2005-522423 A (AMOREPACIFIC CORP.) 28 July 2005 (2005-07-28)<br>See claims 1, 6 and 7. | 29-31 |
| A | KR 10-2019-0119936 A (GACHON UNIVERSITY OF INDUSTRY-ACADEMIC COOPERATION FOUNDATION et al.) 23 October 2019 (2019-10-23)<br>See claims 1-17. | 1-42 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 July 2022** | **26 July 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2022/095079**

| Patent document cited in search report | Publication date (day/month/year) | Patent family member(s) | Publication date (day/month/year) |
|---|---|---|---|
| KR 10-2004-0033380 A | 28 April 2004 | None | |
| KR 10-0465012 B1 | 13 January 2005 | AT 473730 T | 15 July 2010 |
| | | AT 493176 T | 15 January 2011 |
| | | AU 2003-274785 A1 | 07 June 2004 |
| | | BR PI0209619 B1 | 19 May 2015 |
| | | CA 2446971 A1 | 21 November 2002 |
| | | CN 1507339 A | 23 June 2004 |
| | | CN 1711069 A | 21 December 2005 |
| | | DE 60237003 D1 | 26 August 2010 |
| | | DE 60335563 D1 | 10 February 2011 |
| | | EP 1387660 A1 | 11 February 2004 |
| | | EP 1560558 A1 | 10 August 2005 |
| | | EP 1560558 B1 | 29 December 2010 |
| | | ES 2348225 T3 | 01 December 2010 |
| | | ES 2358587 T3 | 12 May 2011 |
| | | JP 2004-530685 A | 07 October 2004 |
| | | JP 4214181 B2 | 28 January 2009 |
| | | JP 4647310 B2 | 09 March 2011 |
| | | KR 10-0439467 B1 | 09 July 2004 |
| | | KR 10-2004-0039622 A | 12 May 2004 |
| | | MX PA03010027 A | 08 September 2005 |
| | | PL 208536 B1 | 31 May 2011 |
| | | PL 366407 A1 | 24 January 2005 |
| | | RU 2003135846 A | 27 March 2005 |
| | | RU 2291682 C2 | 20 January 2007 |
| | | TR 200301931 T2 | 21 September 2004 |
| | | US 2003-0186857 A1 | 02 October 2003 |
| | | US 2003-0207798 A1 | 06 November 2003 |
| | | US 2004-0087496 A1 | 06 May 2004 |
| | | US 6987090 B2 | 17 January 2006 |
| | | WO 0209-2032 A1 | 21 November 2002 |
| | | WO 2004-041221 A1 | 21 May 2004 |
| | | ZA 200308532 A | 01 July 2004 |
| | | ZA 200308532 B | 01 July 2004 |
| JP 2005-522423 A | 28 July 2005 | AU 2003-206169 A1 | 02 September 2003 |
| | | CA 2472242 A1 | 07 August 2003 |
| | | CN 1625391 A | 08 June 2005 |
| | | EP 1469840 A1 | 27 October 2004 |
| | | KR 10-2003-0065831 A | 09 August 2003 |
| | | US 2003-0147954 A1 | 07 August 2003 |
| | | WO 0306-3841 A1 | 07 August 2003 |
| KR 10-2019-0119936 A | 23 October 2019 | KR 10-2146704 B1 | 21 August 2020 |
| | | WO 2019-198923 A1 | 17 October 2019 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Helv. Chim. Acta,* 1987, vol. 70, 13-36
- *J of Con Release,* 2011, vol. 151, 286-294
- *Quimica Nova,* 2012, vol. 35 (4), 723-727
- *Current Drug Delivery,* 2006, vol. 3, 343-349
- *The AAPS Journal,* 2007, vol. 9 (2
- *Materials Research,* 2008, vol. 11 (2), 207-211
- *International Journal of Pharmaceutics,* 2010, vol. 389, 186-194